# EUROPEAN PATENT SPECIFICATION

(11) **EP 1 149 076 B1**
(45) Date of publication and mention of the grant of the patent: **22.10.2003**
(21) Application number: 99901985.4
(22) Date of filing: 05.02.1999
(51) Int. Cl.: C07C 333/04, A61K 31/325

(54) **O-THIOCARBAMOYL-AMINOALKANOL COMPOUNDS, THEIR PHARMACEUTICALLY ACCEPTABLE SALTS AND PROCESS FOR PREPARING THE SAME**
O-THIOCARBAMOYL-AMINOALKANOL-DERIVATE, IHRE PHARMAZEUTISCH VERTRÄGLICHEN SALZE UND VERFAHREN ZU IHRER HERSTELLUNG
COMPOSES D'O-THIOCARBAMOYL-AMINOALCANOL, LEURS SELS ACCEPTABLES SUR LE PLAN PHARMACEUTIQUE ET LEUR PROCEDE DE PREPARATION

(43) Date of publication of application: 31.10.2001
(73) Proprietor: SK Corporation, Seoul 110-110 (KR)
(72) Inventor: CHOI, Yong, Moon, Towaco, NJ 07082 (US); KIM, Yong Kil, Yusung-ku, Tajon 305-390 (KR)
(74) Representative: Knowles, James Atherton
(86) International application number: KR9900059
(87) International publication number: WO00046191

(56) References cited:
- WO-A1-96/20922
- WO-A1-96/32378
- US-A- 2 901 501
- US-A- 2 937 119

## Description

### BACKGROUND OF THE INVENTION

### Field of the Invention

The present invention relates, in general, to novel thiocarbamates of aminoalkanols and pharmaceutically useful salts thereof, useful in the treatment of the diseases of the central nervous system. More particularly, the present invention relates to racemic mixture or enantiomerically enriched O-thiocarbamoyl- aminoalkanol compounds and pharmaceutically useful salts thereof, processes for their production, compositions thereof and methods of treating central nervous system disorders.

### Description of the Prior Art

Phenylethylamine derivatives, one important class of therapeutical medicines useful in managing central nervous system (CNS) diseases, have been used mainly to treat obesity, narcolepsy, minimal brain dysfunction and mild depression.

Organic thiocarbamates or carbamates have been effectively used for controlling various CNS disorders. For example, U.S. Pat. No. 2,901,501 discloses 1,3-dithiocarbamoyl-1,3-propanediols that are further substituted at position-2 with hydrocarbon based moieties that include aryl groups. J. Am. Chem. Soc., 73, 5779 (1951) discloses 2-methyl-2-propyl-1,3-propandiol dicarbamate and its pharmaceutical activity was verified in J. Pharmacol. Exp. Ther., 104, 229 (1952). Besides, there are many carbamate compounds that are suggested as therapeutics for CNS disease in the prior art. For example, U.S. Pat. Nos. 2,884,444 and 2,937,119 disclose carbamates, such as 2-phenyl-1,3-propandiol dicarbamate and isopropylmeprobamate, respectively. These compounds are found to be very effectively used as therapeutics for treating CNS disorders, especially as anti epileptic and centrally acting muscle relaxants. WO 96/32378 discloses 3-N-substituted thiocarbamoyl-2-phenyl-1,3-propanediol carbamate useful to treat the diseases of the central nervous system, and methods of preparing the same. Research in the development of thiocarbamate or carbamate therapeutics for CNS diseases has been and continues to be actively advanced.

Recent design of pharmacologically useful compounds has been based on amino acids or the derivatives thereof, which is mainly attributable to the fact that many of the compounds found in biological systems come from amino acids or the derivatives thereof. In addition, in most cases, the function of a pharmaceutically useful compound is effected after it binds to an enzyme or receptor, which may trigger the regulatory mechanisms of the enzyme or receptor.

### SUMMARY OF THE INVENTION

As a result of intensive and thorough research, it has been found that O-thiocarbamoyl-aminoalkanol compounds are pharmaceutically useful for CNS disorders, especially for depression and anxiety.

Accordingly, it is a principal object of the present invention to provide racemic O-thiocarbamoyl-aminoalkanol compounds, represented by the following general structural formula (VI): 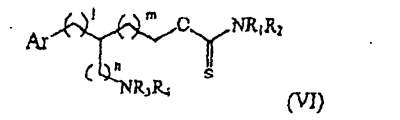 wherein Ar is a phenyl group as described as followings: 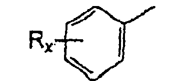 wherein R is a member selected from the group consisting of hydrogen, lower lkyl of 1 to 8 carbon atoms, halogen selected from F, Cl, Br, and I, alkoxy containing 1 to 3 carbon atoms, thioalkoxy containing 1 to 3 carbon atoms, nitro, hydroxy, or trifluorocarbon, and x is an integer from 1 to 3, with the proviso that R is the same or different when x is 2 or 3. R₁ and R₂ may be same or different from each other and are independently selected from the group consisting of hydrogen, lower alkyl from 1 to 8 carbon atoms, aryl, 3 to 7-membered aliphatic cyclic compounds and R₁ and R₂, together with the adjoining N-atom, form a 5 to 7-membered cyclic compound which optionally comprises zero to one additional nitrogen atom substituted with a member selected from the group consisting of hydrogen, alkyl and aryl groups, or zero to one oxygen atom directly unconnected. R₃ and R₄ may be same or different from each other and are independently selected from the group consisting of hydrogen, lower alkyl from 1 to 8 carbon atoms, aryl, 3 to 7-membered aliphatic cyclic compounds and R₃ and R₄, together with the adjoining N-atom, form a 5 to 7-membered cyclic compound which optionally comprises zero to one additional nitrogen atom substituted with a member selected from the group consisting of hydrogen, alkyl and aryl groups, or zero to one oxygen atom directly unconnected. Each of 1, m and n is zero or 1, and the pharmaceutically acceptable salts thereof.

It is also a principal object of the present invention to provide O-thiocarbamoyl-(D)-aminoalkanol compounds, represented by the following structural formula VIII: (alternatively, "D" can be referred to as the R-configuration at the chiral center in structural formula VIII) 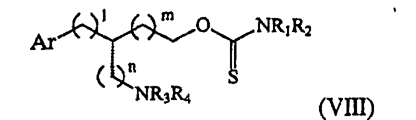 wherein Ar is a phenyl group as described as follows: 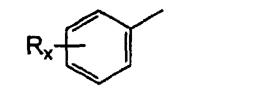 wherein R is a member selected from the group consisting of hydrogen, lower alkyl of 1 to 8 carbon atoms, halogen selected from F, Cl, Br, and I, alkoxy containing 1 to 3 carbon atoms, thioalkoxy containing 1 to 3 carbon atoms, nitro, hydroxy, or trifluorocarbon, and x is an integer from 1 to 3, with the proviso that R is the same or different when x is 2 or 3. R₁ and R₂ may be the same or different from each other and are independently selected from the group consisting of hydrogen, lower alkyl from 1 to 8 carbon atoms, aryl, 3 to 7-membered aliphatic cyclic compounds and R₁ and R₂, together with the adjoining N-atom, form a 5 to 7-membered cyclic compound which optionally comprises zero to one additional nitrogen atom substituted with a member selected from the group consisting of hydrogen, alkyl and aryl groups, or zero to one oxygen atoms directly unconnected. R₃ and R₄ may be same or different from each other and are independently selected from the group consisting of hydrogen, lower alkyl from 1 to 8 carbon atoms, aryl, 3 to 7-membered aliphatic cyclic compounds and R₃ and R₄, together with the adjoining N-atom, form a 5 to 7-membered cyclic compound which optionally comprises zero to one additional nitrogen atoms substituted with a member selected from the group consisting of hydrogen, alkyl and aryl groups, or zero to one oxygen atom directly unconnected. Each of 1, m and n is zero or 1, and the pharmaceutically acceptable salts thereof.

It is also a principal object of the present invention to provide O-thiocarbamoyl-(L)-aminoalkanol compounds, represented by the following structural formula IX: (alternatively, "L" can be referred to as the S-configuration at the chiral center in structural formula (IX) 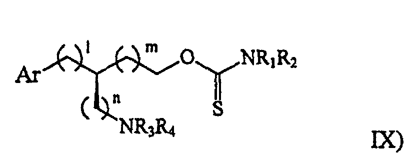 wherein Ar is a phenyl group as described as follows: 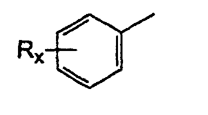 wherein R is a member selected from the group consisting of hydrogen, lower alkyl of 1 to 8 carbon atoms, halogen selected from F, Cl, Br, and I, alkoxy containing 1 to 3 carbon atoms, thioalkoxy containing 1 to 3 carbon atoms, nitro, hydroxy, or trifluorocarbon, and x is an integer from 1 to 3, with the proviso that R is the same or different when x is 2 or 3. R₁ and R₂ may be same or different from each other and are independently selected from the group consisting of hydrogen, lower alkyl of 1 to 8 carbon atoms, aryl, 3 to 7-membered aliphatic cyclic compounds and R₁ and R₂ together with the adjoining N-atom, form a 5 to 7-membered cyclic compound which may comprise zero to one additional nitrogen atom substituted with a member selected from the group consisting of hydrogen, alkyl and aryl groups, or zero to one oxygen atom directly unconnected. R₃ and R₄ may be same or different from each other and are independently selected from the group consisting of hydrogen, lower alkyl of 1 to 8 carbon atoms, aryl, 3 to 7-membered aliphatic cyclic compounds and R₃ and R₄ together with the adjoining N-atom, form a 5 to 7-membered cyclic compound which may comprise zero to one additional nitrogen atom substituted with a member selected from the group consisting of hydrogen, alkyl and aryl groups, or zero to one oxygen atom directly unconnected. Each of I, m and n is zero or 1, and the pharmaceutically acceptable salts thereof.

It is also a principal object of the present invention to provide a racemic or enantiomerically enriched O-Thiocarbamoyl-aminoalkanol compound represented by the following structural formula (X): 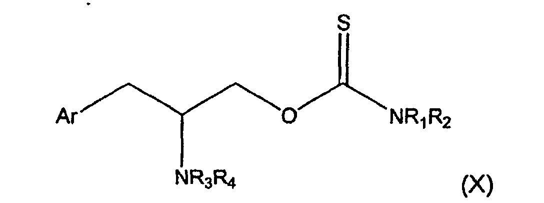 wherein Ar, R₁, R₂, R₃ and R₄ are as defined above.

It is also a principal object of the present invention to provide an enantiomerically enriched O-Thiocarbamoyl-(D)-aminoalkanol compound represented by the following structural formula (XI): 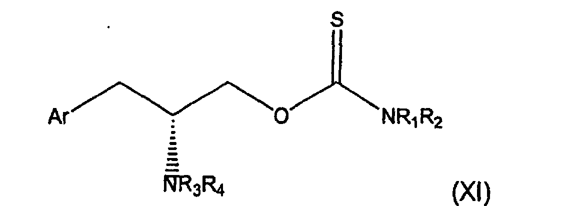 wherein Ar, R₁, R₂, R₃ and R₄ are as defined above.

It is also a principal object of the present invention to provide an enantiomerically enriched O-Thiocarbamoyl-(L)-aminoalkanol compound represented by the following structural formula (XII): 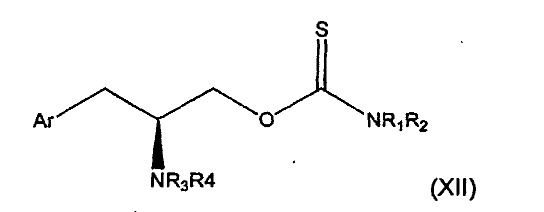 wherein Ar, R₁, R₂, R₃ and R₄ are as defined above.

It is also a principal object of the present invention to provide a racemic or enantiomerically enriched O-Thiocarbamoyl-aminoalkanol compound represented by the following structural formula (XIII): 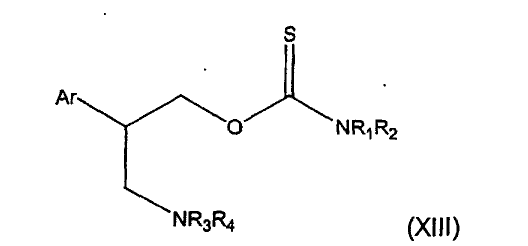 wherein Ar, R₁, R₂, R₃ and R₄ are as defined above.

It is also a principal object of the present invention to provide an enantiomerically enriched O-Thiocarbamoyl-(D)-aminoalkanol compound represented by the following structural formula (XIV): 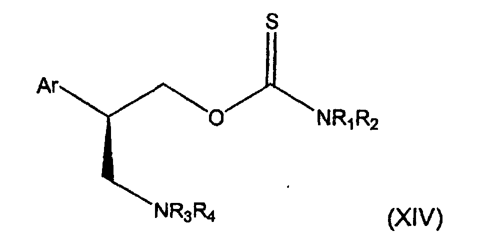 wherein Ar, R₁, R₂, R₃ and R₄ are as defined above.

It is also a principal object of the present invention to provide an enantiomerically enriched O-Thiocarbamoyl-(L)-aminoalkanol compound represented by the following structural formula (XV): 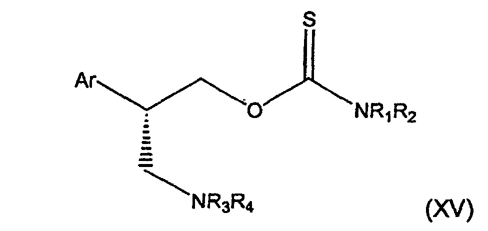 wherein Ar, R₁, R₂, R₃ and R₄ are as defined above.

It is also a principal object of the present invention to provide a racemic or enantiomerically enriched O-Thiocarbamoyl-aminoalkanol compound represented by the following structural formula (XVI): 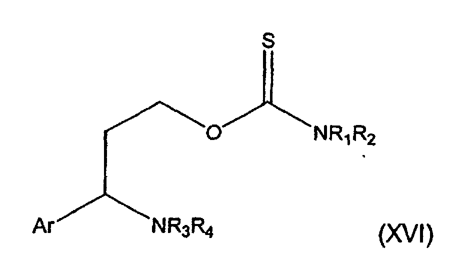 wherein Ar, R₁, R₂, R₃ and R₄ are as defined above.

It is also a principal object of the present invention to provide an enantiomerically enriched O-Thiocarbamoyl-(D)-aminoalkanol compound represented by the following structural formula (XVII): 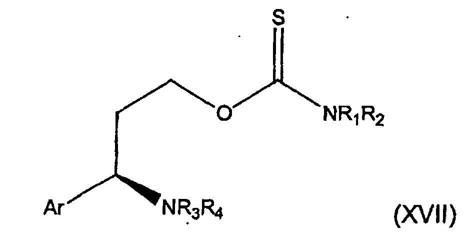 wherein Ar, R₁, R₂, R₃ and R₄ are as defined above.

It is also a principal object of the present invention to provide an enantiomerically enriched O-Thiocarbamoyl-(L)-aminoalkanol compound represented by the following structural formula (XVIII): 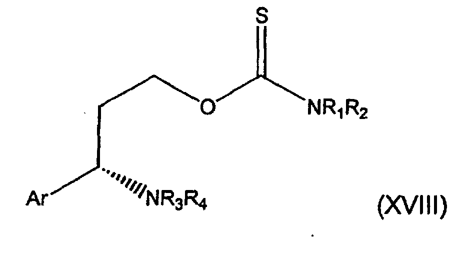 wherein Ar, R₁, R₂, R₃ and R₄ are as defined above.

It is also a principal object of the present invention to provide a racemic or enantiomerically enriched O-Thiocarbamoyl-aminoalkanol compound represented by the following structural formula (XIX): 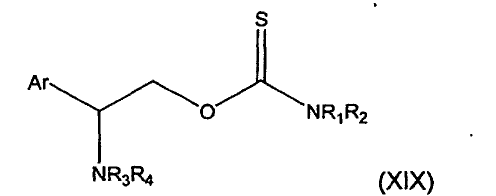 wherein Ar, R₁, R₂, R₃ and R₄ are as defined above.

It is also a principal object of the present invention to provide an enantiomerically enriched O-Thiocarbamoyl-(D)-aminoalkanol compound represented by the following structural formula (XX): 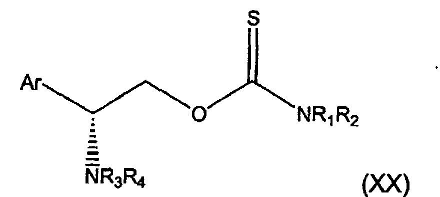 wherein Ar, R₁, R₂, R₃ and R₄ are as defined above.

It is also a principal object of the present invention to provide an enantiomerically enriched O-Thiocarbamoyl-(L)-aminoalkanol compound represented by the following structural formula (XXI): 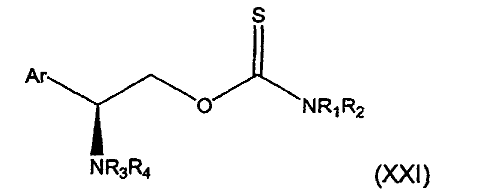 wherein Ar, R₁, R₂, R₃ and R₄ are as defined above.

### DETAILED DESCRIPTION OF THE INVENTION

In accordance with the present invention, the racemic or enantiomerically enriched O-thiocarbamoyl-aminoalkanols represented by the structural formula VI, VIII and IX and pharmaceutically acceptable salts thereof can be prepared by the following steps staring from readily available staring materials represented by the following general structural formula II: 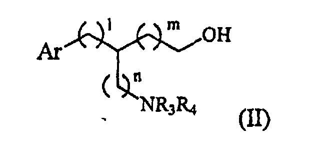 wherein Ar is the same as defined above.

It should be noted that the stereochemistry of the final products (VI, VIII and IX) depend solely on that of the starting material (II); a starting material (II) with an D-enantiomer yields only a product with D-enantiomer (VIII) and a starting material (II) with an L-enantiomer yields only a product with L-enantiomer (IX)

The first method for preparing the novel compounds of the general formula VI in which R₃=R₄=H or R₄=H will be described below in detail.

Initially, aminoalkanol (II) is reacted with Di-*t*-butyl dicarbonate to synthesize N-*t*-butyloxycarbonyl-aminoalkanol represented by the general formula (III).

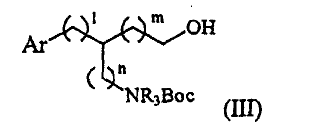

This is followed by the treatment with sodium hydride, carbon disulfide and methyl iodide in an ethereal solution which is followed by the treatment with an amine base (IV) to yield O-thiocarbamoyl-N-*t*-butyloxycarbonyl- aminoalkanol represented by the general formula (V): 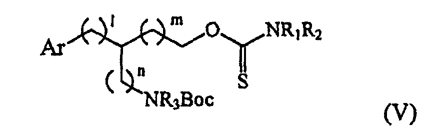 wherein Ar, R₁, R₂, R₃, R₄, l, m and n are as defined above and Boc represents t-butyloxy carbonyl radical . Then, this intermediate is deprotected by aqueous hydrochloric acid solution. As a result of the deprotection, there is obtained O-thiocarbamoyl-aminoalkanol represented by the general formula VI. Without further purification, the compound of formula VI may be converted into pharmaceutically acceptable salts (I) as described above.

This procedure is summarized as set forth in Reaction Scheme I below.

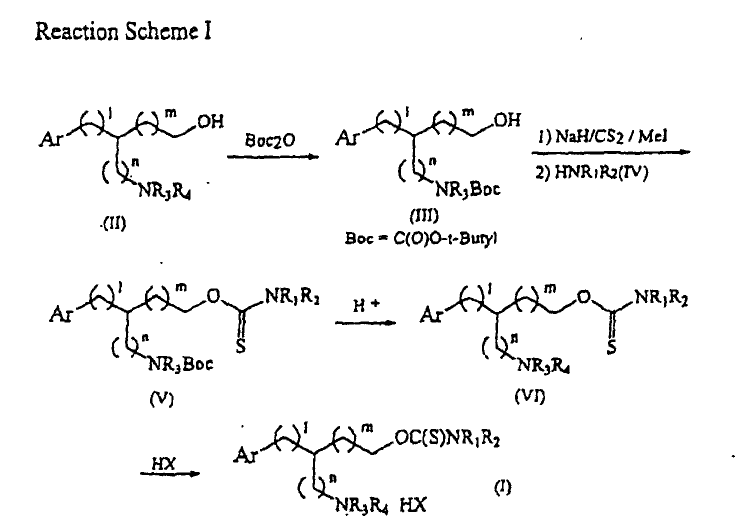

Details of the reaction conditions described in Reaction Scheme I are as follows. In the first step, the concentration of the starting material (II) is 0.005 to 3 moles with di-t-butyl dicarbonate ranging from 1.0 to 2.0 equivalents. The basic aqueous solution has a pH value between 7 and 14 and the conversion is carried out at temperature from -10 to 70 °C. For the conversion of the compound (III) to the compound (IV), sodium hydride and carbon disulfide ranging from 1.0 to 2.0 equivalents and methyl iodide ranging from 1.0 to 2.5 equivalent is used and is preferably carried out at a temperature of -10 to 70 °C. Without purification, the resulting intermediate is treated with 1 to 5 equivalents of amine at a temperature of -10 to 30 °C, to give the compound of the general formula (V). For this thiocarbamoylation, an ethereal solvent, such as diethyl ether and tetrahydrofuran, or a polar aprotic solvent, such as dimethylformamide and dimethyl sulfoxide, is employed. The compound of the general formula (VI) (0.005 to 3 moles) is treated with aqueous to 12 N hydrochloric acid at a temperature of -10 to 30 °C, followed by neutralization.

In the Reaction Scheme I, HX represents an acid capable of forming a pharmacologically useful salt with the basic nitrogen atom. Specific examples of the anhydrous acid used for the preparation of the compound (I) from the compound (VI) include hydrochloric acid, sulfuric acid, phosphoric acid, acetic acid, benzoic acid, citric acid, malonic acid, salicylic acid, malic acid, fumaric acid, oxalic acid, succinic acid, tartaric acid, lactic acid, gluconic acid, ascorbic acid, maleic acid, aspartic acid, benzene sulfonic acid, methane sulfonic acid, ethane sulfonic acid, hydroxymethane sulfonic acid and hydroxyethane sulfonic acid . Additional acids can refer to "Pharmaceutical Salts", J. Pharm. Sci., 1977; 66 (I): 1-19. This preparation is executed in a reaction media which can be exemplified by an ethereal solvent such as THF, an alcoholic solvent such as methanol, an ester solvent such as ethyl acetate, an aromatic solvent, and any compositional mixture thereof. An ethereal solvent is recommended as an addition solution, including ethyl ether, propyl ether, isopropyl ether, butyl ether, isobutyl ether. The concentration of the compound (VI) is on the order of 0.01 to 5 mole.

The second method for preparing the novel compounds of the general formula (VI) in which R₃ and R₄ are not hydrogen will be described in detail below.

Initially, aminoalkanol (II) is reacted with sodium hydride, carbon disulfide and methyl iodide in an ethereal solution resulting in a xanthate which is , then, treated with an amine base (IV) to yield O-thiocarbamoyl- aminoalkanol represented by the general formula (VI) 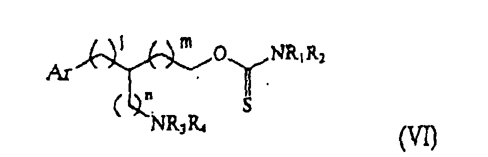 wherein Ar, R₁, R₂, R₃, R₄, l, m and n are as defined above. Without further purification, the compound of formula (VI) may be converted into pharmaceutically acceptable salts (I) as described above.

This procedure is summarized as set forth in Reaction Scheme II below.

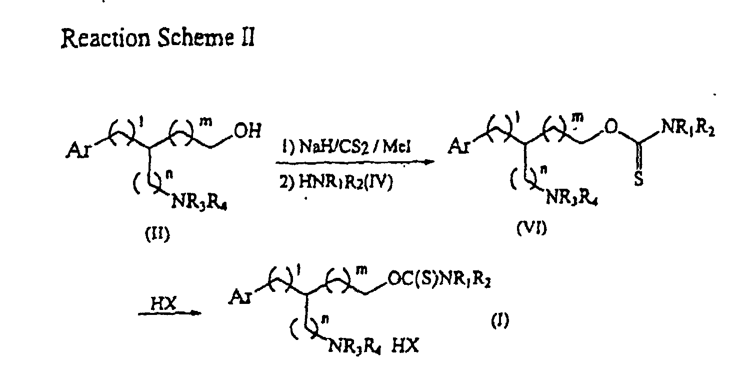

Details of the reaction conditions described in Reaction Scheme I are as follows. In the first step, for the conversion of the compound (II) to the compound (VI), sodium hydride and carbon disulfide ranging from 1.0 to 2.0 equivalents and methyl iodide ranging from 1.0 to 2.5 equivalents is used and is preferably carried out at a temperature of -10 to 70 °C. Without purification, the resulting intermediate is treated with 1 to 5 equivalents of amine(IV) at a temperature of -10 to 30 °C, to give the compound of the general formula (VI). For this thiocarbamoylation, an ethereal solvent, such as diethyl ether and tetrahydrofuran, or a polar aprotic solvent ,such as dimethylformamide and dimethyl sulfoxide, is employed. The compound of the general formula (VI) (0.005 to 3 moles) is treated with aqueous 1 to 12 N hydrochloric acid at a temperature of -10 to 30 °C, followed by neutralization.

In the Reaction Scheme II, HX represents an acid capable of forming a pharmacologically useful salt with the basic nitrogen atom.

The third method for preparing the novel compounds of the general formula (VI) in which R₁=R₃ = R₄ = H or R₁=R₄ = H will be described in detail below.

Initially, reacting N-(t-butyloxycarbonyl)-aminoalkanol (III) with isothiocyanate (VII) yields a N-(t-butyloxycarbonyl)-O-thiocarbamoyl-aminoalkanol represented by the general formula (V): 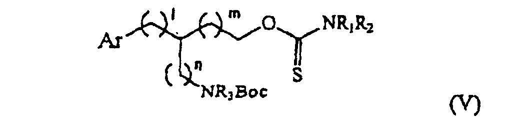 wherein Ar, R₁, R₂, R₃, l, m and n are as defined above and Boc represents t-butyloxy carbonyl radical . Then, this intermediate is deprotected by aqueous hydrochloric acid solution. As a result of the deprotection, there is obtained O-thiocarbamoyl-aminoalkanol represented by the general formula (VI). Without further purification, the compound of formula (VI) may be converted into pharmaceutically acceptable salts (I) as described above.

This procedure is summarized as set forth in Reaction Scheme III below.

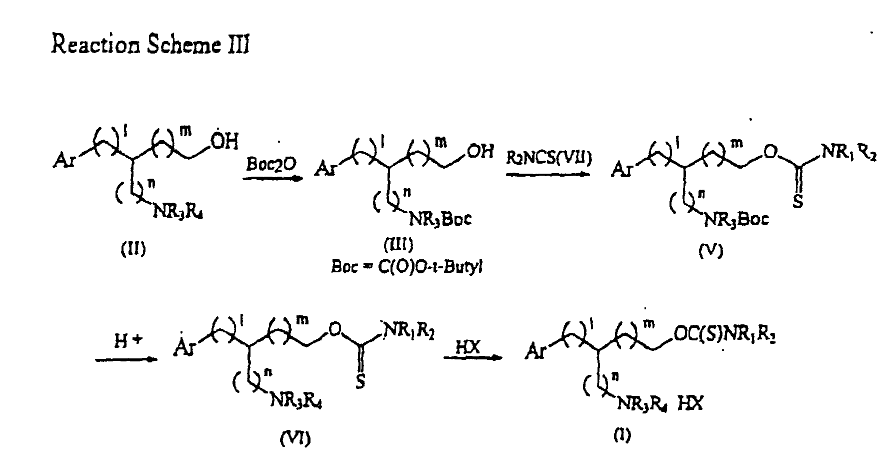

Details of the reaction conditions described in Reaction Scheme III are as follows. In the second step, the concentration of the starting material (III) is 0.005 to 3 moles with isothiocyanate (VII) ranging from 1.0 to 2.0 equivalents. The conversion is carried out at temperature from 30 to 110 °C. For this thiocarbamoylation, an ethereal solvent such as diethyl ether and tetrahydrofuran, a halogenated hydrocarbon solvent, such as dichloromethane and chloroform or an aromatic solvent, such as benzene and toluene may be used, with the halogenated hydrocarbon solvent, such as dichloromethane and chloroform being preferred. The compound of the general formula (VI) (0.005 to 3 moles) is treated with aqueous 1 to 12 N hydrochloric acid at a temperature of -10 to 30 °C, followed by neutralization.

The fourth method for preparing the novel compounds of the general formula (VI) in which R₃ and R₄ are not hydrogen and R₁ is hydrogen will be described in detail below.

Initially, aminoalkanol (II) is reacted with isothiocyanate (VII) in a halogenated hydrocarbon solvent to yield a O-thiocarbamoyl-aminoalkanol represented by the general formula (VI): 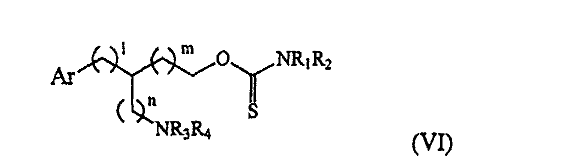 wherein Ar, R₁, R₂, R₃ , l, m and n are as defined above. Without further purification, the compound of formula (VI) may be converted into pharmaceutically acceptable salts (I) as described above.

This procedure is summarized as set forth in Reaction Scheme IV below.

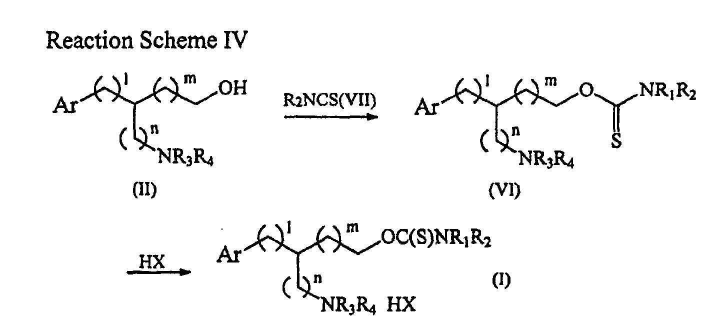

Representative examples of the compounds (VI), (VIII) and (IX) from Reaction Scheme I, II, III and IV are shown in Table I:

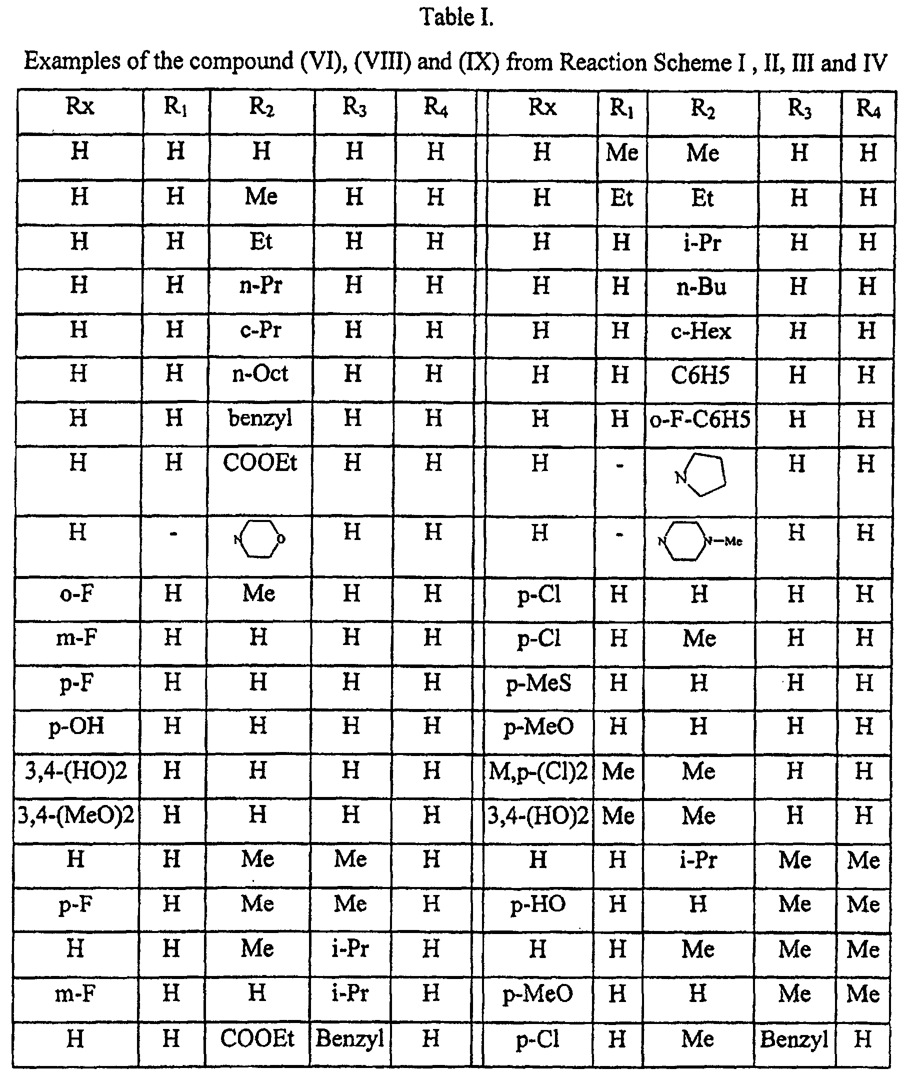

For therapeutic use in medicines for treating pain, depression, anxiety, epilepsy, stroke, demential and Parkinson's disease, the compounds of the present invention, alone or in combination with a pharmaceutically acceptable carrier, are administered to patients at a dosage of from 0.7 to 7,000 mg per day. For a normal human adult with a body weight of approximately 70 kg, the administration amount is translated into a daily dose of 0.01 to 100 mg per kg of body weight, The specific dosage employed, however, will vary depending upon the requirements of the patient, the severity of the patient's condition and the activity of the compound. The determination of optimum dosages for a particular situation must be determined clinically and is within the skill of the art.

In utilizing the compounds of the present invention for the treatment of disorders and diseases of the central nervous system, particularly to treat depression, it is preferred to administer the compounds orally. Since the compounds arc well-absorbed orally, it usually will not be necessary to resort to parenteral administration. For oral administration, the compound (I) of female is preferably combined with a pharmaceutical carrier. The ratio of the carrier to the compound of Formula (I) is not critical to express the effects of the medicine on the central nervous system, and it can vary considerably depending on whether the composition is to be filled into capsules or formed into tablets. In tableting, various edible pharmaceutical carriers or mixtures thereof can be used. Suitable carriers, for example, are a mixture of lactose, diabasic calcium phosphate and/or corn starch. Other pharmaceutically acceptable ingredients can be further added, including lubricants such as magnesium stearate.

Besides the compound of Formula (I), compositions comprising it are within the scope of the present invention. Furthermore., the present invention includes uses of the compound (I) and/or the composition.

As described hereinbefore, the compounds represented by Structural Formula I were observed to be useful for the prophylaxis and treatment of CNS disorders including pain, depression, anxiety, epilepsy, stroke, demential and Parkinson's disease.

The therapeutic use of the compounds claimed in the present invention as antidepressants has been proven by the "Forced Swimming Test", a well known pharmacological screening methods for depression and the results are shown in the following Table II.

The procedure described by Porsolt et al., (1997) was used. Test compound was administered p.o. and i.p. to mice (CD-1 strain), one hour prior to the animals being immersed in a 1500mL glass beaker (16.5cm deep, 13.0cm diameter) filled with water (20 ∼ 23°C) to about 5cm from the top. Mice were kept in the water for a period of 15 minutes and the duration of immobility observed within the 15-minute test period was recorded. A mouse was judged to be immobile if it floated motionlessly in the water making only those movements necessary to keep its head above the water.

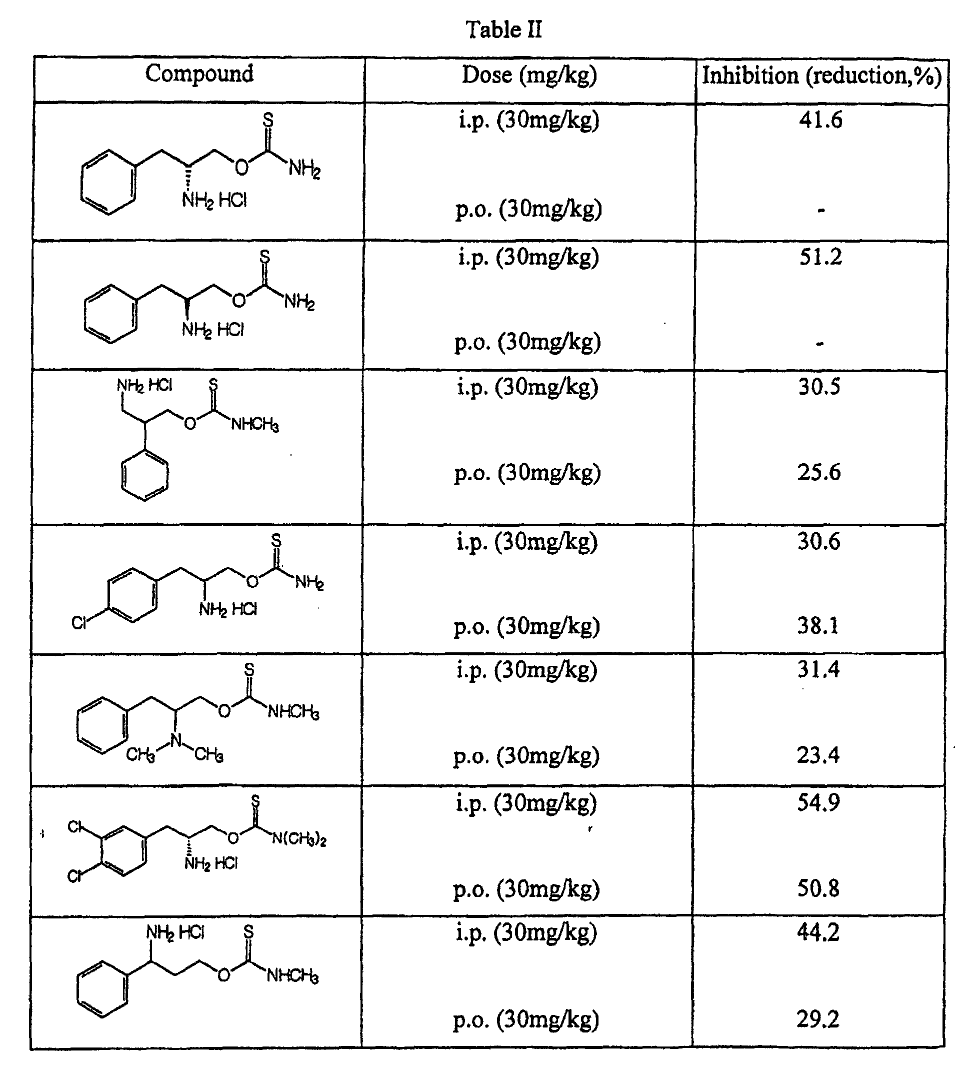

The therapeutic use of the compounds claimed in the present invention as anticonvulsants has been proven by the "Maximal ElectroShock (MES)" test, which is a well-established pharmacological screening method for anticonvulsants against partial seizures, and the results are presented in Table III.

The procedure employed in the MES test for anticonvulsants follows. The compound dosing solutions were prepared in saline, and the subject, namely, mice (CD-1 strain), were dosed orally. After the designated number of hours, maximal electroshock was induced in mice via corneal electrodes using IITC Life Science model 11A Shocker at 50mA-60Hz for 0.2 second. Upon inducing maximal electroshock, the elimination of hindlimb tonic extension was considered as providing evidence of the protection by an anticonvulsant. Median efficacy dose (ED50) levels were determined using three different dose levels with at least 6 mice in each group. Compounds with smaller ED50 value are more potent as anticonvulsants.

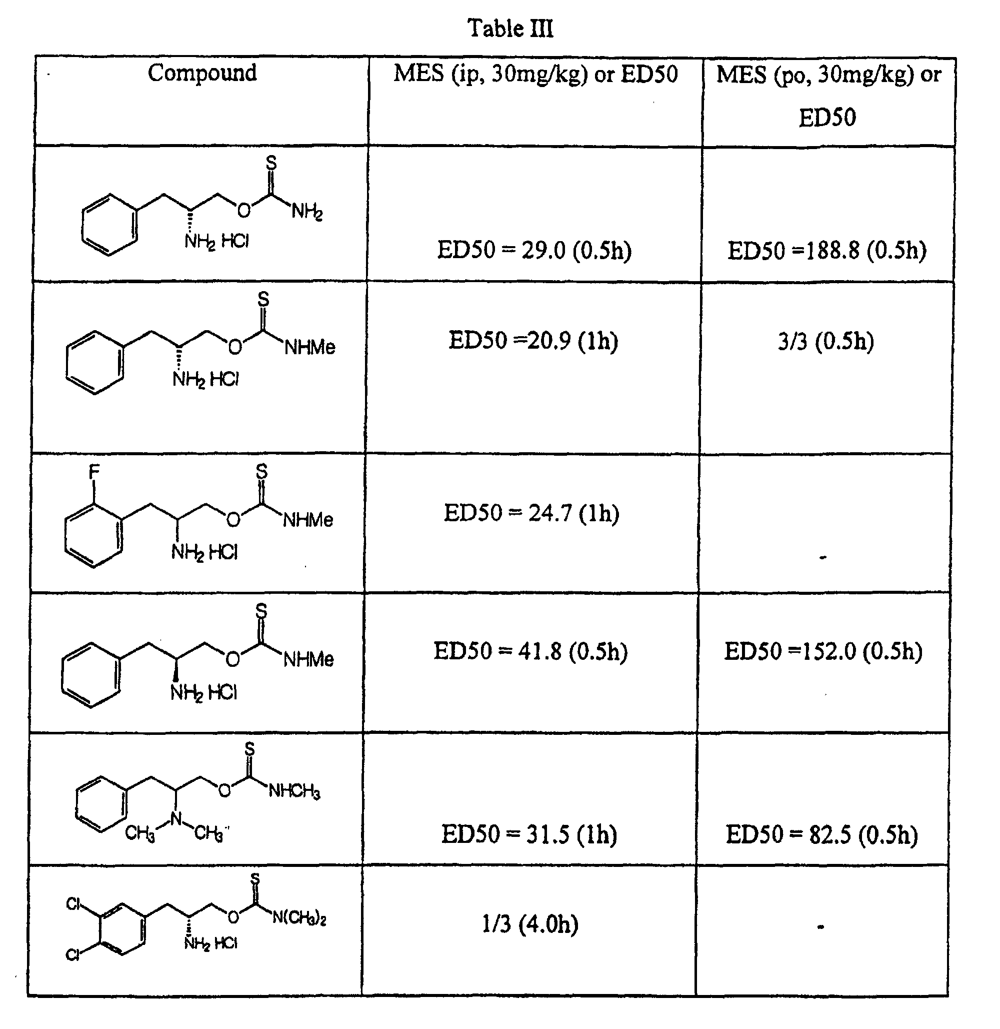

A better understanding of the present invention may be obtained in light of following examples which are set forth to illustrate, but are not to be construed to limit, the present invention.

### Example 1

### O-Thiocarbamoyl-N-(t-butyloxycarbonyl)-(DL)-phenylalaninol

In a 500mL flask equipped with magnetic stirrer, N-(t-butyloxycarbonyl)-(DL)-phenylalaninol(0.051 mole, 13.6g) was dissolved in 200mL of THF and was added with NaH(0.061 mole, 1.47g) at 0°C. The reaction mixture was stirred at 0°C for 1 hour, followed by the addition of CS₂(0.061 mole, 4.66g). After being stirred for 40 min. at 0°C, MeI(0.061 mole, 8.69g) was added to the reaction mixture. The reaction mixture was stirred at room temperature for 2 hours, followed by the addition of 5N NH₄OH (0.090 mole, 18.0mL) for 6 hours. The resulting solution was quenched with water. The organic layer was extracted 3 times with dichloromethane, dried over magnesium sulfate and distilled in vacuo, to give a solid. This was recrystalized in a solution mixture of n-hexane and diethyl ether, to produce 14.7g of O-thiocarbamoyl-N-(t-butyloxycarbonyl)-(DL)-phenylalaninol: Yield 80%.

₁H-NMR(CDCl₃, 200MHz), ppm(δ) ; 1.35(s,9H), 2.65-2.98(m,2H), 4.05-4.25(br,1H), 4.33-4.43(m,2H), 4.62-4.83(br,1H), 6.25(s,1H), 6.58(s,1H), 7.01-7.42(m,5H).

### Example 2

### O-Thiocarbamoyl-N-(t-butyloxycarbonyl)-(L)-phenylalaninol

The procedure given in Example 1 was followed using N-(t-butyloxycarbonyl)-(L)-phenylalaninol as a starting material, instead of N-(t-butyloxycarbonyl)-(DL)-phenylalaninol, to give 1.90g of the title compound. A yield of 79% was obtained.

₁H-NMR(CDCl₃, 200MHz), ppm(δ) ; 1.38(s,9H), 2.72-2.95(m,2H), 4.01-4.28(br,1H), 4.30-4.48(m,2H), 4.62-4.83(br,1H), 6.22(s,1H), 6.67(s,1H), 7.08-7.39(m,5H).

### Example 3

### O-Thiocarbamoyl-N-(t-butyloxycarbonyl)-(D)-phenylalaninol

In a 500mL flask equipped with magnetic stirrer, N-(t-butyloxycarbonyl)-(D)-phenylalaninol(0.040 mole, 10.05g) was dissolved in 200mL of THF and was added with NaH(0.048 mole, 1.15g) at 0°C. The reaction mixture was stirred at 0°C for 1 hour, followed by the addition of CS₂(0.048 mole, 3.65g). After being stirred for 40 min. at 0°C, MeI(0.048 mole, 6.81g) was added to the reaction mixture. The reaction mixture was stirred at room temperature for 2 hours, followed by the addition of 5N NH₄OH (0.090 mole, 18.0mL) for 6 hours. The resulting solution was quenched with water. The organic layer was extracted 3 times with dichloromethane, dried over magnesium sulfate and distilled in vacuo, to give a solid. This was recrystalized in a solution mixture of n-hexane and diethyl ether, to produce 11.3g of O-thiocarbamoyl-N-(t-butyloxycarbonyl)-(D)-phenylalaninol: Yield 78%.

₁H-NMR(CDCl₃, 200MHz), ppm(δ) ; 1.45(s,9H), 2.77-3.01(m,2H), 4.01-4.28(br,1H), 4.30-4.51(m,2H), 4.60-4.83(br,1H), 6.25(s,1H), 6.55(s,1H), 7.11-7.51(m,5H).

### Example 4

### O-(N-Methyl)thiocarbamoyl-N-(t-butyloxycarbonyl)-(D)-phenylalaninol

The procedure given in Example 3 was followed using methylamine as a reactant, instead of NH₄OH, to give 2.33g of the title compound. A yield of 75% was obtained.

₁H-NMR(CDCl₃, 200MHz), ppm(δ) ; 1.45(s,9H), 2.68-2.98(m,2.5H), 3.12(d,1.5H), 4.06-4.28(br,1H), 4.31-4.55(m,2H), 6.35(br,0.5H), 6.65(br,0.5H), 7.05-7.48(m,5H).

### Example 5

### O-(N-Cyclopropyl)thiocarbamoyl-N-(t-butyloxycarbonyl)-(D)-phenylalaninol

The procedure given in Example 3 was followed using cyclopropylamine as a reactant, instead of NH₄OH, to give 2.49g of the title compound. A yield of 74% was obtained.

₁H-NMR(CDCl₃, 200MHz), ppm(δ) ; 0.51-0.98(m,4H), 1.45(s,9H), 2.68-3.05(m,3H), 4.05-4.82(m,4H), 6.42(br,0.5H), 6.82(br,0.5H), 7.05-7.48(m,5H).

### Example 6

### O-(N-Octyl)thiocarbamoyl-N-(t-butyloxycarbonyl)-(D)-phenylalaninol

The procedure given in Example 3 was followed using n-octylamine as a reactant, instead of NH₄OH, to give 3.34g of the title compound. A yield of 78% was obtained.

₁H-NMR(CDCl₃, 200MHz), ppm(δ) ; 0.89(t,3H), 1.02-1.78(m,21H), 2.68-3.02(m,2H), 3.25(q,1H), 3.52(q,1H), 4.01-4.82(m,4H), 6.45(br,0.5H), 6.95(br,0.5H), 7.05-7.48(m,5H).

### Example 7

### O-(N-Isopropyl)thiocarbamoyl-N-(t-butyloxycarbonyl)-(D)-phenylalaninol

The procedure given in Example 3 was followed using isopropylamine as a reactant, instead of NH₄OH, to give 3.0g of the title compound. A yield of 80% was obtained.

₁H NMR(CDCl₃, 200MHz), ppm(δ) ; 1.12-1.65(m,15H), 2.72-2.955(m,2H), 4.05-4.55(m,3H), 4.65(br,1H), 6.15(br,0.5H), 6.52(br,0.5H), 7.05-7.48(m,5H)

### Example 8

### O-(N-Dimethyl)thiocarbamoyl-N-(t-butyloxycarbonyl)-(D)-phenylalaninol

The procedure given in Example 3 was followed using dimethylamine as a reactant, instead of NH₄OH, to give 2.56g of the title compound. A yield of 67% was obtained.

₁H-NMR(CDCl₃, 200MHz), ppm(δ) ; 1.45(s,9H), 2.78-2.98(m,2H), 3.12(s,3H), 3.38(s,3H), 4.18-4.52(m,3H), 4.58-4.74(br,1H), 7.10-7.38(m,5H).

### Example 9

### O-(N-Morpholino)thiocarbamoyl-N-(t-butyloxycarbonyl)-(D)-phenylalaninol

The procedure given in Example 3 was followed using morpholine as a reactant, instead of NH₄OH, to give 2.97g of the title compound. A yield of 75% was obtained.

₁H-NMR(CDCl₃, 200MHz), ppm(δ) ; 1.45(s,9H), 2.68-2.98(m,2H), 3.55-3.92(m,6H), 4.01-4.75(m,6H), 7.05-7.48(m,5H).

### Example 10

### O-(N-Methyl)thiocarbamoyl-N-(t-butyloxycarbonyl)-(DL)-(o-fluoro) phenylalaninol

The procedure given in Example 1 was followed using N-(t-butyloxycarbonyl)-(DL)-(o-fluoro)phenylalaninol as a starting material and methylamine as a reactant, instead of N-(t-butyloxycarbonyl)-(DL)-phenylalaninol and NH₄OH, to give 1.01g of the title compound. A yield of 60% was obtained.

₁H-NMR(CDCl₃, 200MHz), ppm(δ) ; 1.35(s,9H), 2.72-3.15(m,5H), 4.01-4.28(br,1H), 4.30-4.52(m,2H), 4.58-4.83(br,1H), 6.42(s,1H), 6.71(s,1H), 6.98-7.32(m,5H).

### Example 11

### O-Thiocarbamoyl-N-(t-butyloxycarbonyl)-(DL)-(p-chloro)phenylalaninol

The procedure given in Example 1 was followed using N-(t-butyloxycarbonyl)-(DL)-(p-chloro)phenylalaninol as a starting material, instead of N-(t-butyloxycarbonyl)-(DL)-phenylalaninol, to give 3.0g of the title compound. A yield of 62% was obtained.

₁H-NMR(DMSO-d₆,200MHz), ppm(δ) ; 1.32(s,9H), 2.62-2.82(m,2H), 3.79-4.01(br,1H), 4.03-4.42(m,3H), 6.98-7.42(m,5H), 8.25(s,1H), 8.65(s,1H).

### Example 12

### O-Thiocarbamoyl-N-(t-butyloxycarbonyl)-(D)-(t-butyloxycarbonyloxy) phenylalaninol

The procedure given in Example 3 was followed using N-(t-butyloxycarbonyl)-(D)-(t-butyloxycarbonyloxy)phenylalaninol as a starting material, instead of N-(t-butyloxycarbonyl)-(D)-phenylalaninol, to give 2.9g of the title compound. A yield of 60% was obtained.

₁H-NMR(CDCl₃, 200MHz), ppm(δ) ; 1.18-1.75(m,18H), 2.78-3.01(m,2H), 4.11-4.25(m,1H), 4.35-4.51(m,2H), 4.65-4.85(m,1H), 6.22(br.s,1H), 6.66(br.s,1H), 7.02-7.35(m,4H).

### Example 13

### O-(N-Dimethyl)thiocarbamoyl-N-(t-butyloxycarbonyl)-(D)-(m,p-dichloro) phenylalaninol

The procedure given in Example 3 was followed using N-(t-butyloxycarbonyl)-(D)-(m,p-dichloro)phenylalaninol as a starting material and dimethylamine as a reactant, instead of N-(t-butyloxycarbonyl)-(D)-phenylalaninol and NH₄OH, to give 2.5g of the title compound. A yield of 62% was obtained.

₁H-NMR(CDCl₃, 200MHz), ppm(δ) ; 1.28-1.42(m,9H), 2.69-2.88(m,2H), 3.11(d,3H), 3.37(d,3H), 4.09-4.28(m,1H), 4.35-4.51(m,2H), 4.61-4.81(m,1H), 7.01-7.41(m,3H).

### Example 14

### O-(N-Ethoxycarbonyl)thiocarbamoyl-N-(t-butyloxycarbonyl)-(D)-phenylalaninol

In a 100mL flask equipped with magnetic stirrer, N-(t-butyloxycarbonyl)-(D)-phenylalaninol(0.013 mole, 3.3g) was dissolved in 50mL of chloroform and was added with ethoxycarbonyl isothiocyanate(0.014 mole, 1.7mL) at room temperature. The reaction mixture was heated under reflux for 6 hours and then cooled to room temperature. The resulting solution was quenched with water. The organic layer was extracted 3 times with dichloromethane, dried over magnesium sulfate and distilled in vacuo, to give a solid. This was recrystalized in a solution mixture of n-hexane and diethyl ether, to produce 2.56g of O-(N-ethoxycarbonyl)thiocarbamoyl-N-(t-butyloxycarbonyl)-(D)-phenylalaninol. A yield of 56% was obtained.

₁H-NMR(CDCl₃, 200MHz), ppm(δ) ; 1.18-1.61(m,12H), 2.82-3.08(m,2H), 4.22-4.38(m,3H), 4.45(d,2H), 5.01(d,1H), 7.02-7.45(m,5H), 8.25(s,1H).

### Example 15

### O-(N-Ethoxycarbonyl)thiocarbamoyl-N-(t-butyloxycarbonyl)-(L)-phenylalaninol

The procedure given in Example 14 was followed using N-(t-butyloxycarbonyl)-(L)-phenylalaninol as a starting material, instead of N-(t-butyloxycarbonyl)-(D)-phenylalaninol, to give 3.4g of the title compound. A yield of 64% was obtained.

₁H-NMR(CDCl₃, 200MHz), ppm(δ) ; 1.18-1.48(m,12H), 2.85-3.01(m,2H), 4.22-4.32(m,3H), 4.45(d,2H), 5.01(d,1H), 7.12-7.35(m,5H), 8.28(s,1H).

### Example 16

### 3-N-(t-Butyloxycarbonyl)amino-3-phenyl-1-O-((N-methyl)thiocarbamoyl) propanol

The procedure given in Examplel was followed using 3-N-(t-butyloxycarbonyl)amino-3-phenyl-1-propanol as a starting material and methyl amine as a reactant instead of N-(t-butyioxycarbonyi)-(DL)-phenylalaninol and NH₄OH to give 3-N-(t-butyloxycarbonyl) amino-3-phenyl-1-O-((N-methyl)thiocarbamoyl) propanol. A yield of 70% was obtained.

₁H-NMR(CDCl₃, 200MHz), ppm(δ) ; 1.18-1.48(m,9H), 2.68-32.92(m,3H), 3.02(d,2H), 4.01-4.18(m,1H), 4.29-4.48(m,2H), 4.85-4.92(m,1H), 6.81(br.,0.4H), 7.05-7.31(m,5H), 7.42(br.,0.6H).

### Example 17

### 3-N-(t-Butyloxycarbonyl)amino-2-phenyl-1-O-(thiocarbamoyl)propanol

The procedure given in Examplel was followed using 3-N-(t-butyloxycarbonyl)amino-2-phenyl-1-propanol as a starting material instead of N-(t-butyloxycarbonyl)-(DL)-phenylalaninol, to give 3-N-(t-butyloxycarbonyl)amino-2-phenyl-1-O-(thiocarbamoyl) propanol. A yield of 66% was obtained.

₁H-NMR(CDCl₃, 200MHz), ppm(δ) ; 1.42 (s, 9H), 3.21-3.40 (m, 2H), 3.55-3.68 (m, 1H), 4.50-4.70 (m, 3H), 6.20 (bs, 1H), 6.42 (bs, 1H), 7.20-7.40 (m, 5H)

### Example 18

### 3-N-(t-Butyloxycarbonyl)amino-2-phenyl-1-O-((N-methly)thiocarbamoyl) propanol

The procedure given in Examplel was followed using 3-N-(t-butyloxycarbonyl)amino-2-phenyl-1-propanol as a starting material and methyl amine as a reactant instead of N-(t-butyloxycarbonyl)-(DL)-phenylalaninol and NH₄OH to give 3-N-(t-butyloxycarbonyl)amino-2-phenyl-1-O-((N-methyl)thiocarbamoyl) propanol. A yield of 92% was obtained.

₁H-NMR(CDCl₃, 200MHz), ppm(δ) ; 1.40 (9H), 2.89 (1H), 3.06 (H), 3.13-3.45(2H), 3.45-3.72 (1H), 4.40-5.80 (2H), 6.30-6.70 (1H), 7.10-7.40 (5H)

### Example 19

### 3-N-(t-Butyloxycarbonyl)amino-2-phenyl-1-O-((N-dimethyl)thiocarbamoyl) propanol

The procedure given in Example 1 was followed using 3-N-(t-butyloxycarbonyl)amino-2-phenyl-1-propanol as a starting material and dimethyl amine as a reactant instead of N-(t-butyloxycarbonyl)-(DL)-phenylalaninol and NH₄OH to give 3-N-(t-butyloxycarbonyl) amino-2-phenyl-1-O-((N-dimethyl)thiocarbamoyl) propanol. A yield of 65% was obtained.

₁H-NMR(CDCl₃, 200MHz), ppm(δ) ; 1.41 is, 9H), 3.01 (s, 3H), 3.33 (3, 3H), 3.20-3.45 (m, 2H), 3.60 (m, 1H), 4.55-4.70 (m, 3H), 7.20-7.40 (m, 5H)

### Example 20

### O-(N-Methyl)thiocarbamoyl-N-dimethyl-(D)-phenylalaninol

The procedure given in Example 3 was followed using N-dimethyl-D-phenylalaninol as a starting material and methylamine as a reactant, instead of N-(t-butyloxycarbonyl)-(D)-phenylalaninol and NH₄OH, to give 3.52g of the title compound. A yield of 92% was obtained.

₁H-NMR(CDCl₃, 200MHz), ppm(δ) ; 2.35(s,7.6H), 2.65-2.82(m,1.4H), 2.85-3.18(m,3H), 4.25-4.59(m,2H), 6.55(br,0.76H), 6.75(br,0.14H), 6.98-7.32(m,5H).

### Example 21

### O-Thiocarbamoyl-(DL)-phenylalaninol Hydrochloride

In a 100mL flask equipped with magnetic stirrer, O-thiocarbamoyl-N-(t-butyloxycarbonyl)-(DL)-phenylalaninol obtained in Example 1 was dissloved in 40mLof THF and was added with 20mL of 6N aqueous hydrochloric acid solution. The reaction mixture was stirred at room temperature for 8 hours, followed by the neutralization with saturated aqueous potassium carbonate solution. Thereafter, the organic layer was extracted 3 times with dichloromethane, dried over magnesium sulfate and distilled in vacuo, to give a yellowish liquid. This was dissolved in 30mL of THF and added with anhydrous hydrochloric acid at 0°C, to obtained desirable white precipitates. To this was added 30mL of anhydrous diethyl ether, with the aim of maximizing the precipitation. As a result, 1.32g of the title compound was obtained: Yield 82%.

Melting point : 181.3 - 181.6 °C

₁H-NMR(DMSO-d₆, 200MHz), ppm(δ) ; 2.78-3.18(m,2H), 3.78-4.02(m,1H), 4.15(d-d,1H), 4.37(d-d,1H), 7.08-7.45(m,5H), 8.38(br,4H), 9.02(s,1H).

### Example 22

### O-Thiocarbamoyl-(L)-phenylalaninol Hydrochloride

The procedure given in Example 21 was followed using O-thiocarbamoyl-N-(t-butyloxycarbonyl)-(L)-phenylalaninol as a starting material, to give the title compound.

Melting point : 173.2 - 173.6 °C

₁H-NMR(DMSO-d₆, 200MHz), ppm(δ) ; 2.78-3.15(m,2H), 3.58-3.82(br,1H), 4.15(d-d,1H), 4.35(d-d,1H), 7.02-7.43(m,5H), 8.45(br,4H), 9.01(s,1H).

### Example 23

### O-Thiocarbamoyl-(D)-phenylalaninol Hydrochloride

The procedure given in Example 21 was followed using O-thiocarbamoyl-N-(t-butyloxycarbonyl)-(D)-phenylalaninol as a starting material, to give the title compound.

Melting point : 181.5 - 181.7 °C

₁H-NMR(DMSO-d₆, 200MHz), ppm(δ) ; 2.78-3.22(m,2H), 3.58-3.82(br,1H), 4.08-4.48(m,2H), 7.12-7.58(m,5H), 8.45(br,4H), 9.05(s,1H).

### Example 24

### O-(N-Methyl)thiocarbamoyl-(D)-phenylalaninol Hydrochloride

The procedure given in Example 21 was followed using O-(N-methyl)thiocarbamoyl-N-(t-butyloxycarbonyl)-(D)-phenylalaninol as a starting material, to give the title compound.

Melting point : 178.1 - 179.5 °C

₁H-NMR(DMSO-d₆, 200MHz), ppm(δ) ; 2.75-3.25(m,5H), 3.65-3.95(br,1H), 4.11-4.55(m,2H), 7.18-7.61(m,5H), 7.48(br,3H), 9.25(d-d,1H).

### Example 25

### O-(N-Cyclopropyl)thiocarbamoyl-(D)-phenylalaninol Hydrochloride

The procedure given in Example 21 was followed using O-(N-cyclopropyl) thiocarbamoyl-N-(t-butyloxycarbonyl)-(D)-phenylalaninol as a starting material, to give the title compound.

Melting point : 180 - 181 °C

₁H-NMR(DMSO-d₆, 200MHz), ppm(δ) ; 0.45-0.91(m,4H), 2.80-3.26(m,3H), 3.62-3.92(m,1H), 4.15-4.61(m,2H), 7.18-7.58(m,5H), 8.45(br,3H), 9.45(d-d,1H).

### Example 26

### O-(N-Octyl)thiocarbamoyl-(D)-phenylalaninol Hydrochloride

The procedure given in Example 21 was followed using O-(N-octyl)thiocarbamoyl-N-(t-butyloxycarbonyl)-(D)-phenylalaninol as a starting material, to give the title compound.

Melting point : 123 - 124 °C

₁H-NMR(DMSO-d₆, 200MHz), ppm(δ) ; 0.89(t,3H), 1.12-1.68(m,14H), 2.78-3.35(m,4H), 3.75(br,1H), 4.01-4.82(m,4H), 7.05-7.48(m,5H), 8.52(br,3H), 9.32(d-d,1H).

### Example 27

### O-(N-Isopropyl)thiocarbamoyl-(D)-phenylalaninol Hydrochloride

The procedure given in Example 21 was followed using O-(N-isopropyl) thiocarbamoyl-N-(t-butyloxycarbonyl)-(D)-phenylalaninol as a starting material, to give the title compound.

Melting point : 200.1 - 201 °C

₁H-NMR(DMSO-d₆, 200MHz), ppm(δ) ; 1.05-1.45(m,6H), 2.82-3.22(m,2H), 3.62-3.92(m,1H), 4.05-4.55(m,3H), 7.18-7.58(m,5H), 8.45(br,3H), 9.25(t,1H).

### Example 28

### O-(N-Dimethyl)thiocarbamoyl-(D)-phenylalaninol Hydrochloride

The procedure given in Example 21 was followed using O-(N-dimethyl) thiocarbamoyl-N-(t-butyloxycarbonyl)-(D)-phenylalaninol as a starting material, to give the title compound.

Melting point : 185.6 - 186.4 °C

₁H-NMR(DMSO-d₆, 200MHz), ppm(δ) ; 2.75-3.55(m,7H), 3.99(br.s,1H), 4.12-4.58(m,2H), 7.11-7.58(m,5H), 8.65(br,3H).

### Example 29

### O-(N-Morpholino)thiocarbamoyl-(D)-phenylalaninol Hydrochloride

The procedure given in Example 21 was followed using O-(N-morpholino) thiocarbamoyl-N-(t-butyloxycarbonyl)-(D)-phenylalaninol as a starting material, to give the title compound.

Melting point : 202 - 203 °C

₁H-NMR(DMSO-d₆, 200MHz), ppm(δ) ; 2.78-3.18(m,2H), 3.38-4.08(m,11H), 4.18-4.53(m,2H), 7.18-7.48(m,5H), 8.41(br,3H).

### Example 30

### O-Thiocarbamoyl-(D)-(p-hydroxy)phenylalaninol Hydrochloride

The procedure given in Example 21 was followed using O-thiocarbamoyl-N-(t-butyloxycarbonyl)-(D)-(p-t-butyloxycarbonyloxy)phenylalaninol as a starting material, to give the title compound.

Melting point : 173.8 - 175.4 °C

₁H-NMR(DMSO-d₆, 200MHz), ppm(δ) ; 2.68-3.18(m,2H), 3.38-3.81(m,1H), 3.90-4.51(m,2H), 6.45-7.22(m,4H), 8.35(br,3H), 9.01 (s,1H), 9.41(s,1H).

### Example 31

### O-(N-Dimethyl)thiocarbamoyl-(D)-(3,4-dichloro)phenylalaninol Hydrochloride

The procedure given in Example 21 was followed using O-(N-dimethyl) thiocarbamoyl-N-(t-butyloxycarbonyl)-(D)-(m,p-dichloro)phenylalaninol as a starting material, to give the title compound.

Melting point : 189.7 - 190.3 °C

₁H-NMR(DMSO-d₆, 200MHz), ppm(δ) ; 2.82-3.48(m,8H), 3.72-3.94(m,1H), 4.25-4.59(m,2H), 7.32(d,1H), 7.52-7.75(m,2H), 8.35(br,3H).

### Example 32

### O-(N-Methyl)thiocarbamoyl-(DL)-(o-fluoro)phenylalaninol Hydrochloride

The procedure given in Example 21 was followed using O-(N-methyl)thiocarbamoyl-N-(t-butyloxycarbonyl)-(DL)-(o-fluoro)phenylalaninol as a starting material, to give the title compound.

Melting point : 169.6 - 170.2 °C

₁H-NMR(DMSO-d₆, 200MHz), ppm(δ) ; 2.78(d-d,3H), 2.85-3.22(m,2H), 3.62-3.81(m,1H), 4.18(d-d,1H), 4.46(d-d,1H), 6.98-7.48(m,4H), 8.45(br,3H), 9.20(d-d,1H).

### Example 33

### O-Thiocarbamoyl-(DL)-(p-chloro)phenylalaninol Hydrochloride

The procedure given in Example 21 was followed using O-thiocarbamoyl-N-(t-butyloxycarbonyl)-(DL)-(p-chloro)phenylalaninol as a starting material, to give the title compound.

Melting point : 187.5 - 187.7°C

₁H-NMR(DMSO-d₆, 200MHz), ppm(δ) ; 2.82-3.15(m,2H), 3.62-3.82(m,1H), 4.18(d-d,1H), 4.39(d-d,1H), 7.18-7.49(m,4H), 8.45(br,4H), 9.01(s,1H).

### Example 34

### O-(N-Ethoxycarbonyl)thiocarbamoyl-(D)-phenylalaninol Hydrochloride

The procedure given in Example 21 was followed using O-(N-ethoxyxcarbonyl) thiocarbamoyl-N-(t-butyloxycarbonyl)-(D)-phenylalaninol as a starting material, to give the title compound.

Melting point : 157 - 157.8 °C

₁H-NMR(DMSO-d₆, 200MHz), ppm(δ) ; 1.22(t,3H), 2.78-3.18(m,2H), 3.82(br,1H) 4.22(q,3H), 4.58(d-d,1H), 7.18-7.45(m,5H), 8.42(br,3H), 11.78(s,1H).

### Example 35

### O-(N-Ethoxycarbonyl)thiocarbamoyl-(L)-phenylalaninol Hydrochloride

The procedure given in Example 21 was followed using O-(N-ethoxycarbonyl) thiocarbamoyl-N-(t-butyloxycarbonyl)-(L)-phenylalaninol as a starting material, to give the title compound.

Melting point : 144.4 - 145.0 °C

₁H-NMR(DMSO-d₆, 200MHz), ppm(δ) ; 1.22(t,3H), 2.78-3.18(m,2H), 3.62-3.92(m,1H), 3.95-4.38(m,3H), 4.42-4.62(m,1H), 7.02-7.45(m,5H), 8.42(br,3H), 11.85(s,1H).

### Example 36

### 3-Amino-3-phenyl-l-(O-(N-methyl)thiocarbamoyl)propanol Hydrochloride

The procedure given in Example 21 was followed using 3-N-(t-Butyloxycarbonyl) amino-3-phenyl-1-O-((N-methyl)thiocarbamoyl)-propanol as a starting material, to give the title compound.

Melting Point : 182.1 - 182.4 °C

₁H-NMR(DMSO-d₆, 200MHz), ppm(δ) ; 2.71-3.12(m,3H), 3.22-3.48(m,2H), 3.75-3.95(m,1H), 4.18-4.62(m,2H), 7.25-7.62(m,5H), 8.25(br.,3H), 9.20(d,1H).

### Example 37

### 3-Amino-2-phenyl-1-(O-thiocarbamoyl)propanol Hydrochloride

The procedure given in Example 21 was followed using 3-N-(t-butyloxycarbonyl) amino-2-phenyl-1-O-(thiocarbamoyl)propanol as a starting material, to give the title compound.

Melting Point : 189.5-190.0 °C

₁H-NMR(DMSO-d₆, 200MHz), ppm(δ) ; 3.05-3.50 (m, 3H), 4.35-4.55 (m, 2H), 7.30 (s, 5H), 7.95 (bs, 3H), 8.53 (s, 1H), 8.85 (s, 1H).

### Example 38

### 3-Amino-2-phenyl-1-O-((N-methyl)thiocarbamoyl)propanol Hydrochloride

The procedure given in Example 21 was followed using 3-N-(t-butyloxycarbonyl) amino-2-phenyl-1-O-((N-methyl)thiocarbamoyl)-propanol as a starting material, to give the title compound.

Melting Point : 95.0-96.0 °C

₁H-NMR(DMSO-d₆, 200MHz), ppm(δ) ; 2.60 (d, 0.6H), 2.86 (d, 2.4 H), 3.07-3.48 (m, 3H), 4.39-4.63 (m, 2H), 7.37 (s, 5H), 8.10 (bs, 3H), 9.15 (bs, 0.2H), 9.30 (bs, 0.8 H)

### Example 39

### 3-Amino-2-phenyl-1-O-((N-dimethyl)thiocarbamoyl)propanol Hydrochloride

The procedure given in Example 21 was followed using 3-N-(t-butyloxycarbonyl)-amino-2-phenyl-1-O-((N-dimethyl)thiocarbamoyl)propanol as a starting material, to give the title compound.

Melting Point : 164.1-164.6 °C

₁H-NMR(DMSO-d₆, 200MHz), ppm(δ) ; 2.98 (s, 3H), 3.22 (s, 3H), 3.30-3.60 (m, 3H), 4.53 (m, 2H), 7.37 (m, 5H), 8.13 (bs, 3H)

### Example 40

### O-(N-Methyl)thiocarbamoyl- N-dimethyl-(D)-phenylalaninol Hydrochloride

The procedure given in Example 21 was followed using O-(N-methyl) thiocarbamoyl-N-dimethyl-(D)-phenylalanlnol as a starting material, to give the title compound.

Melting point : 152.5 - 152.7 °C

₁H-NMR(DMSO-d₆, 200MHz), ppm(δ) ; 2.55-3.15(m,9H), 3.15-3.45(m,2H), 3.75-4.02(m,1H), 4.15-4.71(m,2H), 7.05-7.58(m,5H), 9.25-9.65(m,1H), 11.05(br,1H).

The present invention has been described in an illustrative manner, and it is to be understood that the terminology used herein is intended to be in the nature of description rather than of limitation.

Many modifications and variations of the present invention are possible in light of the above teachings. Therefore, it is to be understood that within the scope of the appended claims, the invention may be practiced otherwise than as specifically described.

## Claims

1. A racemic or enantiomerically enriched O-Thiocarbamoyl-aminoalkanol compound represented by the following structural formula (VI): 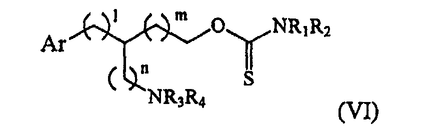 wherein Ar is a phenyl group as described as follows: 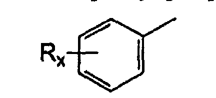 wherein R is a member selected from the group consisting of hydrogen, lower alkyl of 1 to 8 carbon atoms, halogen selected from F, Cl, Br, and I, alkoxy containing 1 to 3 carbon atoms, thioalkoxy containing 1 to 3 carbon atoms, nitro, hydroxy, or trifluorocarbon, and x is an integer from 1 to 3, with the proviso that R is the same or different when x is 2 or 3, R₁ and R₂ may be the same or different from each other and are independently selected from the group consisting of hydrogen, lower alkyl of 1 to 8 carbon atoms, aryl, 3 to 7-membered aliphatic cyclic compounds and R₁ and R₂, together with the adjoining N-atom, form a 5 to 7-membered cyclic compound which optionally comprises zero to one additional nitrogen atoms substituted with a member selected from the group consisting of hydrogen, alkyl and aryl groups, or zero to one oxygen atoms directly unconnected, R₃ and R₄ may be same or different from each other and are independently selected from the group consisting of hydrogen, lower alkyl of 1 to 8 carbon atoms, aryl, 3 to 7-membered aliphatic cyclic compounds and R₃ and R₄, together with the adjoining N-atom, form a 5 to 7-membered cyclic compound which optionally comprises zero to one additional nitrogen atoms substituted with a member selected from the group consisting of hydrogen, alkyl and aryl groups, or zero to one oxygen atoms directly unconnected each of l, m and n is zero or 1 , and the pharmaceutically acceptable salts thereof.

2. The O-Thiocarbamoyl-aminoalkanol compound represented by the structural formula (VI), in accordance with claim 1, wherein R is hydrogen, and the pharmaceutically acceptable salts thereof.

3. The O-Thiocarbamoyl-aminoalkanol compound represented by the structural formula (VI), in accordance with claim 1, wherein R is hydrogen, R₁ and R₂ may be the same or different from each other and are independently selected from the group consisting of hydrogen, lower alkyl of 1 to 8 carbon atoms, 3 to 7-membered aliphatic cyclic compounds and R₁ and R₂, together with the adjoining N-atom, form a 5 to 7-membered cyclic compound which optionally comprises zero to one additional nitrogen atom substituted with a member selected from the group consisting of hydrogen, alkyl and aryl groups, or zero to one oxygen atom directly unconnected, R₃ and R₄ may be same or different from each other and are independently selected from the group consisting of hydrogen, lower alkyl of 1 to 8 carbon atoms, 3 to 7-membered aliphatic cyclic compounds and R₃ and R₄, together with the adjoining N-atom, form a 5 to 7-membered cyclic compound which optionally comprises zero to one additional nitrogen atoms substituted with a member selected from the group consisting of hydrogen, alkyl and aryl groups, or zero to one oxygen atom directly unconnected, and the pharmaceutically acceptable salts thereof.

4. The O-Thiocarbamoyl-aminoalkanol compound represented by the structural formula (VI), in accordance with claim 1, wherein x is 1, and the pharmaceutically acceptable salts thereof.

5. An enantiomerically enriched O-Thiocarbamoyl-(D)-aminoalkanol compound according to claim 1 represented by the following structural formula (VIII):

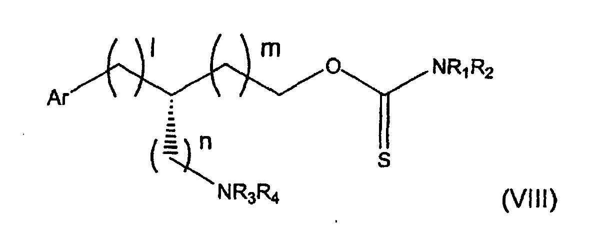

6. The O-Thiocarbamoyl-aminoalkanol compound represented by the structural formula (VIII), in accordance with claim 5, wherein R is hydrogen, and the pharmaceutically acceptable salts thereof.

7. The O-Thiocarbamoyl-aminoalkanol compound represented by the structural formula (VIII), in accordance with claim 5, wherein R is hydrogen, R₁ and R₂ may be the same or different from each other and are independently selected from the group consisting of hydrogen, lower alkyl of 1 to 8 carbon atoms, 3. to 7-membered aliphatic cyclic compounds and R₁ and R₂, together with the adjoining N-atom, form a 5 to 7-membered cyclic compound which optionally comprises zero to one additional nitrogen atom substituted with a member selected from the group consisting of hydrogen, alkyl and aryl groups, or zero to one oxygen atom directly unconnected, F₃ and R₄ may be same or different from each other and are independently selected from the group consisting of hydrogen, lower alkyl of 1 to 8 carbon atoms, 3 to 7-membered aliphatic cyclic compounds and R₃ and R₄, together with the adjoining N-atom, form a 5 to 7-membered cyclic compound which optionally comprises zero to one additional nitrogen atoms substituted with a member selected from the group consisting of hydrogen, alkyl and aryl groups, or zero to one oxygen atoms directly unconnected, and the pharmaceutically acceptable salts thereof.

8. The O-Thiocarbamoyl-aminoalkanol compound represented by the structural formula (VIII), in accordance with claim 5, wherein x is 1, and the pharmaceutically acceptable salts thereof.

9. An enantiomerically enriched O-Thiocarbamoyl-(L)-aminoalkanol compound according to claim 1 represented by the following structural formula (IX):

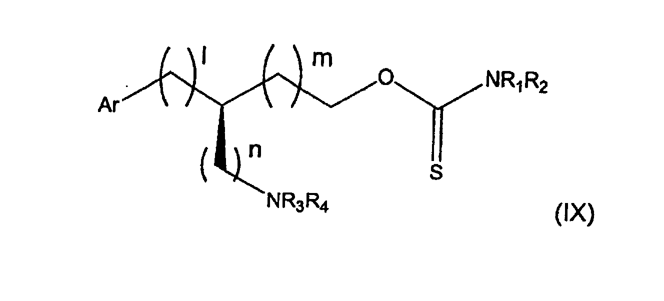

10. The O-Thiocarbamoyl-aminoalkanol compound represented by the structural formula (IX), in accordance with claim 9, wherein R is hydrogen, and the pharmaceutically acceptable salts thereof.

11. The O-Thiocarbamoyl-aminoalkanol compound represented by the structural formula (IX), in accordance with claim 9, wherein R is hydrogen, R₁ and R₂ may be same or different from each other and are independently selected from the group consisting of hydrogen, lower alkyl of 1 to 8 carbon atoms, 3 to 7-membered aliphatic cyclic compounds and R₁ and R₂, together with the adjoining N-atom, form a 5 to 7-membered cyclic compound which optionally comprises zero to one additional nitrogen atoms substituted with a member selected from the group consisting of hydrogen, alkyl and aryl groups, or zero to one oxygen atoms directly unconnected, R₃ and R₄ may be same or different from each other and are independently selected from the group consisting of hydrogen, lower alkyl of 1 to 8 carbon atoms, 3 to 7-membered aliphatic cyclic compounds and R₃ and R₄, together with the adjoining N-atom, form a 5 to 7-membered cyclic compound which optionally comprises zero to one additional nitrogen atoms substituted with a member selected from the group consisting of hydrogen, alkyl and aryl groups, or zero to one oxygen atoms directly unconnected, and the pharmaceutically acceptable salts thereof.

12. The O-Thiocarbamoyl-aminoalkanol compound represented by the structural formula (IX), in accordance with claim 9, wherein x is 1, and the pharmaceutically acceptable salts thereof.

13. A racemic or enantiomerically enriched O-Thiocarbamoyl-aminoalkanol compound according to claim 1 represented by the following structural formula (X):

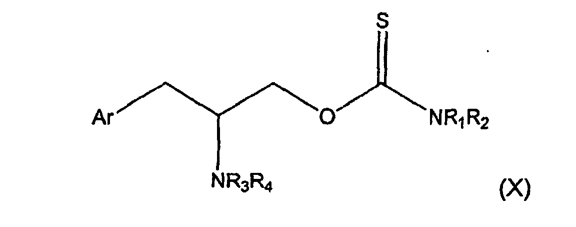

14. The O-Thiocarbamoyl-aminoalkanol compound represented by the structural formula (X), in accordance with claim 13, wherein R is hydrogen, and the pharmaceutically acceptable salts thereof.

15. The O-Thiocarbamoyl-aminoalkanol compound represented by the structural formula (X), in accordance with claim 13, wherein R is hydrogen, R₁ and R₂ may be same or different from each other and are independently selected from the group consisting of hydrogen, lower alkyl of 1 to 8 carbon atoms, 3 to 7-membered aliphatic cyclic compounds and R₁ and R₂, together with the adjoining N-atom, form a 5 to 7-membered cyclic compound which optionally comprises zero to one additional nitrogen atoms substituted with a member selected from the group consisting of hydrogen, alkyl and aryl groups, or zero to one oxygen atoms directly unconnected, R₃ and R₄ may be same or different from each other and are independently selected from the group consisting of hydrogen, lower alkyl of 1 to 8 carbon atoms, 3 to 7-membered aliphatic cyclic compounds and R₃ and R₄, together with the adjoining N-atom, form a 5 to 7-membered cyclic compound which optionally comprises zero to one additional nitrogen atoms substituted with a member selected from the group consisting of hydrogen, alkyl and aryl groups, or zero to one oxygen atoms directly unconnected, and the pharmaceutically acceptable salts thereof.

16. The O-Thiocarbamoyl-aminoalkanol compound represented by the structural formula (X), in accordance with claim 13, wherein x is 1, and the pharmaceutically acceptable salts thereof.

17. An enantiomerically enriched O-Thiocarbamoyl-(D)-aminoalkanol compound according to claim 1 represented by the following structural formula (XI):

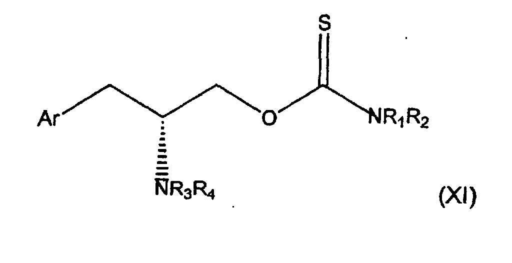

18. O-Thiocarbamoyl-aminoalkanol compound represented by the structural formula (XI), in accordance with claim 17, wherein R is hydrogen, and the pharmaceutically acceptable salts thereof.

19. The O-Thiocarbamoyl-aminoalkanol compound represented by the structural formula (XI), in accordance with claim 17, wherein R is hydrogen, R₁ and R₂ may be same or different from each other and are independently selected from the group consisting of hydrogen, lower alkyl of 1 to 8 carbon atoms, 3 to 7-membered aliphatic cyclic compounds and R₁ and R₂, together with the adjoining N-atom, form a 5 to 7-membered cyclic compound which optionally comprises zero to one additional nitrogen atoms substituted with a member selected from the group consisting of hydrogen, alkyl and aryl groups, or zero to one oxygen atoms directly unconnected, R₃ and R₄ may be same or different from each other and are independently selected from the group consisting of hydrogen, lower alkyl of 1 to 8 carbon atoms, 3 to 7-membered aliphatic cyclic compounds and R₃ and R₄, together with the adjoining N-atom, form a 5 to 7-membered cyclic compound which optionally comprises zero to one additional nitrogen atoms substituted with a member selected from the group consisting of hydrogen, alkyl and aryl groups, or zero to one oxygen atoms directly unconnected, and the pharmaceutically acceptable salts thereof.

20. The O-Thiocarbamoyl-aminoalkanol compound represented by the structural formula (XI), in accordance with claim 17, wherein x is 1, and the pharmaceutically acceptable salts thereof.

21. An enantiomerically enriched O-Thiocarbamoyl-(L)-aminoalkanol compound according to claim 1 represented by the following structural formula (XII):

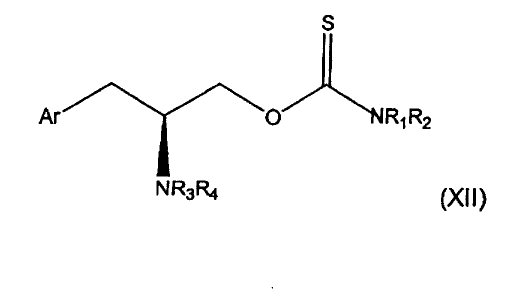

22. The O-Thiocarbamoyl-aminoalkanol compound represented by the structural formula (XII), in accordance with claim 21, wherein R is hydrogen, and the pharmaceutically acceptable salts thereof.

23. The O-Thiocarbamoyl-aminoalkanol compound represented by the structural formula (XII), in accordance with claim 21, wherein R is hydrogen, R₁ and R₂ may be the same or different from each other and are independently selected from the group consisting of hydrogen, lower alkyl of 1 to 8 carbon atoms, 3 to 7-membered aliphatic cyclic compounds and R₁ and R₂, together with the adjoining N-atom, form a 5 to 7-membered cyclic compound which optionally comprises zero to one additional nitrogen atoms substituted with a member selected from the group consisting of hydrogen, alkyl and aryl groups, or zero to one oxygen atoms directly unconnected, R₃ and R₄ may be same or different from each other and are independently selected from the group consisting of hydrogen, lower alkyl of 1 to 8 carbon atoms, 3 to 7-membered aliphatic cyclic compounds and R₃ and R₄, together with the adjoining N-atom, form a 5 to 7-membered cyclic compound which optionally comprises zero to one additional nitrogen atoms substituted with a member selected from the group consisting of hydrogen, alkyl and aryl groups, or zero to one oxygen atoms directly unconnected, and the pharmaceutically acceptable salts thereof.

24. The O-Thiocarbamoyl-aminoalkanol compound represented by the structural formula (XII), in accordance with claim 21, wherein x is 1, and the pharmaceutically acceptable salts thereof.

25. A racemic or enantiomerically enriched O-Thiocarbamoyl-aminoalkanol compound according to claim 1 represented by the following structural formula (XIII):

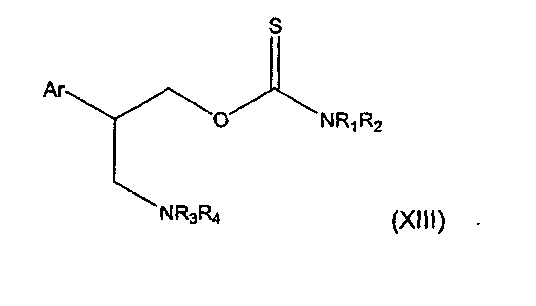

26. The O-Thiocarbamoyl-aminoalkanol compound represented by the structural formula (XIII), in accordance with claim 25, wherein R is hydrogen, and the pharmaceutically acceptable salts thereof.

27. The O-Thiocarbamoyl-aminoalkanol compound represented by the structural formula (XIII), in accordance with claim 25, wherein R is hydrogen, R₁ and R₂ may be same or different from each other and are independently selected from the group consisting of hydrogen, lower alkyl of 1 to 8 carbon atoms, 3 to 7-membered aliphati: cyclic compounds and R₁ and R₂, together with the adjoining N-atom, form a 5 to 7-membered cyclic compound which optionally comprises zero to one additional nitrogen atoms substituted with a member selected from the group consisting of hydrogen, alkyl and aryl groups, or zero to one oxygen atoms directly unconnected, R₃ and R₄ may be same or different from each other and are independently selected from the group consisting of hydrogen, lower alkyl of 1 to 8 carbon atoms, 3 to 7-membered aliphatic cyclic compounds and R₃ and R₄, together with the adjoining N-atom, form a 5 to 7-membered cyclic compound which optionally comprises zero to one additional nitrogen atoms substituted with a member selected from the group consisting of hydrogen, alkyl and aryl groups, or zero to one oxygen atoms directly unconnected, and the pharmaceutically acceptable salts thereof.

28. The O-Thiocarbamoyl-aminoalkanol compound represented by the structural formula (XIII), in accordance with claim 25, wherein x is 1, and the pharmaceutically acceptable salts thereof.

29. An enantiomerically enriched O-Thiocarbamoyl-(D)-aminoalkanol compound according to claim 1 represented by the following structural formula (XIV):

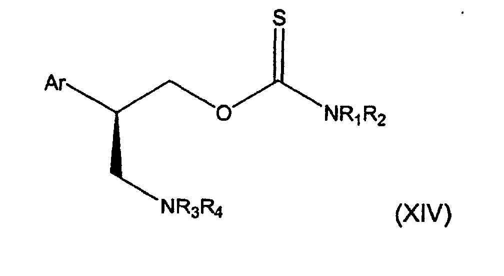

30. The O-Thiocarbamoyl-aminoalkanol compound represented by the structural formula (XIV), in accordance with claim 29, wherein R is hydrogen, and the pharmaceutically acceptable salts thereof.

31. The O-Thiocarbamoyl-aminoalkanol compound represented by the structural formula (XIV), in accordance with claim 29, wherein R is hydrogen, R₁ and R₂ may be same or different from each other and are independently selected from the group consisting of hydrogen, lower alkyl of 1 to 8 carbon atoms, 3 to 7-membered aliphatic cyclic compounds and R₁ and R₂, together with the adjoining N-atom, form a 5 to 7-membered cyclic compound which optionally comprises zero to one additional nitrogen atoms substituted with a member selected from the group consisting of hydrogen, alkyl and aryl groups, or zero to one oxygen atoms directly unconnected, R₃ and R₄ may be same or different from each other and are independently selected from the group consisting of hydrogen, lower alkyl of 1 to 8 carbon atoms, 3 to 7-membered aliphatic cyclic compounds and R₃ and R₄, together with the adjoining N-atom, form a 5 to 7-membered cyclic compound which optionally comprises zero to one additional nitrogen atoms substituted with a member selected from the group consisting of hydrogen, alkyl and aryl groups, or zero to one oxygen atoms directly unconnected, and the pharmaceutically acceptable salts thereof.

32. The O-Thiocarbamoyl-aminoalkanol compound represented by the structural formula (XIV), in accordance with claim 29, wherein x is 1, and the pharmaceutically acceptable salts thereof.

33. An enantiomerically enriched O-Thiocarbamoyl-(L)-aminoalkanol compound according to claim 1 represented by the following structural formula (XV):

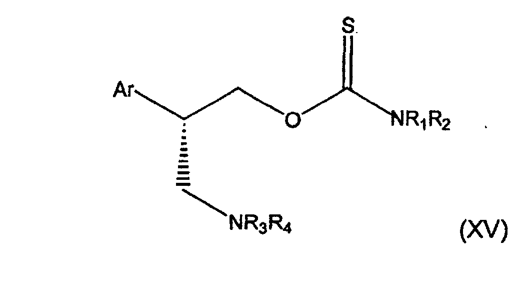

34. The O-Thiocarbamoyl-aminoalkanol compound represented by the structura formula (XV), in accordance with claim 33, wherein R is hydrogen, and the pharmaceutically acceptable salts thereof.

35. The O-Thiocarbamoyl-aminoalkanol compound represented by the structura formula (XV), in accordance with claim 33, wherein R is hydrogen, R₁ and R₂ may be same or different from each other and are independently selected from the group consisting of hydrogen, lower alkyl of 1 to 8 carbon atoms, 3 to 7-membered aliphatic cyclic compounds and R₁ and R₂, together with the adjoining N-atom, form a 5 to 7-membered cyclic compound which optionally comprises zero to one additional nitrogen atoms substituted with a member selected from the group consisting of hydrogen, alkyl and aryl groups, or zero to one oxygen atoms directly unconnected, R₃ and R₄ may be same or different from each other and are independently selected from the group consisting of hydrogen, lower alkyl of 1 to 8 carbon atoms, 3 to 7-membered aliphatic cyclic compounds and R₃ and R₄, together with the adjoining N-atom, form a 5 to 7 membered cyclic compound which optionally comprises zero to one additional nitrogen atoms substituted with a member selected from the group consisting of hydrogen, alky and aryl groups, or zero to one oxygen atoms directly unconnected, and the pharmaceutically acceptable salts thereof.

36. The O-Thiocarbamoyl-aminoalkanol compound represented by the structural formula (XV), in accordance with claim 33, wherein x is 1, and the pharmaceutically acceptable salts thereof.

37. A racemic or enantiomerically enriched O-Thiocarbamoyl-aminoalkanol compound according to claim 1 represented by the following structural formula (XVI):

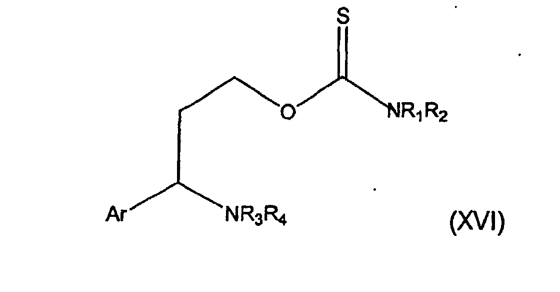

38. The O-Thiocarbamoyl-aminoalkanol compound represented by the structural formula (XVI), in accordance with claim 37, wherein R is hydrogen, and the pharmaceutically acceptable salts thereof.

39. The O-Thiocarbamoyl-aminoalkanol compound represented by the structural formula (XVI), in accordance with claim 37, wherein R is hydrogen, R₁ and R₂ may be same or different from each other and are independently selected from the group consisting of hydrogen, lower alkyl of 1 to 8 carbon atoms, 3 to 7-membered aliphatic cyclic compounds and R₁ and R₂, together with the adjoining N-atom, form a 5 to 7-membered cyclic compound which optionally comprises zero to one additional nitrogen atoms substituted with a member selected from the group consisting of hydrogen, alkyl and aryl groups, or zero to one oxygen atoms directly unconnected, R₃ and R₄ may be same or different from each other and are independently selected from the group consisting of hydrogen, lower alkyl of 1 to 8 carbon atoms, 3 to 7-membered aliphatic cyclic compounds and R₃ and R₄, together with the adjoining N-atom, form a 5 to 7-membered cyclic compound which optionally comprises zero to one additional nitrogen atoms substituted with a member selected from the group consisting of hydrogen, alkyl and aryl groups, or zero to one oxygen atoms directly unconnected, and the pharmaceutically acceptable salts thereof.

40. The O-Thiocarbamoyl-aminoalkanol compound represented by the structural formula (XVI), in accordance with claim 37, wherein x is 1, and the pharmaceutically acceptable salts thereof.

41. An enantiomerically enriched O-Thiocarbamoyl-(D)-aminoalkanol compound according to claim 1 represented by the following structural formula (XVII):

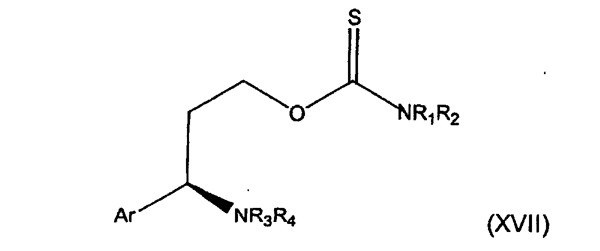

42. The O-Thiocarbamoyl-aminoalkanol compound represented by the structural formula (XVII), in accordance with claim 41, wherein R is hydrogen, and the pharmaceutically acceptable salts thereof.

43. The O-Thiocarbamoyl-aminoalkanol compound represented by the structure l formula (XVII), in accordance with claim 41, wherein R is hydrogen, R₁ and R₂ may be same or different from each other and are independently selected from the group consisting of hydrogen, lower alkyl of 1 to 8 carbon atoms, 3 to 7-membered aliphatic cyclic compounds and R₁ and R₂, together with the adjoining N-atom, form a 5 to 7-membered cyclic compound which optionally comprises zero to one additional nitrogen atoms substituted with a member selected from the group consisting of hydrogen, alkyl and aryl groups, or zero to one oxygen atoms directly unconnected, R₃ and R₄ may be same or different from each other and are independently selected from the group consisting of hydrogen, lower alkyl of 1 to 8 carbon atoms, 3 to 7-membered aliphatic cyclic compounds and R₃ and R₄, together with the adjoining N-atom, form a 5 to 7-membered cyclic compound which bptionally comprises zero to one additional nitrogen atoms substituted with a member selected from the group consisting of hydrogen, alkyl and aryl groups, or zero to one oxygen atoms directly unconnected, and the pharmaceutically acceptable salts thereof.

44. The O-Thiocarbamoyl-aminoalkanol compound represented by the structural formula (XVII), in accordance with claim 41, wherein x is 1, and the pharmaceutically acceptable salts thereof.

45. An enantiomerically enriched O-Thiocarbamoyl-(L)-aminoalkanol compound according to claim 1 represented by the following structural formula (XVIII):

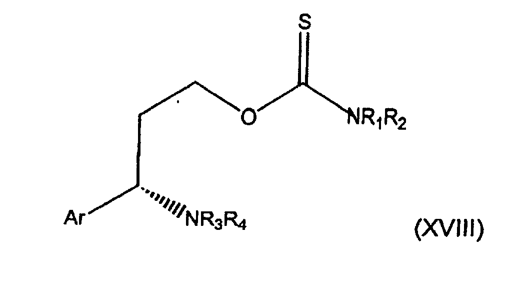

46. The O-Thiocarbamoyl-aminoalkanol compound represented by the structural formula (XVIII), in accordance with claim 45, wherein R is hydrogen, and the pharmaceutically acceptable salts thereof.

47. The O-Thiocarbamoyl-aminoalkanol compound represented by the structural formula (XVIII), in accordance with claim 45, wherein R is hydrogen, R₁ and R₂ may be same or different from each other and are independently selected from the group consisting of hydrogen, lower alkyl of 1 to 8 carbon atoms, 3 to 7-membered aliphatic cyclic compounds and R₁ and R₂, together with the adjoining N-atom, form a 5 to 7-membered cyclic compound which optionally comprises zero to one additional nitrogen atoms substituted with a member selected from the group consisting of hydrogen, alkyl and aryl groups, or zero to one oxygen atoms directly unconnected, R₃ and R₄ may be same or different from each other and are independently selected from the group consisting of hydrogen, lower alkyl of 1 to 8 carbon atoms, 3 to 7-membered aliphatic cyclic compounds and R₃ and R₄, together with the adjoining N-atom, form a 5 to 7-membered cyclic compound which optionally comprises zero to one additional nitrogen atoms substituted with a member selected from the group consisting of hydrogen, alkyl and aryl groups, or zero to one oxygen atoms directly unconnected, and the pharmaceutically acceptable salts thereof.

48. The O-Thiocarbamoyl-aminoalkanol compound represented by the structural formula (XVIII), in accordance with claim 45, wherein x is 1, and the pharmaceutically acceptable salts thereof.

49. A racemic or enantiomerically enriched O-Thiocarbamoyl-aminoalkanol compound according to claim 1 represented by the following structural formula (XIX):

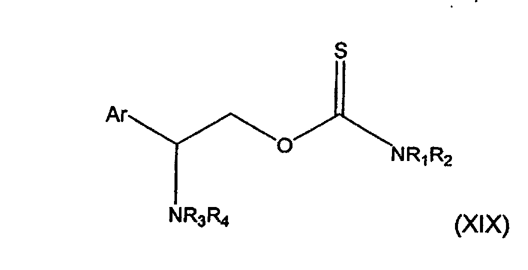

50. The O-Thiocarbamoyl-aminoalkanol compound represented by the structural formula (XIX), in accordance with claim 49, wherein R is hydrogen, and the pharmaceutically acceptable salts thereof.

51. The O-Thiocarbamoyl-aminoalkanol compound represented by the structural formula (XIX), in accordance with claim 49, wherein R is hydrogen, R₁ and R₂ may be same or different from each other and are independently selected from the group consisting of hydrogen, lower alkyl of 1 to 8 carbon atoms, 3 to 7-membered aliphatic cyclic compounds and R₁ and R₂, together with the adjoining N-atom, form a 5 to 7 membered cyclic compound which optionally comprises zero to one additional nitroger atoms substituted with a member selected from the group consisting of hydrogen, alky and aryl groups, or zero to one oxygen atoms directly unconnected, R₃ and R₄ may be same or different from each other and are independently selected from the group consisting of hydrogen, lower alkyl of 1 to 8 carbon atoms, 3 to 7-membered aliphatic cyclic compounds and R₃ and R₄, together with the adjoining N-atom, form a 5 to 7-membered cyclic compound which optionally comprises zero to one additional nitroger atoms substituted with a member selected from the group consisting of hydrogen, alkyl and aryl groups, or zero to one oxygen atoms directly unconnected; and the pharmaceutically acceptable salts thereof.

52. The O-Thiocarbamoyl-aminoalkanol compound represented by the structural formula (XIX), in accordance with claim 49, wherein x is 1, and the pharmaceutically acceptable salts thereof.

53. An enantiomerically enriched O-Thiocarbamoyl-(D)-aminoalkanol compound according to claim 1 represented by the following structural formula (XX):

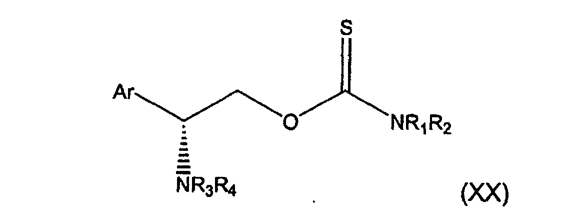

54. The O-Thiocarbamoyl-aminoalkanol compound represented by the structural formula (XX), in accordance with claim 53, wherein R is hydrogen, and the pharmaceutically acceptable salts thereof.

55. The O-Thiocarbamoyl-aminoalkanol compound represented by the structural formula (XX), in accordance with claim 53, wherein R is hydrogen, R₁ and R₂ may be same or different from each other and are independently selected from the group consisting of hydrogen, lower alkyl of 1 to 8 carbon atoms, 3 to 7-membered aliphatic cyclic compounds and R₁ and R₂, together with the adjoining N-atom, form a 5 to 7-membered cyclic compound which optionally comprises zero to one additional nitrogen atoms substituted with a member selected from the group consisting of hydrogen, alkyl and aryl groups, or zero to one oxygen atoms directly unconnected, R₃ and R₄ may be same or different from each other and are independently selected from the group consisting of hydrogen, lower alkyl of 1 to 8 carbon atoms, 3 to 7-membered aliphatic cyclic compounds and R₃ and R₄, together with the adjoining N-atom, form a 5 to 7-membered cyclic compound which optionally comprises zero to one additional nitrogen atoms substituted with a member selected from the group consisting of hydrogen, alkyl and aryl groups, or zero to one oxygen atoms directly unconnected, and the pharmaceutically acceptable salts thereof.

56. The O-Thiocarbamoyl-aminoalkanol compound represented by the structural formula (XX), in accordance with claim 53, wherein x is 1, and the pharmaceutically acceptable salts thereof.

57. An enantiomerically enriched O-Thiocarbamoyl-(L)-aminoalkanol compound according to claim 1 represented by the following structural formula (XXI): 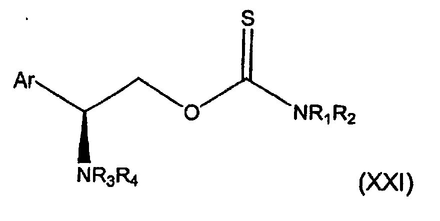 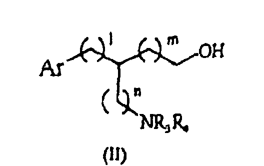 is reacted with Di-*t*-butyl dicarbonate to synthesize N-*t*-butyloxycarbonyl aminoalkanol represented by the formula (III), 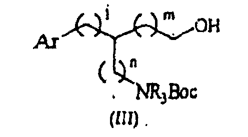 followed by the treatment with sodium hydride, carbon disulfide and methyl iodide in an etheral solution, which is followed by the treatment with an amine compound of formula (IV)
HNR₁R₂ (IV)
to yield O-thiocarbamoyl-N-*t*-butyloxycarbonyl- aminoalkanol represented by general formula(V), 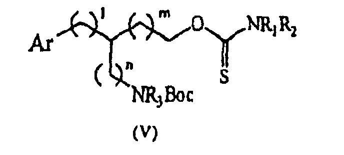 wherein Boc represents t-butyloxy carbonyl radical, which is deprotected by aqueous hydrochloric acid solution to give a O-thiocarbamoyl-aminoalkanol represented by the general formula (VI), 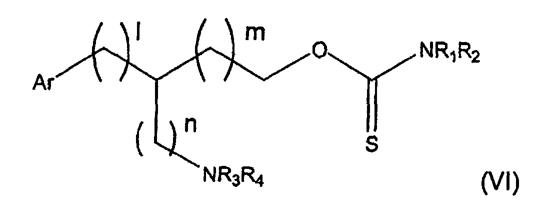 wherein Ar, R₁, R₂, R₃, R₄, l, m and n are as defined above in claim 1 with the proviso that R₃=R₄=H or R₄=H.

58. The O-Thiocarbamoyl-aminoalkanol compound represented by the structural formula (XXI), in accordance with claim 57, wherein R is hydrogen, and the pharmaceutically acceptable salts thereof.

59. The O-Thiocarbamoyl-aminoalkanol compound represented by the structural formula (XXI), in accordance with claim 57, wherein R is hydrogen, R₁ and R₂ may be same or different from each other and are independently selected from the group consisting of hydrogen, lower alkyl of 1 to 8 carbon atoms, 3 to 7-membered aliphatic cyclic compounds and R₁ and R₂, together with the adjoining N-atom, form a 5 to 7-membered cyclic compound which optionally comprises zero to one additional nitrogen atoms substituted with a member selected from the group consisting of hydrogen, alkyl and aryl groups, or zero to one oxygen atoms directly unconnected, R₃ and R₄ may be same or different from each other and are independently selected from the group consisting of hydrogen, lower alkyl of 1 to 8 carbon atoms, 3 to 7-membered aliphatic cyclic compounds and R₃ and R₄, together with the adjoining N-atom, form a 5 to 7-membered cyclic compound which optionally comprises zero to one additional nitrogen atoms substituted with a member selected from the group consisting of hydrogen, alkyl and aryl groups, or zero to one oxygen atoms directly unconnected, and the pharmaceutically acceptable salts thereof.

60. The O-Thiocarbamoyl-aminoalkanol compound represented by the structural formula (XXI), in accordance with claim 57, wherein x is 1, and the pharmaceutically acceptable salts thereof.

61. A method for the preparation of a compound of formula (VI) as defined in claim 1, in which an aminoalkanol of general formula (II)

62. The method of claim 61 wherein without further purification, the compound of formula (VI) may be converted into its pharmaceutically acceptable salts. formula (I)

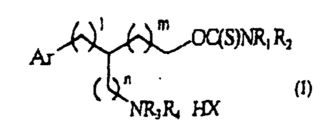

63. A method for the preparation of a compound of formula (VI) wherein as defined in claim 1, in which an aminoalkanol of general formula (II) 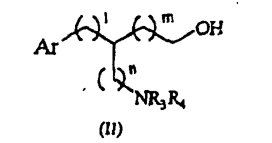 is reacted with sodium hydride, carbon disulfide and methyl iodide in an ethera: solution which is followed by the treatment with an amine compound of formula (IV)
HNR₁R₂ (IV)
to yield O-thiocarbamoyl-aminoalkanol represented by the general formula (VI). 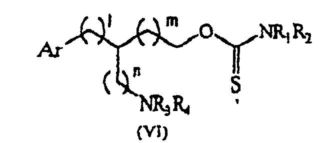 wherein Ar, R₁, R₂, R₃, R₄, l, m and n are as defined above in claim 1 with the proviso that R₃ and R₄ are not hydrogen.

64. The method of claim 63 wherein without further purification, the compound of formula (VI) may be converted into its pharmaceutically acceptable salts, formula (I)

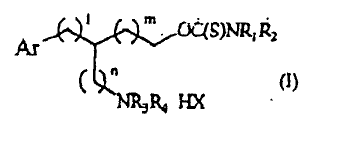

65. A method for the preparation of a compound of formula (VI) as defined in claim 1, in which an aminoalkanol of general formula (II) 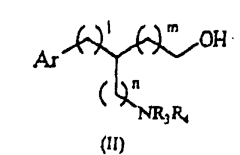 is reacted with Di-*t*-butyl dicarbonate to synthesize N-*t*-butyloxycarbonyl-aminoalkanol represented by the formula (III), 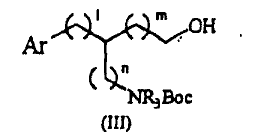 followed by the treatment with isothiocyanate compound of formula (VII)
R₂NCS (VII)
in an halogenated hydrocarbon solution to yield O-thiocarbamoyl-N-*t* -butyloxycarbonyl-aminoalkanol represented by general formula (V), 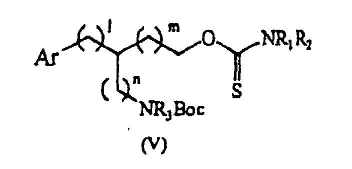 wherein Boc represents t-butyloxy carbonyl radical, which is deprotected by aqueous hydrochloric acid solution to give a O-thiocarbamoyl-aminoalkanol represented by the general formula (VI), 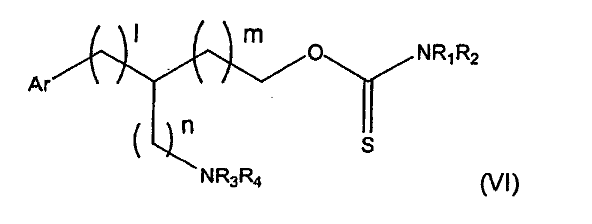 wherein Ar, R₁, R₂, R₃, R₄, l, m and n are as defined above in claim 1 with the proviso that R₁=R₃=R₄=H or R₁=R₄=H.

66. The method of claim 65 wherein without further purification, the compound of formula (VI) may be converted into its pharmaceutically acceptable salts. formula (I)

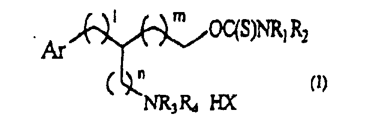

67. A method for the preparation of a compound of formula (VI) wherein as defined in claim 1, in which an aminoalkanol of general formula (II) 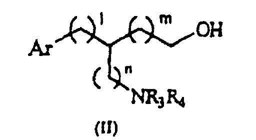 is reacted with isothiocyanate compound of formula (VII)
R₂NCS (VII)
in an halogenated hydrocarbon solution to yield a O-thiocarbamoyl-aminoalkanol represented by the general formula (VI). 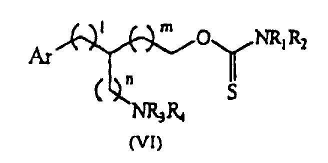 wherein Ar, R₁, R₂, R₃, R₄, l, m and n are as defined above in claim 1 with the proviso that R₁ is hydrogen and R₃ and R₄ are not hydrogen.

68. The method of claim 67, wherein without further purification, the compound of formula (VI) may be converted into its pharmaceutically acceptable salts. formula (I)

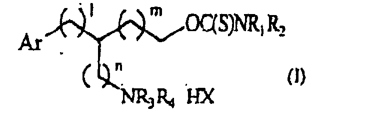

69. A method for the preparation of a compound of formula (VIII) according to claim 61 in which an (D)-aminoalkanol is employed.

70. A method for the preparation of a compound of formula (VIII) according to claim 63 in which an (D)-aminoalkanol is employed

71. A method for the preparation of a compound of formula (VIII) according to claim 65 in which an (D)-aminoalkanol is employed

72. A method for the preparation of a compound of formula (VIII) according to claim 67 in which an (D)-aminoalkanol is employed

73. A method for the preparation of a compound of formula (IX) according to claim 61 in which an (L)-aminoalkanol is employed.

74. A method for the preparation of a compound of formula (VIII) according to claim 63 in which an (L)-aminoalkanol is employed

75. A method for the preparation of a compound of formula (VIII) according to claim 65 in which an (L)-aminoalkanol is employed

76. A method for the preparation of a compound of formula (VIII) according to claim 67 in which an (L)-aminoalkanol is employed

77. A pharmaceutical composition, comprising a compound of formula (VI) or a pharmaceutically acceptable salt thereof as claimed in claime 1, together with a non-toxic pharmaceutically acceptable carrier or diluent therefor.

78. A pharmaceutical composition, comprising a compound of formula (VIII) or a pharmaceutically acceptable salt thereof as claimed in claim 5, together with a non-toxic pharmaceutically acceptable carrier or diluent therefor.

79. A pharmaceutical composition, comprising a compound of formula (IX) or a pharmaceutically acceptable salt thereof as claimed in claim 9, together with a non-toxic pharmaceutically acceptable carrier or diluent therefor.

80. A compound according to claim 1 wherein the compound is selected from O-Thiocarbamoyl-(DL)-phenylalaninol, O-Thiocarbamoyl-(D)-phenylalaninol, and O-Thiocarbamoyl-(L)-phenylalaninol; and non-toxic pharmacologically acceptable salts thereof.

81. A pharmaceutical composition, comprising a compound according to claim 1 wherein the compound is O-Thiocarbamoyl-(D)-phenylalaninol and non-toxic pharmacologically acceptable salts thereof.

82. A pharmaceutical composition, comprising a compound according to claim 1 wherein the compound is O-Thiocarbamoyl-(L)-phenylalaninol and non-toxic pharmacologically acceptable salts thereof.

## Patentansprüche

1. Racemische oder enantiomer angereicherte O-Thiocarbamoyl-aminoalkanol-Verbindung, die durch die folgende Strukturformel (VI) dargestellt wird: 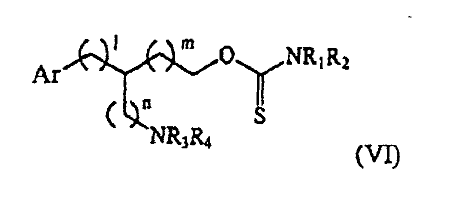 worin Ar eine Phenylgruppe ist, wie sie nachfolgend beschrieben wird: 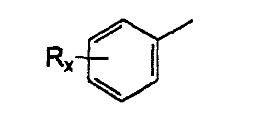 worin R ein Rest ist, der aus der Gruppe, bestehend aus Wasserstoff, Niederalkyl mit 1 bis 8 Kohlenstoffatomen, Halogen, ausgewählt aus F, Cl, Br und I, Alkoxy mit 1 bis 3 Kohlenstoffatomen, Thioalkoxy mit 1 bis 3 Kohlenstoffatomen, Nitro, Hydroxy oder Trifluorkohlenstoff ausgewählt ist und x eine ganze Zahl von 1 bis 3 ist, mit der Maßgabe, dass die Reste R gleich oder voneinander verschieden sind, wenn x 2 oder 3 ist, R₁ und R₂ gleich oder voneinander verschieden sein können und unabhängig voneinander aus der Gruppe, bestehend aus Wasserstoff, Niederalkyl mit 1 bis 8 Kohlenstoffatomen, Aryl, 3- bis 7-gliedrigen aliphatischen cyclischen Verbindungen ausgewählt sind, und R₁ und R₂ zusammen mit dem benachbarten N-Atom eine 5- bis 7-gliedrige cyclische Verbindung bilden, die gegebenenfalls nicht direkt verbunden 0 bis 1 zusätzliches Stickstoffatom, substituiert mit einem Rest, ausgewählt aus der Gruppe, bestehend aus Wasserstoff, Alkyl- und Aryl-gruppen, oder 0 bis 1 Sauerstoffatom umfassen kann, R₃ und R₄ gleich oder voneinander verschieden sein können und jeweils unabhängig voneinander aus der Gruppe, bestehend aus Wasserstoff, Niederalkyl mit 1 bis 8 Kohlenstoffatomen, Aryl, 3- bis 7-gliedrigen aliphatischen cyclischen Verbindungen ausgewählt sind und R₃ und R₄ zusammen mit dem benachbarten N-Atom eine 5- bis 7-gliedrige cyclische Verbindung bilden, die gegebenenfalls nicht direkt verbunden 0 bis 1 zusätzliches Stickstoffatom, substituiert mit einem Rest, ausgewählt aus der Gruppe, bestehend aus Wasserstoff, Alkyl- und Arylgruppen, oder 0 bis 1 Sauerstoffatom umfassen kann, 1, m und n jeweils 0 oder 1 sind,
und die pharmazeutisch annehmbaren Salze davon.

2. O-Thiocarbamoyl-aminoalkanol-Verbindung gemäß Anspruch 1, die durch die Strukturformel (VI) dargestellt wird, wobei R Wasserstoff ist, und die pharmazeutisch annehmbaren Salze davon.

3. O-Thiocarbamoyl-aminoalkanol-Verbindung nach Anspruch 1, die durch die Strukturformel (VI) dargestellt wird, wobei R Wasserstoff ist, R₁ und R₂ gleich oder voneinander verschieden sein können und unabhängig voneinander aus der Gruppe, bestehend aus Wasserstoff, Niederalkyl mit 1 bis 8 Kohlenstoffatomen, 3- bis 7-gliedrigen aliphatischen cyclischen Verbindungen ausgewählt sind und R₁ und R₂ zusammen mit dem benachbarten N-Atom eine 5- bis 7-gliedrige cyclische Verbindung bilden, die gegebenenfalls nicht direkt verbunden 0 bis 1 zusätzliches Stickstoffatom, substituiert mit einem Rest, ausgewählt aus der Gruppe, bestehend aus Wasserstoff, Alkyl- und Arylgruppen, oder 0 bis 1 Sauerstoffatom umfasst, R₃ und R₄ gleich oder voneinander verschieden sein können und unabhängig voneinander aus der Gruppe, bestehend aus Wasserstoff, Niederalkyl mit 1 bis 8 Kohlenstoffatomen, 3- bis 7-gliedrigen aliphatischen cyclischen Verbindungen ausgewählt sind, und R₃ und R₄ zusammen mit dem benachbarten N-Atom eine 5- bis 7-gliedrige cyclische Verbindung bilden, die gegebenenfalls nicht direkt verbunden 0 bis 1 zusätzliches Stickstoffatom, substituiert mit einem Rest, ausgewählt aus der Gruppe, bestehend aus Wasserstoff, Alkyl- und Arylgruppen, oder 0 bis 1 Sauerstoffatom umfasst,
und die pharmazeutisch annehmbaren Salze davon.

4. O-Thiocarbamoyl-aminoalkanol-Verbindung gemäß Anspruch 1, die durch die Strukturformel (VI) dargestellt wird, worin x 1 ist, und die pharmazeutisch annehmbaren Salze davon.

5. Enantiomer angereicherte O-Thiocarbamoyl-(D)-aminoalkanol-Verbindung nach Anspruch 1, die durch die folgende Strukturformel (VIII) dargestellt wird:

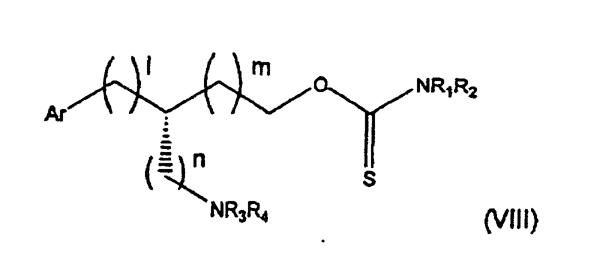

6. O-Thiocarbamoyl-aminoalkanol-Verbindung nach Anspruch 5, die durch die Strukturformel (VIII) dargestellt wird, wobei R Wasserstoff ist, und die pharmazeutisch annehmbaren Salze davon.

7. O-Thiocarbamoyl-aminoalkanol-Verbindung nach Anspruch 5, die durch die Strukturformel (VIII) dargestellt wird, wobei R Wasserstoff ist, R₁ und R₂ gleich oder voneinander verschieden sein können und unabhängig voneinander aus der Gruppe, bestehend aus Wasserstoff, Niederalkyl mit 1 bis 8 Kohlenstoffatomen, 3- bis 7-gliedrigen aliphatischen cyclischen Verbindungen ausgewählt sind und R₁ und R₂ zusammen mit dem benachbarten N-Atom eine 5- bis 7-gliedrige cyclische Verbindung bilden, die gegebenenfalls nicht direkt verbunden 0 bis 1 zusätzliches Stickstoffatom, substituiert mit einem Rest, ausgewählt aus der Gruppe, bestehend aus Wasserstoff, Alkyl- und Aryl-Gruppen, oder 0 bis 1 Sauerstoffatom umfasst, R₃ und R₄ gleich oder voneinander verschieden sein können und unabhängig voneinander aus der Gruppe, bestehend aus Wasserstoff, Niederalkyl mit 1 bis 8 Kohlenstoffatomen, 3- bis 7-gliedrigen aliphatischen cyclischen Verbindungen ausgewählt sind and R₃ und R₄ zusammen mit dem benachbarten N-Atom eine 5- bis 7-gliedrige cyclische Verbindung bilden, die gegebenenfalls nicht direkt verbunden 0 bis 1 zusätzliches Stickstoffatom, substituiert mit einem Rest, ausgewählt aus der Gruppe, bestehend aus Wasserstoff, Alkyl- und Aryl-Gruppen, oder 0 bis 1 Sauerstoffatom umfasst,
und die pharmazeutisch annehmbaren Salze davon.

8. O-Thiocarbamoyl-aminoalkanol-Verbindung gemäß Anspruch 5, die durch die Strukturformel (VIII) dargestellt wird, wobei x 1 ist, und die pharmazeutisch annehmbaren Salze davon.

9. Enantiomer angereicherte O-Thiocarbamoyl-(L)-aminoalkanol-Verbindung gemäß Anspruch 1, die durch die folgende Strukturformel (IX) dargestellt wird:

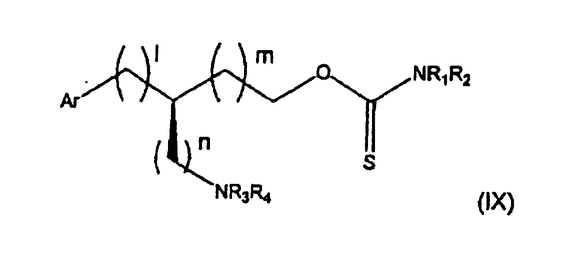

10. O-Thiocarbamoyl-aminoalkanol-Verbindung gemäß Anspruch 9, die durch die Strukturformel (IX) dargestellt wird, wobei R Wasserstoff ist, und die pharmazeutisch annehmbaren Salze davon.

11. O-Thiocarbamoyl-aminoalkanol-Verbindung gemäß Anspruch 9, die durch die Strukturformel (IX) dargestellt wird, wobei R Wasserstoff ist, R₁ und R₂ gleich oder voneinander verschieden sein können und unabhängig voneinander aus der Gruppe, bestehend aus Wasserstoff, Niederalkyl mit 1 bis 8 Kohlenstoffatomen, 3- bis 7-gliedrigen aliphatischen cyclischen Verbindungen ausgewählt sind und R₁ und R₂ zusammen mit dem benachbarten N-Atom eine 5- bis 7-gliedrige cyclische Verbindung bilden, die gegebenenfalls nicht direkt verbunden 0 bis 1 zusätzliches Stickstoffatom, substituiert mit einem Rest, ausgewählt aus der Gruppe, bestehend aus Wasserstoff, Alkyl- und Arylgruppen, oder 0 bis 1 Sauerstoffatom umfasst, R₃ und R₄ gleich oder voneinander verschieden sein können und unabhängig voneinander aus der Gruppe, bestehend aus Wasserstoff, Niederalkyl mit 1 bis 8 Kohlenstoffatomen, 3- bis 7-gliedrigen aliphatischen cyclischen Verbindungen ausgewählt sind, und R₃ and R₄ zusammen mit dem benachbarten N-Atom eine 5- bis 7-gliedrige cyclische Verbindung bilden, die gegebenenfalls nicht direkt verbunden 0 bis 1 zusätzliches Stickstoffatom, substituiert mit einem Rest, ausgewählt aus der Gruppe, bestehend aus Wasserstoff, Alkyl- und Aryl-Gruppen, oder 0 bis 1 Sauerstoffatom umfasst,
und die pharmazeutisch annehmbaren Salze davon.

12. O-Thiocarbamoyl-aminoalkanol-Verbindung gemäß Anspruch 9, die durch die Strukturformel (IX) dargestellt wird, wobei x 1 ist, und die pharmazeutisch annehmbaren Salze davon.

13. Racemische oder enantiomer angereicherte O-Thiocarbamoyl-aminoalkanol-Verbindung gemäß Anspruch 1, die durch die folgende Strukturformel (X) dargestellt wird:

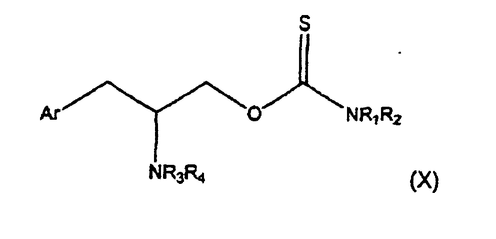

14. O-Thiocarbamoyl-aminoalkanol-Verbindung gemäß Anspruch 13, die durch die Strukturformel (X) dargestellt wird, wobei R Wasserstoff ist, und die pharmazeutisch annehmbaren Salze davon.

15. O-Thiocarbamoyl-aminoalkanol-Verbindung gemäß Anspruch 13, die durch die Strukturformel (X) dargestellt wird, wobei R Wasserstoff ist, R₁ und R₂ gleich oder voneinander verschieden sein können und unabhängig voneinander aus der Gruppe, bestehend aus Wasserstoff, Niederalkyl mit 1 bis 8 Kohlenstoffatomen, 3- bis 7-gliedrigen aliphatischen cyclischen Verbindungen ausgewählt sind, und R₁ und R₂ zusammen mit dem benachbarten N-Atom eine 5- bis 7-gliedrige cyclische Verbindung bilden, die gegebenenfalls nicht direkt verbunden 0 bis 1 zusätaliches Stickstoffatom, substituiert mit einem Rest, ausgewählt aus der Gruppe, bestehend aus Wasserstoff, Alkyl- und Aryl-Gruppen, oder 0 bis 1 Sauerstoffatom umfasst, R₃ und R₄ gleich oder voneinander verschieden sein können und unabhängig voneinander aus der Gruppe, bestehend aus Wasserstoff, Niederalkyl mit 1 bis 8 Kohlenstoffatomen, 3- bis 7-gliedrigen aliphatischen cyclischen Verbindungen ausgewählt sind, und R₃ und R₄ zusammen mit dem benachbarten N-Atom eine 5- bis 7-gliedrige cyclische Verbindung bilden, die gegebenenfalls nicht direkt verbunden 0 bis 1 zusätzliches Stickstoffatom, substituiert mit einem Rest, ausgewählt aus der Gruppe, bestehend aus Wasserstoff, Alkyl- und Aryl-Gruppen, oder 0 bis 1 Sauerstoffatom umfasst,
und die pharmazeutisch annehmbaren Salze davon.

16. O-Thiocarbamoyl-aminoalkanol-Verbindung gemäß Anspruch 13, die durch die Strukturformel (X) dargestellt wird, wobei x 1 ist, und die pharmazeutisch annehmbaren Salze davon.

17. Enantiomer angereicherte O-Thiocarbamoyl-(D)-aminoalkanol-Verbindung gemäß Anspruch 1, die durch die folgende Strukturformel (XI) dargestellt wind:

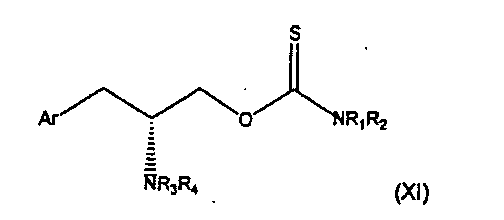

18. O-Thiocarbamoyl-aminoalkanol-Verbindung gemäß Anspruch 17, die durch die Strukturformel (XI) dargestellt wird, wobei R Wasserstoff ist, und die pharmazeutisch annehmbaren Salze davon.

19. O-Thiocarbamoyl-aminoalkanol-Verbindung gemäß Anspruch 17, die durch die Strukturformel (XI) dargestellt wird, wobei R Wasserstoff ist, R₁ und R₂ gleich oder voneinander verschieden sein können und unabhängig voneinander aus der Gruppe, bestehend aus Wasserstoff, Niederalkyl mit 1 bis 8 Kohlenstoffatomen, 3- bis 7-gliedrigen aliphatischen cyclischen Verbindungen ausgewählt sind, und R₁ und R₂ zusammen mit dem benachbarten N-Atom eine 5- bis 7-gliedrige cyclische Verbindung bilden, die gegebenenfalls nicht direkt verbunden 0 bis 1 zusätzliches Stickstoffatom, substituiert mit einem Rest, ausgewählt aus der Gruppe, bestehend aus Wasserstoff, Alkyl- und Aryl-Gruppen, oder 0 bis 1 Sauerstoffatom umfasst, R₃ und R₄ gleich oder voneinander verschieden sein können und unabhängig voneinander aus der Gruppe, bestehend aus Wasserstoff, Niederalkyl mit 1 bis 8 Kohlenstoffatomen, 3- bis 7-gliedrigen aliphatischen cyclischen Verbindungen ausgewählt sind, und R₃ und R₄ zusammen mit dem benachbarten N-Atom eine 5- bis 7-gliedrige cyclische Verbindung bilden, die gegebenenfalls nicht direkt verbunden 0 bis 1 zusätzliches Stickstoffatom, substituiert mit einem Rest, ausgewählt aus der Gruppe, bestehend aus Wasserstoff, Alkyl- und Aryl-Gruppen, oder O bis 1 Sauerstoffatom umfasst,
und die pharmazeutisch annehmbaren Salze davon.

20. O-Thiocarbamoyl-aminoalkanol-Verbindung gemäß Anspruch 17, die durch die Strukturformel (XI) dargestellt wird, wobei x 1 ist, und die pharmazeutisch annehmbaren Salze davon.

21. Enantiomer angereicherte O-Thiocarbamoyl-(L)-aminoalkanol-Verbindung gemäß Anspruch 1, die durch die folgende Strukturformel (XII) dargestellt wird:

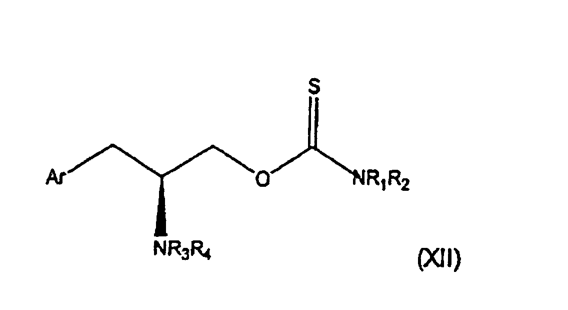

22. O-Thiocarbamoyl-aminoalkanol-Verbindung gemäß Anspruch 21, die durch die Strukturformel (XII) dargestellt wird, wobei R Wasserstoff ist, und die pharmazeutisch annehmbaren Salze davon.

23. O-Thiocarbamoyl-aminoalkanol-Verbindung gemäß Anspruch 21, die durch die Strukturformel (XII) dargestellt wird, wobei R Wasserstoff ist, R₁ und R₂ gleich oder voneinander verschieden sein können und unabhängig voneinander aus der Gruppe, bestehend aus Wasserstoff, Niederalkyl mit 1 bis 8 Kohlenstoffatomen, 3- bis 7-gliedrigen aliphatischen cyclischen Verbindungen ausgewählt sind, und R₁ und R₂ zusammen mit dem benachbarten N-Atom eine 5- bis 7-gliedrige cyclische Verbindung bilden, die gegebenenfalls nicht direkt verbunden 0 bis 1 zusätzliches Stickstoffatom, substituiert mit einem Rest, ausgewählt aus der Gruppe, bestehend aus Wasserstoff, Alkyl- und Aryl-Gruppen, oder 0 bis 1 Sauerstoffatom umfasst, R₃ und R₄ gleich oder voneinander verschieden sein können und unabhängig voneinander aus der Gruppe, bestehend aus Wasserstoff, Niederalkyl mit 1 bis 8 Kohlenstoffatomen, 3- bis 7-gliedrigen aliphatischen cyclischen Verbindungen ausgewählt sind, und R₃ und R₄ zusammen mit dem benachbarten N-Atom eine 5- bis 7-gliedrige cyclische Verbindung bilden, die gegebenenfalls nicht direkt verbunden 0 bis 1 zusätzliches Stickstoffatom, substituiert mit einem Rest, ausgewählt aus der Gruppe, bestehend aus Wasserstoff, Alkyl- und Aryl-Gruppen, oder 0 bis 1 Sauerstoffatom umfasst, und die pharmazeutisch annehmbaren Salze davon.

24. O-Thiocarbamoyl-aminoalkanol-Verbindung gemäß Anspruch 21, die durch die Strukturformel (XII) dargestellt wird, wobei x 1 ist, und die pharmazeutisch annehmbaren Salze davon.

25. Racemische oder enantiomer angereicherte O-Thio-carbamoylaminoalkanol-Verbindung gemäß Anspruch 1, die durch die folgende Strukturformel (XIII) dargestellt wird:

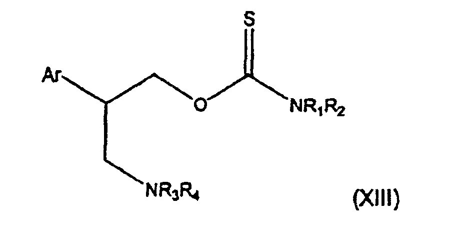

26. O-Thiocarbamoyl-aminoalkanol-Verbindung gemäß Anspruch 25, die durch die Strukturformel (XIII) dargestellt wird, wobei R Wasserstoff ist, und die pharmazeutisch annehmbaren Salze davon.

27. O-Thiocarbamoyl-aminoalkanol-Verbindung gemäß Anspruch 25, die durch die Strukturformel (XIII) dargestellt wird, wobei R Wasserstoff ist, R₁ und R₂ gleich oder voneinander verschieden sein können und unabhängig voneinander aus der Gruppe, bestehend aus Wasserstoff, Niederalkyl mit 1 bis 8 Kohlenstoffatomen, 3- bis 7-gliedrigen aliphatischen cyclischen Verbindungen ausgewählt sind, und R₁ und R₂ zusammen mit dem benachbarten N-Atom eine 5- bis 7-gliedrige cyclische Verbindung bilden, die gegebenenfalls nicht direkt verbunden 0 bis 1 zusätzliches Stickstoffatom, substituiert mit einem Rest, ausgewählt aus der Gruppe, bestehend aus Wasserstoff, Alkyl- und Aryl-Gruppen, oder 0 bis 1 Sauerstoffatom umfasst, R₃ und R₄ gleich oder voneinander verschieden sein können und unabhängig voneinander aus der Gruppe, bestehend aus Wasserstoff, Niederalkyl mit 1 bis 8 Kohlenstoffatomen, 3- bis 7-gliedrigen aliphatischen cyclischen Verbindungen ausgewählt sind, und R₃ und R₄ zusammen mit dem benachbarten N-Atom eine 5- bis 7-gliedrige cyclische Verbindung bilden, die gegebenenfalls nicht direkt verbunden 0 bis 1 zusätzliches Stickstoffatom, substituiert mit einem Rest, ausgewählt aus der Gruppe, bestehend aus Wasserstoff, Alkyl- und Aryl-Gruppen, oder 0 bis 1 Sauerstoffatom umfasst,
und die pharmazeutisch annehmbaren Salze davon.

28. O-Thiocarbamoyl-aminoalkanol-Verbindung gemäß Anspruch 25, die durch die Strukturformel (XIII) dargestellt wird, wobei x 1 ist, und die pharmazeutisch annehmbaren Salze davon.

29. Enantiomer angereicherte O-Thiocarbamoyl-(D)-aminoalkanol-Verbindung gemäß Anspruch 1, die durch die folgende Strukturformel (XIV) dargestellt wird:

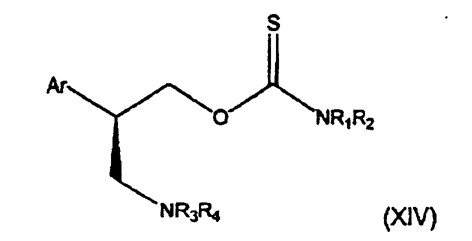

30. O-Thiocarbamoyl-aminoalkanol-Verbindung gemäß Anspruch 29, die durch die Strukturformel (XIV) dargestellt wird, wobei R Wasserstoff ist, und die pharmazeutisch annehmbaren Salze davon.

31. O-Thiocarbamoyl-aminoalkanol-Verbindung gemäß Anspruch 29, die durch die Strukturformel (XIV) dargestellt wird, wobei R Wasserstoff ist, R₁ und R₂ gleich oder voneinander verschieden sein können und unabhängig voneinander aus der Gruppe, bestehend aus Wasserstoff, Niederalkyl mit 1 bis 8 Kohlenstoffatomen, 3- bis 7-gliedrigen aliphatischen cyclischen Verbindungen ausgewählt sind, und R₁ und R₂ zusammen mit dem benachbarten N-Atom eine 5- bis 7-gliedrige cyclische Verbindung bilden, die gegebenenfalls nicht direkt verbunden 0 bis 1 zusätzliches Stickstoffatom, substituiert mit einem Rest, ausgewählt aus der Gruppe, bestehend aus Wasserstoff, Alkyl- und Aryl-Gruppen, oder 0 bis 1 Sauerstoffatom umfasst, R₃ und R₄ gleich oder voneinander verschieden sein können und unabhängig voneinander aus der Gruppe, bestehend aus Wasserstoff, Niederalkyl mit 1 bis 8 Kohlenstoffatomen, 3- bis 7-gliedrigen aliphatischen cyclischen Verbindungen ausgewählt sind, und R₃ und R₄ zusammen mit dem benachbarten N-Atom eine 5- bis 7-gliedrige cyclische Verbindung bilden, die gegebenenfalls nicht direkt verbunden 0 bis 1 zusätzliches Stickstoffatom, substituiert mit einem Rest, ausgewählt aus der Gruppe, bestehend aus Wasserstoff, Alkyl- und Aryl-Gruppen, oder 0 bis 1 Sauerstoffatom umfasst,
und die pharmazeutisch annehmbaren Salze davon.

32. O-Thiocarbamoyl-aminoalkanol-Verbindung gemäß Anspruch 29, die durch die Strukturformel (XIV) dargestellt wird, wobei x 1 ist, und die pharmazeutisch annehmbaren Salze davon.

33. Enantiomer angereicherte O-Thiocarbamoyl-(L)-aminoalkanol-Verbindung gemäß Anspruch 1, die durch die folgende Strukturformel (XV) dargestellt wird:

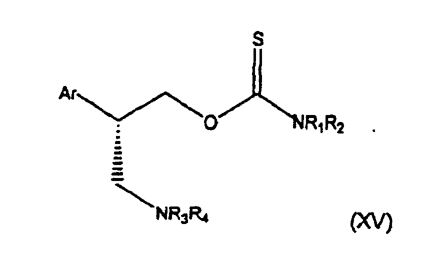

34. O-Thiocarbamoyl-aminoalkanol-Verbindung gemäß Anspruch 33, die durch die Strukturformel (XV) dargestellt wird, wobei R Wasserstoff ist, und die pharmazeutisch annehmbaren Salze davon.

35. O-Thiocarbamoyl-aminoalkanol-Verbindung gemäß Anspruch 33, die durch die Strukturformel(XV) dargestellt wind, wobei R Wasserstoff ist, R₁ und R₂ gleich oder voneinander verschieden sein können und unabhängig voneinander aus der Gruppe, bestehend aus Wasserstoff, Niederalkyl mit 1 bis 8 Kohlenstoffatomen, 3- bis 7-gliedrigen aliphatischen cyclischen Verbindungen ausgewählt sind, und R₁ und R₂ zusammen mit dem benachbarten N-Atom eine 5- bis 7-gliedrige cyclische Verbindung bilden, die gegebenenfalls nicht direkt verbunden 0 bis 1 zusätzliches Stickstoffatom, substituiert mit einem Rest, ausgewählt aus der Gruppe, bestehend aus Wasserstoff, Alkyl- und Aryl-Gruppen, oder 0 bis 1 Sauerstoffatom umfasst, R₃ und R₄ gleich oder voneinander verschieden sein können und unabhängig voneinander aus der Gruppe, bestehend aus Wasserstoff, Niederalkyl mit 1 bis 8 Kohlenstoffatomen, 3- bis 7-gliedrigen aliphatischen cyclischen Verbindungen ausgewählt sind, und R₃ und R₄ zusammen mit dem benachbarten N-Atom eine 5- bis 7-gliedrige cyclische Verbindung bilden, die gegebenenfalls nicht direkt verbunden 0 bis 1 zusätzliches Stickstoffatom, substituiert mit einem Rest, ausgewählt aus der Gruppe, bestehend aus Wasserstoff, Alkyl- und Aryl-Gruppen, oder 0 bis 1 Sauerstoffatom umfasst,
und die pharmazeutisch annehmbaren Salze davon.

36. O-Thiocarbamoyl-aminoalkanol-Verbindung gemäß Anspruch 33, die durch die Strukturformel (XV) dargestellt wird, wobei x 1 ist, und die pharmazeutisch annehmbaren Salze davon.

37. Racemische oder enantiomer angereicherte O-Thio-carbamoylaminoalkanol-Verbindung gemäß Anspruch 1, die durch die folgende Strukturformel (XVI) dargestellt wird:

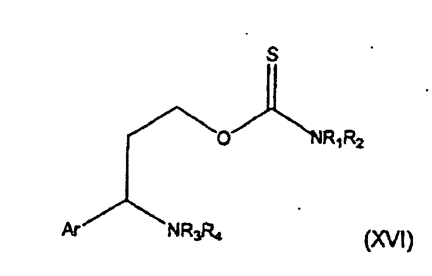

38. O-Thiocarbamoyl-aminoalkanol-Verbindung gemäß Anspruch 37, die durch die Strukturformel (XVI) dargestellt wird, wobei R Wasserstoff ist, und die pharmazeutisch annehmbaren Salze davon.

39. O-Thiocarbamoyl-aminoalkanol-Verbindung gemäß Anspruch 37, die durch die Strukturformel (XVI) dargestellt wird, wobei R Wasserstoff ist, R₁ und R₂ gleich oder voneinander verschieden sein können und unabhängig voneinander aus der Gruppe, bestehend aus Wasserstoff, Niederalkyl mit 1 bis 8 Kohlenstoffatomen, 3- bis 7-gliedrigen aliphatischen cyclischen Verbindungen ausgewählt sind, und R₁ und R₂ zusammen mit dem benachbarten N-Atom eine 5- bis 7-gliedrige cyclische Verbindung bilden, die gegebenenfalls nicht direkt daran gebunden 0 bis 1 zusätzliches Stickstoffatom, substituiert mit einem Rest, ausgewählt aus der Gruppe, bestehend aus Wasserstoff, Alkyl- und Aryl-Gruppen, oder 0 bis 1 Sauerstoffatom umfasst, R₃ und R₄ gleich oder voneinander verschieden sein können und unabhängig voneinander aus der Gruppe, bestehend aus Wasserstoff, Niederalkyl mit 1 bis 8 Kohlenstoffatomen, 3- bis 7-gliedrigen aliphatischen cyclischen Verbindungen ausgewählt sind, und R₃ und R₄ zusammen mit dem benachbarten N-Atom eine 5- bis 7-gliedrige cyclische Verbindung bilden, die gegebenenfalls nicht direkt verbunden 0 bis 1 zusätzliches Stickstoffatom, substituiert mit einem Rest, ausgewählt aus der Gruppe, bestehend aus Wasserstoff, Alkyl- und Aryl-Gruppen, oder 0 bis 1 Sauerstoffatom umfasst,
und die pharmazeutisch annehmbaren Salze davon.

40. O-Thiocarbamoyl-aminoalkanol-Verbindung gemäß Anspruch 37, die durch die Strukturformel (XVI) dargestellt wird, wobei x 1 ist, und die pharmazeutisch annehmbaren Salze davon.

41. Enantiomer angereicherte O-Thiocarbamoyl-(D)-aminoalkanol-Verbindung gemäß Anspruch 1, die durch die folgende Strukturformel (XVII) dargestellt wird:

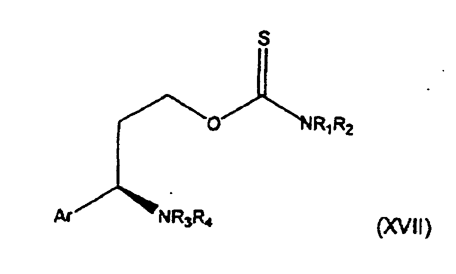

42. O-Thiocarbamoyl-aminoalkanol-Verbindung gemäß Anspruch 41, die durch die Strukturformel (XVII) dargestellt wird, wobei R Wasserstoff ist, und die pharmazeutisch annehmbaren Salze davon.

43. O-Thiocarbamoyl-aminoalkanol-Verbindung gemäß Anspruch 41, die durch die Strukturformel (XVII) dargestellt wird, wobei R Wasserstoff ist, R₁ und R₂ gleich oder voneinander verschieden sein können und unabhängig voneinander aus der Gruppe, bestehend aus Wasserstoff, Niederalkyl mit 1 bis 8 Kohlenstoffatomen, 3- bis 7-gliedrigen aliphatischen cyclischen Verbindungen ausgewählt sind, und R₁ und R₂ zusammen mit dem benachbarten N-Atom eine 5- bis 7-gliedrige cyclische Verbindung bilden, die gegebenenfalls nicht direkt verbunden 0 bis 1 zusätzliches Stickstoffatom, substituiert mit einem Rest, ausgewählt aus der Gruppe, bestehend aus Wasserstoff, Alkyl- und Aryl-Gruppen, oder 0 bis 1 Sauerstoffatom umfasst, R₃ und R₄ gleich oder voneinander verschieden sein können und unabhängig voneinander aus der Gruppe, bestehend aus Wasserstoff, Niederalkyl mit 1 bis 8 Kohlenstoffatomen, 3- bis 7-gliedrigen aliphatischen cyclischen Verbindungen ausgewählt sind, und R₃ und R₄ zusammen mit dem benachbarten N-Atom eine 5- bis 7-gliedrige cyclische Verbindung bilden, die gegebenenfalls nicht direkt verbunden 0 bis 1 zusätzliches Stickstoffatom, substituiert mit einem Rest, ausgewählt aus der Gruppe, bestehend aus Wasserstoff, Alkyl- und Aryl-Gruppen, oder 0 bis 1 Sauerstoffatom umfasst,
und die pharmazeutisch annehmbaren Salze davon.

44. O-Thiocarbamoyl-aminoalkanol-Verbindung gemäß Anspruch 41, die durch die Strukturformel (XVII) dargestellt wird, wobei x 1 ist, und die pharmazeutisch annehmbaren Salze davon.

45. Enantiomer angereicherte O-Thiocarbamoyl-(L)-aminoalkanol-Verbindung gemäß Anspruch 1, die durch die folgende Strukturformel (XVIII) dargestellt wird:

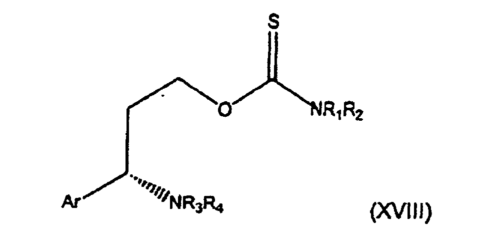

46. O-Thiocarbamoyl-aminoalkanol-Verbindung gemäß Anspruch 45, die durch die Strukturformel (XVIII) dargestellt wird, wobei R Wasserstoff ist, und die pharmazeutisch annehmbaren Salze davon.

47. O-Thiocarbamoyl-aminoalkanol-Verbindung gemäß Anspruch 45, die durch die Strukturformel (XVIII) dargestellt wird, worin R Wasserstoff ist, R₁ und R₂ gleich oder voneinander verschieden sein können und unabhängig voneinander aus der Gruppe, bestehend aus Wasserstoff, Niederalkyl mit 1 bis 8 Kohlenstoffatomen, 3- bis 7-gliedrigen aliphatischen cyclischen Verbindungen ausgewählt sind, und R₁ und R₂ zusammen mit dem benachbarten N-Atom eine 5- bis 7-gliedrige cyclische Verbindung bilden, die gegebenenfalls nicht direkt verbunden 0 bis 1 zusätzliches Stickstoffatom, substituiert mit einem Rest, ausgewählt aus der Gruppe, bestehend aus Wasserstoff, Alkyl- und Aryl-Gruppen, oder 0 bis 1 Sauerstoffatom umfasst, R₃ und R₄ gleich oder voneinander verschieden sein können und unabhängig voneinander aus der Gruppe, bestehend aus Wasserstoff, Niederalkyl mit 1 bis 8 Kohlenstoffatomen, 3- bis 7-gliedrigen aliphatischen cyclischen Verbindungen ausgewählt sind, und R₃ und R₄ zusammen mit dem benachbarten N-Atom eine 5- bis 7-gliedrige cyclische Verbindung bilden, die gegebenenfalls nicht direkt verbunden 0 bis 1 zusätzliches Stickstoffatom, substituiert mit einem Rest, ausgewählt aus der Gruppe, bestehend aus Wasserstoff, Alkyl- und Aryl-Gruppen, oder 0 bis 1 Sauerstoffatom umfasst,
und die pharmazeutisch annehmbaren Salze davon.

48. O-Thiocarbamoyl-aminoalkanol-Verbindung gemäß Anspruch 45, die durch die Strukturformel (XVIII) dargestellt wird, wobei x 1 ist, und die pharmazeutisch annehmbaren Salze davon.

49. Racemische oder enantiomer angereicherte O-Thio-carbamoyl-(L)-aminoalkanol-Verbindung gemäß Anspruch 1, die durch die folgende Strukturformel (XIX) dargestellt wird:

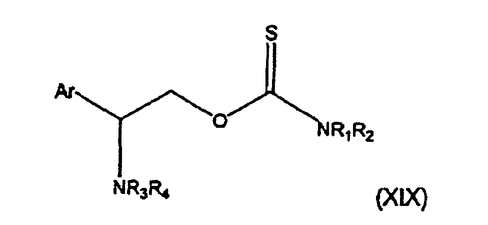

50. O-Thiocarbamoyl-aminoalkanol-Verbindung gemäß Anspruch 49, die durch die Strukturformel (XIX) dargestellt wind, wobei R Wasserstoff ist, und die pharmazeutisch annehmbaren Salze davon.

51. O-Thiocarbamoyl-aminoalkanol-Verbindung gemäß Anspruch 49, die durch die Strukturformel (XIX) dargestellt wird, worin R Wasserstoff ist, R₁ und R₂ gleich oder voneinander verschieden sein können und unabhängig voneinander aus der Gruppe, bestehend aus Wasserstoff, Niederalkyl mit 1 bis 8 Kohlenstoffatomen, 3- bis 7-gliedrigen aliphatischen cyclischen Verbindungen ausgewählt sind, und R₁ und R₂ zusammen mit dem benachbarten N-Atom eine 5- bis 7-gliedrige cyclische Verbindung bilden, die gegebenenfalls nicht direkt verbunden 0 bis 1 zusätzliches Stickstoffatom, substituiert mit einem Rest, ausgewählt aus der Gruppe, bestehend aus Wasserstoff, Alkyl- und Aryl-Gruppen, oder 0 bis 1 Sauerstoffatom umfasst, R₃ und R₄ gleich oder voneinander verschieden sein können und unabhängig voneinander aus der Gruppe, bestehend aus Wasserstoff, Niederalkyl mit 1 bis 8 Kohlenstoffatomen, 3- bis 7-gliedrigen aliphatischen cyclischen Verbindungen ausgewählt sind, und R₃ und R₄ zusammen mit dem benachbarten N-Atom eine 5- bis 7-gliedrige cyclische Verbindung bilden, die gegebenenfalls nicht direkt verbunden 0 bis 1 zusätzliches Stickstoffatom, substituiert mit einem Rest, ausgewählt aus der Gruppe, bestehend aus Wasserstoff, Alkyl- und Aryl-Gruppen, oder 0 bis 1 Sauerstoffatom umfasst,
und die pharmazeutisch annehmbaren Salze davon.

52. O-Thiocarbamoyl-aminoalkanol-Verbindung gemäß Anspruch 49, die durch die Strukturformel (XIX) dargestellt wird, wobei x 1 ist, und die pharmazeutisch annehmbaren Salze davon.

53. Enantiomer angereicherte O-Thiocarbamoyl-(D)-amino-alkanol-Verbindung gemäß Anspruch 1, die durch die folgende Strukturformel (XX) dargestellt wind:

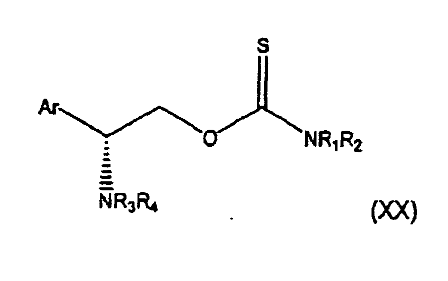

54. O-Thiocarbamoyl-aminoalkanol-Verbindung gemäß Anspruch 53, die durch die Strukturformel (XX) dargestellt wird, wobei R Wasserstoff ist, und die pharmazeutisch annehmbaren Salze davon.

55. O-Thiocarbamoyl-aminoalkanol-Verbindung gemäß Anspruch 53, die durch die Strukturformel (XX) dargestellt wird, worin R Wasserstoff ist, R₁ und R₂ gleich oder voneinander verschieden sein können und unabhängig voneinander aus der Gruppe, bestehend aus Wasserstoff, Niederalkyl mit 1 bis 8 Kohlenstoffatomen, 3- bis 7-gliedrigen aliphatischen cyclischen Verbindungen ausgewählt sind, und R₁ und R₂ zusammen mit dem benachbarten N-Atom eine 5- bis 7-gliedrige cyclische Verbindung bilden, die gegebenenfalls nicht direkt verbunden 0 bis 1 zusätzliches Stickstoffatom, substituiert mit einem Rest, ausgewählt aus der Gruppe, bestehend aus Wasserstoff, Alkyl- und Aryl-Gruppen, oder 0 bis 1 Sauerstoffatom umfasst, R₃ und R₄ gleich oder voneinander verschieden sein können und unabhängig voneinander aus der Gruppe, bestehend aus Wasserstoff, Niederalkyl mit 1 bis 8 Kohlenstoffatomen, 3- bis 7-gliedrigen aliphatischen cyclischen Verbindungen ausgewählt sind, und R₃ und R₄ zusammen mit dem benachbarten N-Atom eine 5- bis 7-gliedrige cyclische Verbindung bilden, die gegebenenfalls nicht direkt verbunden 0 bis 1 zusätzliches Stickstoffatom, substituiert mit einem Rest, ausgewählt aus der Gruppe, bestehend aus Wasserstoff, Alkyl- und Aryl-Gruppen, oder 0 bis 1 Sauerstoffatom umfasst,
und die pharmazeutisch annehmbaren Salze davon.

56. O-Thiocarbamoyl-aminoalkanol-Verbindung gemäß Anspruch 53, die durch die Strukturformel (XX) dargestellt wird, wobei x 1 ist, und die pharmazeutisch annehmbaren Salze davon.

57. Enantiomer angereicherte O-Thiocarbamoyl-(L)-aminoalkanol-Verbindung gemäß Anspruch 1, die durch die folgende Strukturformel (XXI) dargestellt wird:

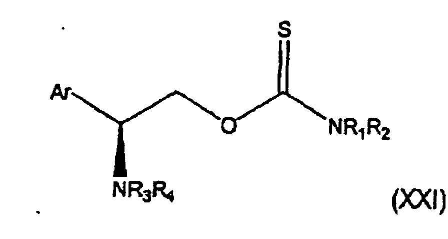

58. O-Thiocarbamoyl-aminoalkanol-Verbindung gemäß Anspruch 57, die durch die Strukturformel (XXI) dargestellt wird, wobei R Wasserstoff ist, und die pharmazeutisch annehmbaren Salze davon.

59. O-Thiocarbamoyl-aminoalkanol-Verbindung gemäß Anspruch 57, die durch die Strukturformel (XXI) dargestellt wird, worin R Wasserstoff ist, R₁ und R₂ gleich oder voneinander verschieden sein können und unabhängig voneinander aus der Gruppe, bestehend aus Wasserstoff, Niederalkyl mit 1 bis 8 Kohlenstoffatomen, 3- bis 7-gliedrigen aliphatischen cyclischen Verbindungen ausgewählt sind, und R₁ und R₂ zusammen mit dem benachbarten N-Atom eine 5- bis 7-gliedrige cyclische Verbindung bilden, die gegebenenfalls nicht direkt verbunden O bis 1 zusätzliches Stickstoffatom, substituiert mit einem Rest, ausgewählt aus der Gruppe, bestehend aus Wasserstoff, Alkyl- und Aryl-Gruppen, oder 0 bis 1 Sauerstoffatom umfasst, R₃ und R₄ gleich oder voneinander verschieden sein können und unabhängig voneinander aus der Gruppe, bestehend aus Wasserstoff, Niederalkyl mit 1 bis 8 Kohlenstoffatomen, 3- bis 7-gliedrigen aliphatischen cyclischen Verbindungen ausgewählt sind, und R₃ und R₄ zusammen mit dem benachbarten N-Atom eine 5- bis 7-gliedrige cyclische Verbindung bilden, die gegebenenfalls nicht direkt verbunden 0 bis 1 zusätzliches Stickstoffatom, substituiert mit einem Rest, ausgewählt aus der Gruppe, bestehend aus Wasserstoff, Alkyl- und Aryl-Gruppen, oder 0 bis 1 Sauerstoffatom umfasst,
und die pharmazeutisch annehmbaren Salze davon.

60. O-Thiocarbamoyl-aminoalkanol-Verbindung gemäß Anspruch 57, die durch die Strukturformel (XXI) dargestellt wird, wobei x 1 ist, und die pharmazeutisch annehmbaren Salze davon.

61. Verfahren zur Herstellung einer Verbindung der Formel (VI), wie sie in Anspruch 1 definiert ist, wobei ein Aminoalkanol der allgemeinen Formel (II) 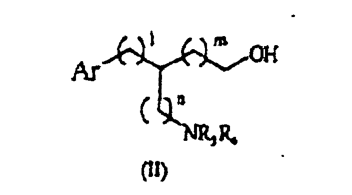 mit Di-t-butyldicarbonat umgesetzt wird, um N-t-Butyl-oxycarbonylaminoalkanol zu synthetisieren, das durch die Formel (III) dargestellt wird 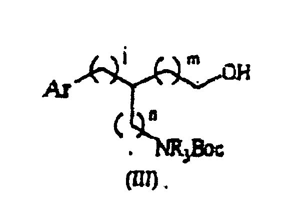 anschließend mit Natriumhydrid, Kohlenstoffdisulfid und Methyliodid in etherischer Lösung behandelt wird, worauf sich die Behandlung mit einer Aminverbindung der Formel (IV) anschließt:
HNR₁R₂ (IV)
wodurch O-Thiocarbamoyl-N-t-butyloxycarbonyl-aminoalkanol erhalten wird, das durch die allgemeine Formel (V) dargestellt wird: 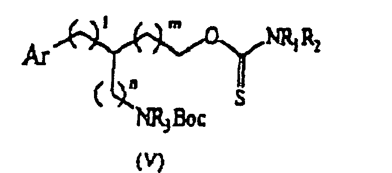 worin Boc für den t-Butyloxycarbonylrest steht, der durch wässrige Salzsäurelösung als Schutzgruppe abgespalten wird, wodurch ein O-Thiocarbamoyl-aminoalkanol erhalten wird, das durch die allgemeine Formel (VI) dargestellt wird: 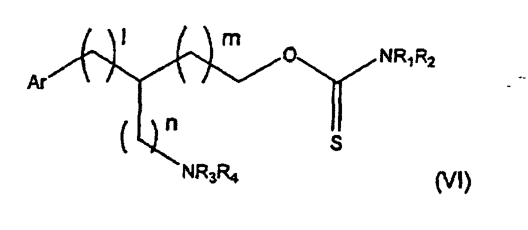 worin Ar, R₁, R₂, R₃, R₄, l, m und n wie oben in Anspruch 1 definiert sind, mit der Maßgabe, dass R₃=R₄=H oder R₄=H.

62. Verfahren nach Anspruch 61, wobei die Verbindung der Formel (VI) ohne weitere Reinigung in ihre pharmazeutisch annehmbaren Salze der Formel (I) übergeführt werden kann:

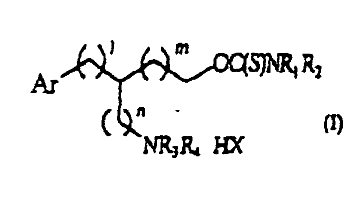

63. Verfahren zur Herstellung einer Verbindung der Formel (VI), wie sie in Anspruch 1 definiert ist, wobei ein Aminoalkanol der allgemeinen Formel (II): 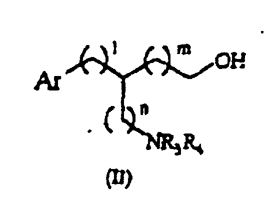 mit Natriumhydrid, Kohlendisulfid und Methyliodid in etherischer Lösung umgesetzt wird, worauf sich die Behandlung mit einer Aminverbindung der Formel (IV) anschließt:
HNR₁R₂ (IV)
wodurch O-Thiocarbamoyl-aminoalkanol, das durch die allgemeine Formel (VI) dargestellt wird, erhalten wird: 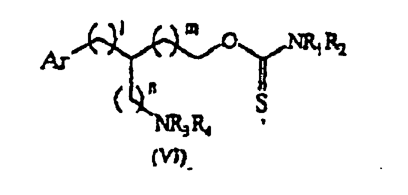 worin Ar, R₁, R₂, R₃, R₄, l, m und n wie oben in Anspruch 1 definiert sind, mit der Maßgabe, dass R₃ und R₄ nicht Wasserstoff sind.

64. Verfahren nach Anspruch 63, wobei die Verbindung der Formel (VI) ohne weitere Reinigung in ihr pharmazeutisch annehmbares Salz der Formel (I) umgewandelt werden kann:

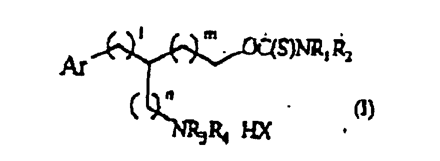

65. Verfahren zur Herstellung einer Verbindung der Formel (VI), wie sie in Anspruch 1 definiert ist, wobei ein Aminoalkanol der allgemeinen Formel (II): 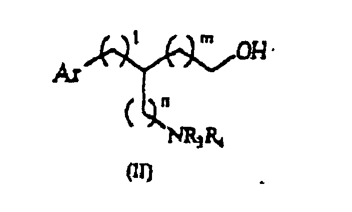 mit Di-t-Butyldicarbonat umgesetzt wird, um N-t-Butyl-oxycarbonylaminoalkanol zu synthetisieren, das durch die Formel (III) dargestellt wird: 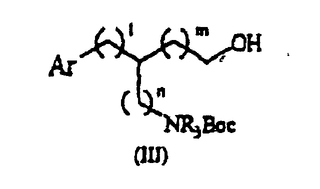 worauf sich die Behandlung mit einer Isothiocyanat-Verbindung der Formel (VII)
R₂NCS (VII)
in Lösung in einem halogenierten Kohlenwasserstoff anschließt, wodurch O-Thiocarbamoyl-N-t-butyloxycarbonyl-aminoalkanol erhalten wird, das durch die allgemeine Formel (V) dargestellt wird: 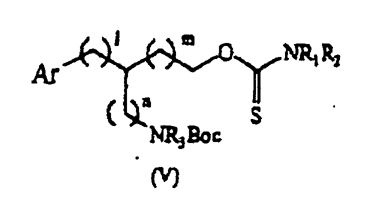 worin Boc den t-Butyloxycarbonylrest darstellt, der durch wässrige Salzsäurelösung als Schutzgruppe abgespalten wird, wodurch ein O-Thiocarbamoyl-aminoalkanol erhalten wird, das durch die allgemeine Formel (VI) dargestellt wird: 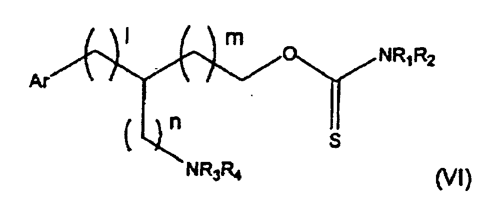 worin Ar, R₁, R₂, R₃, R₄, l, m und n wie oben in Anspruch 1 definiert sind, mit der Maßgabe, dass R₁=R₃=R₄=H oder R₁=R₄=H.

66. Verfahren nach Anspruch 65, wobei die Verbindung der Formel (VI) ohne weitere Reinigung in ihre pharmazeutisch annehmbare Salze der Formel (I) umgewandelt werden kann:

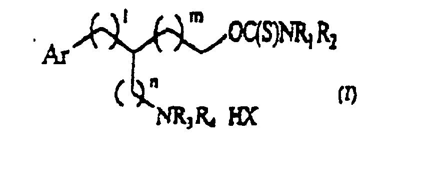

67. Verfahren zur Herstellung einer Verbindung der Formel (VI), wie sie in Anspruch 1 definiert ist, wobei ein Aminoalkanol der allgemeinen Formel (II): 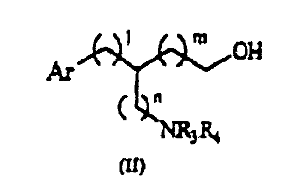 mit einer Isothiocyanat-Verbindung der Formel (VII)
R₂NCS (VII)
in Lösung in einem halogenierten Kohlenwasserstoff umgesetzt wird, wodurch ein O-Thiocarbamoyl-aminoalkanol erhalten wird, das durch die allgemeine Formel (VI) dargestellt wird: 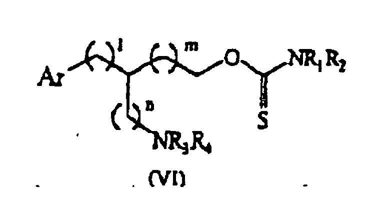 worin Ar, R₁, R₂, R₃, R₄, l, m und n wie oben in Anspruch 1 definiert sind, mit der Maßgabe, dass R₁ Wasserstoff ist und R₃ und R₄ nicht Wasserstoff sind.

68. Verfahren nach Anspruch 67, wobei die Verbindung der Formel (VI) ohne weitere Reinigung in ihre pharmazeutisch annehmbare Salze der Formel (I) übergeführt werden kann:

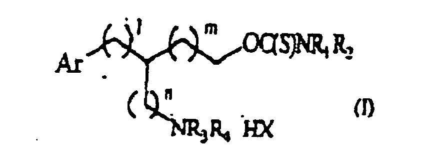

69. Verfahren zur Herstellung einer Verbindung der Formel (VIII) gemäß Anspruch 61, bei dem ein (D)-Amino-alkanol verwendet wird.

70. Verfahren zur Herstellung einer Verbindung der Formel (VIII) gemäß Anspruch 63, wobei ein (D)-Aminoalkanol verwendet wird.

71. Verfahren zur Herstellung einer Verbindung der Formel (VIII) gemäß Anspruch 65, wobei ein (D)-Aminoalkanol verwendet wird.

72. Verfahren zur Herstellung einer Verbindung der Formel (VIII) gemäß Anspruch 67, wobei ein (D)-Aminoalkanol verwendet wird.

73. Verfahren zur Herstellung einer Verbindung der Formel (IX) gemäß Anspruch 61, wobei ein (L)-Aminoalkanol verwendet wird.

74. Verfahren zur Herstellung einer Verbindung der Formel (VIII) gemäß Anspruch 63, wobei ein (L)-Aminoalkanol verwendet wird.

75. Verfahren zur Herstellung einer Verbindung der Formel (VIII) gemäß Anspruch 65, wobei ein (L)-Aminoalkanol verwendet wird.

76. Verfahren zur Herstellung einer Verbindung der Formel (VIII) gemäß Anspruch 67, wobei ein (L)-Aminoalkanol verwendet wird.

77. Pharmazeutische Zusammensetzung, die eine Verbindung der Formel (VI) oder ein pharmazeutisch annehmbares Salz davon gemäß Anspruch 1 zusammen mit einem nicht-toxischen pharmazeutisch annehmbaren Träger oder Verdünnungsmittel dafür umfasst.

78. Pharmazeutische Zusammensetzung, die eine Verbindung der Formel (VIII) oder ein pharmazeutisch annehmbares Salz davon nach Anspruch 5 zusammen mit einem nicht-toxischen pharmazeutisch annehmbaren Träger oder Verdünnungsmittel dafür umfasst.

79. Pharmazeutische Zusammensetzung, die eine Verbindung der Formel (IX) oder ein pharmazeutisch annehmbares Salz davon nach Anspruch 9 zusammen mit einem nicht-toxischen pharmazeutisch annehmbaren Träger oder Verdünnungsmittel dafür umfasst.

80. Verbindung nach Anspruch 1, wobei die Verbindung aus O-Thiocarbamoyl-(DL)-phenylalaninol, O-Thiocarbamoyl-(D)-phenylalaninol und O-Thiocarbamoyl-(L)-phenylalaninol und nicht-toxischen pharmakologisch annehmbaren Salzen davon ausgewählt ist.

81. Pharmazeutische Zusammensetzung, die eine Verbindung nach Anspruch 1 umfasst, wobei die Verbindung O-Thio-carbamoyl-(D)-phenylalaninol und nicht-toxische pharmakologisch annehmbare Salze davon ist.

82. Pharmazeutische Zusammensetzung, die eine Verbindung nach Anspruch 1 umfasst, wobei die Verbindung O-Thio-carbamoyl-(L)-phenylalaninol und nicht-toxische pharmakologisch annehmbare Salze davon ist.

## Revendications

1. Composé O-thiocarbamoyl-aminoalcanol racémique ou enrichi en un énantiomère, représenté par la formule développée suivante (VI): 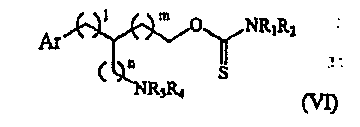 dans lequel Ar est un groupe phényle décrit de la manière suivante : 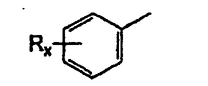 dans lequel R est un membre sélectionné dans le groupe constitué d'un atome d'hydrogène, d'un groupe alkyle inférieur de 1 à 8 atomes de carbone, d'un halogène sélectionné parmi F, Cl, Br et I, d'un alcoxy contenant 1 à 3 atomes de carbone, d'un thioalcoxy contenant 1 à 3 atomes de carbone, d'un groupe nitro, hydroxy ou trifluorocarbone, et x est un entier compris entre 1 et 3, R étant identique ou différent lorsque x vaut 2 ou 3 ; R₁ et R₂ peuvent être identiques ou différents l'un de l'autre et sont sélectionnés indépendamment parmi le groupe constitué d'un atome d'hydrogène, d'un groupe alkyle inférieur de 1 à 8 atomes de carbone, d'un aryle, de composés cycliques aliphatiques de 3 à 7 atomes, de R₁ et R₂, ainsi que l'atome N voisin, formant conjointement un composé cyclique de 5 à 7 atomes qui comprend facultativement 0 à 1 atome d'azote supplémentaire substitué par un membre sélectionné dans le groupe constitué d'un atome d'hydrogène, de groupes alkyles et aryles, ou 0 à 1 atome d'oxygène non directement lié ; R₃ et R₄ peuvent être identiques ou différents l'un de l'autre et sont sélectionnés indépendamment parmi le groupe constitué d'un atome d'hydrogène, d'un groupe alkyle inférieur de 1 à 8 atomes de carbone, d'un aryle, de composés cycliques aliphatiques de 3 à 7 atomes, et de R₃ et R₄, ainsi que l'atome N voisin, formant conjointement un composé cyclique de 5 à 7 atomes qui comprend facultativement 0 à 1 atome d'azote supplémentaire substitué par un membre sélectionné dans le groupe constitué d'un atome d'hydrogène, de groupes alkyles et aryles, ou 0 à 1 atome d'oxygène non directement lié ; 1, m et n valant chacun 0 ou 1 ; et les sels pharmaceutiquement acceptables dudit composé.

2. Composé O-thiocarbamoyl-aminoalcanol représenté par la formule développée (VI), selon la revendication 1, dans lequel R est l'hydrogène ; et les sels pharmaceutiquement acceptables dudit composé.

3. Composé O-thiocarbamoyl-aminoalcanol représenté par la formule développée (VI), selon la revendication 1, dans lequel R est l'hydrogène, R₁ et R₂ peuvent être identiques ou différents l'un de l'autre et sont sélectionnés indépendamment parmi le groupe constitué d'un atome d'hydrogène, d'un groupe alkyle inférieur de 1 à 8 atomes de carbone, de composés cycliques aliphatiques de 3 à 7 atomes et de R₁ et R₂, ainsi que l'atome N voisin, formant conjointement un composé cyclique de 5 à 7 atomes qui comprend facultativement 0 à 1 atome d'azote supplémentaire substitué par un membre sélectionné dans le groupe constitué d'un atome d'hydrogène, de groupes alkyles et aryles, ou 0 à 1 atome d'oxygène non directement lié ; R₃ et R₄ peuvent être identiques ou différents l'un de l'autre et sont sélectionnés indépendamment parmi le groupe constitué d'un atome d'hydrogène, d'un groupe alkyle inférieur de 1 à 8 atomes de carbone, de composés cycliques aliphatiques de 3 à 7 atomes et de R₃ et R₄, ainsi que l'atome N voisin, formant conjointement un composé cyclique de 5 à 7 atomes qui comprend facultativement 0 à 1 atome d'azote supplémentaire substitué par un membre sélectionné dans le groupe constitué d'un atome d'hydrogène, de groupes alkyles et aryles, ou 0 à 1 atome d'oxygène non directement lié ; et les sels pharmaceutiquement acceptables dudit composé.

4. Composé O-thiocarbamoyl-aminoalcanol représenté par la formule développée (VI), selon la revendication 1, dans lequel x vaut 1 ; et les sels pharmaceutiquement acceptables dudit composé.

5. Composé O-thiocarbamoyl-(D)-aminoalcanol enrichi en un énantiomère selon la revendication 1, représenté par la formule développée suivante (VIII):

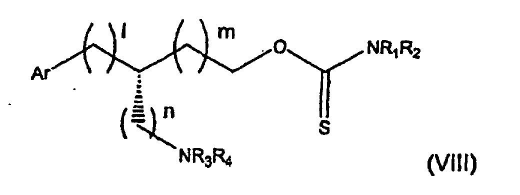

6. Composé O-thiocarbamoyl-aminoalcanol représenté par la formule développée (VIII), selon la revendication 5, dans lequel R est l'hydrogène ; et les sels pharmaceutiquement acceptables dudit composé.

7. Composé O-thiocarbamoyl-aminoalcanol représenté par la formule développée (VIII), selon la revendication 5, dans lequel R est l'hydrogène, R₁ et R₂ peuvent être identiques ou différents l'un de l'autre et sont sélectionnés indépendamment parmi le groupe constitué d'un atome d'hydrogène, d'un groupe alkyle inférieur de 1 à 8 atomes de carbone, de composés cycliques aliphatiques de 3 à 7 atomes et de R₁ et R₂, ainsi que l'atome N voisin, formant conjointement un composé cyclique de 5 à 7 atomes qui comprend facultativement 0 à 1 atome d'azote supplémentaire substitué par un membre sélectionné dans le groupe constitué d'un atome d'hydrogène, de groupes alkyles et aryles, ou 0 à 1 atome d'oxygène non directement lié ; R₃ et R₄ peuvent être identiques ou différents l'un de l'autre et sont sélectionnés indépendamment parmi le groupe constitué d'un atome d'hydrogène, d'un groupe alkyle inférieur de 1 à 8 atomes de carbone, de composés cycliques aliphatiques de 3 à 7 atomes et de R₃ et R₄, ainsi que l'atome N voisin, formant conjointement un composé cyclique de 5 à 7 atomes qui comprend facultativement 0 à 1 atome d'azote supplémentaire substitué par un membre sélectionné dans le groupe constitué d'un atome d'hydrogène, de groupes alkyles et aryles, ou 0 à 1 atome d'oxygène non directement lié ; et les sels pharmaceutiquement acceptables dudit composé.

8. Composé O-thiocarbamoyl-aminoalcanol représenté par la formule développée (VIII), selon la revendication 5, dans lequel x vaut 1 ; et les sels pharmaceutiquement acceptables dudit composé.

9. Composé O-thiocarbamoyl-(L)-aminoalcanol enrichi en un énantiomère selon la revendication 1, représenté par la formule développée suivante (IX) :

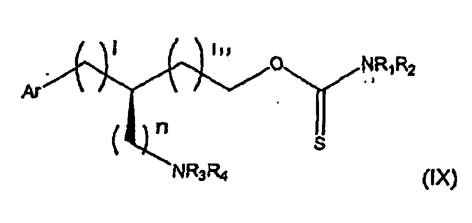

10. Composé O-thiocarbamoyl-aminoalcanol représenté par la formule développée (IX), selon la revendication 9, dans lequel R est l'hydrogène ; et les sels pharmaceutiquement acceptables dudit composé.

11. Composé O-thiocarbamoyl-aminoalcanol représenté par la formule développée (IX), selon la revendication 9, dans lequel R est l'hydrogène, R₁ et R₂ peuvent être identiques ou différents l'un de l'autre et sont sélectionnés indépendamment parmi le groupe constitué d'un atome d'hydrogène, d'un groupe alkyle inférieur de 1 à 8 atomes de carbone, de composés cycliques aliphatiques de 3 à 7 atomes et de R₁ et R₂, ainsi que l'atome N voisin, formant conjointement un composé cyclique de 5 à 7 atomes qui comprend facultativement 0 à 1 atome d'azote supplémentaire substitué par un membre sélectionné dans le groupe constitué d'un atome d'hydrogène, de groupes alkyles et aryles, ou 0 à 1 atome d'oxygène non directement lié ; R₃ et R₄ peuvent être identiques ou différents l'un de l'autre et sont sélectionnés indépendamment parmi le groupe constitué d'un atome d'hydrogène, d'un groupe alkyle inférieur de 1 à 8 atomes de carbone, de composés cycliques aliphatiques de 3 à 7 atomes et de R₃ et R₄, ainsi que l'atome N voisin, formant conjointement un composé cyclique de 5 à 7 atomes qui comprend facultativement 0 à 1 atome d'azote supplémentaire substitué par un membre sélectionné dans le groupe constitué d'un atome d'hydrogène, de groupes alkyles et aryles, ou 0 à 1 atome d'oxygène non directement lié ; et les sels pharmaceutiquement acceptables dudit composé.

12. Composé O-thiocarbamoyl-aminoalcanol représenté par la formule développée (IX), selon la revendication 9, dans lequel x vaut I ; et les sels pharmaceutiquement acceptables dudit composé.

13. Composé O-thiocarbamoyl-aminoalcanol racémique ou enrichi en un énantiomère selon la revendication 1, représenté par la formule développée suivante

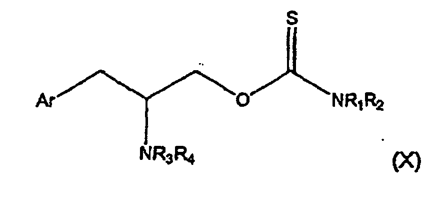

14. Composé O-thiocarbamoyl-aminoalcanol représenté par la formule développée (X), selon la revendication 13, dans lequel R est l'hydrogène ; et les sels pharmaceutiquement acceptables dudit composé.

15. Composé O-thiocarbamoyl-aminoalcanol représenté par la formule développée (X), selon la revendication 13, dans lequel R est l'hydrogène, R₁ et R₂ peuvent être identiques ou différents l'un de l'autre et sont sélectionnés indépendamment parmi le groupe constitué d'un atome d'hydrogène, d'un groupe alkyle inférieur de 1 à 8 atomes de carbone, de composés cycliques aliphatiques de 3 à 7 atomes et de R₁ et R₂, ainsi que l'atome N voisin, formant conjointement un composé cyclique de 5 à 7 atomes qui comprend facultativement 0 à 1 atome d'azote supplémentaire substitué par un membre sélectionné dans le groupe constitué d'un atome d'hydrogène, de groupes alkyles et aryles, ou 0 à 1 atome d'oxygène non directement lié ; R₃ et R₄ peuvent être identiques ou différents l'un de l'autre et sont sélectionnés indépendamment parmi le groupe constitué d'un atome d'hydrogène, d'un groupe alkyle inférieur de 1 à 8 atomes de carbone, de composés cycliques aliphatiques de 3 à 7 atomes et de R₃ et R₄, ainsi que l'atome N voisin, formant conjointement un composé cyclique de 5 à 7 atomes qui comprend facultativement 0 à 1 atome d'azote supplémentaire substitué par un membre sélectionné dans le groupe constitué d'un atome d'hydrogène, de groupes alkyles et aryles, ou 0 à 1 atome d'oxygène non directement lié ; et les sels pharmaceutiquement acceptables dudit composé.

16. Composé O-thiocarbamoyl-aminoalcanol représenté par la formule développée (X), selon la revendication 13, dans lequel x vaut 1 ; et les sels pharmaceutiquement acceptables dudit composé.

17. Composé O-thiocarbamoyl-(D)-aminoalcanol enrichi en un énantiomère selon la revendication 1, représenté par la formule développée suivante (XI):

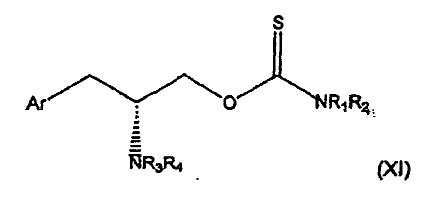

18. Composé O-thiocarbamoyl-aminoalcanol représenté par la formule développée (XI), selon la revendication 17, dans lequel R est l'hydrogène ; et les sels pharmaceutiquement acceptables dudit composé.

19. Composé O-thiocarbamoyl-aminoalcanol représenté par la formule développée (XI), selon la revendication 17, dans lequel R est l'hydrogène, R₁ et R₂ peuvent être identiques ou différents l'un de l'autre et sont sélectionnés indépendamment parmi le groupe constitué d'un atome d'hydrogène, d'un groupe alkyle inférieur de 1 à 8 atomes de carbone, de composés cycliques aliphatiques de 3 à 7 atomes et de R₁ et R₂, ainsi que l'atome N voisin, formant conjointement un composé cyclique de 5 à 7 atomes qui comprend facultativement 0 à 1 atome d'azote supplémentaire substitué par un membre sélectionné dans le groupe constitué d'un atome d'hydrogène, de groupes alkyles et aryles, ou 0 à 1 atome d'oxygène non directement lié ; R₃ et R₄ peuvent être identiques ou différents l'un de l'autre et sont sélectionnés indépendamment parmi le groupe constitué d'un atome d'hydrogène, d'un groupe alkyle inférieur de 1 à 8 atomes de carbone, de composés cycliques aliphatiques de 3 à 7 atomes et de R₃ et R₄, ainsi que l'atome N voisin, formant conjointement un composé cyclique de 5 à 7 atomes qui comprend facultativement 0 à 1 atome d'azote supplémentaire substitué par un membre sélectionné dans le groupe constitué d'un atome d'hydrogène, de groupes alkyles et aryles, ou 0 à 1 atome d'oxygène non directement lié ; et les sels pharmaceutiquement acceptables dudit composé.

20. Composé O-thiocarbamoyl-aminoalcanol représenté par la formule développée (XI), selon la revendication 17, dans lequel x vaut 1; et les sels pharmaceutiquement acceptables dudit composé.

21. Composé O-thiocarbamoyl-(L)-aminoalcanol enrichi en un énantiomère selon la revendication 1, représenté par la formule développée suivante (XII):

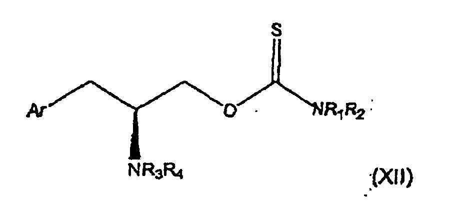

22. Composé O-thiocarbamoyl-aminoalcanol représenté par la formule développée (XII), selon la revendication 21, dans lequel R est l'hydrogène ; et les sels pharmaceutiquement acceptables dudit composé.

23. Composé O-thiocarbamoyl-aminoalcanol représenté par la formule développée (XII), selon la revendication 21, dans lequel R est l'hydrogène, R₁ et R₂ peuvent être identiques ou différents l'un de l'autre et sont sélectionnés indépendamment parmi le groupe constitué d'un atome d'hydrogène, d'un groupe alkyle inférieur de 1 à 8 atomes de carbone, de composés cycliques aliphatiques de 3 à 7 atomes et de R₁ et R₂, ainsi que l'atome N voisin, formant conjointement un composé cyclique de 5 à 7 atomes qui comprend facultativement 0 à 1 atome d'azote supplémentaire substitué par un membre sélectionné dans le groupe constitué d'un atome d'hydrogène, de groupes alkyles et aryles, ou 0 à 1 atome d'oxygène non directement lié ; R₃ et R₄ peuvent être identiques ou différents l'un de l'autre et sont sélectionnés indépendamment parmi le groupe constitué d'un atome d'hydrogène, d'un groupe alkyle inférieur de 1 à 8 atomes de carbone, de composés cycliques aliphatiques de 3 à 7 atomes et de R₃ et R₄, ainsi que l'atome N voisin, formant conjointement un composé cyclique de 5 à 7 atomes qui comprend facultativement 0 à 1 atome d'azote supplémentaire substitué par un membre sélectionné dans le groupe constitué d'un atome d'hydrogène, de groupes alkyles et aryles, ou 0 à 1 atome d'oxygène non directement lié ; et les sels pharmaceutiquement acceptables dudit composé.

24. Composé O-thiocarbamoyl-aminoalcanol représenté par la formule développée (XII), selon la revendication 21, dans lequel x vaut 1 ; et les sels pharmaceutiquement acceptables dudit composé.

25. Composé O-thiocarbamoyl-aminoalcanol racémique enrichi en un énantiomère selon la revendication 1, représenté par la formule développée suivante (XIII):

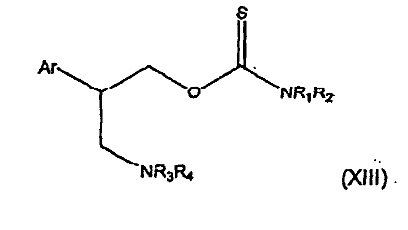

26. Composé O-thiocarbamoyl-aminoalcanol représenté par la formule développée (XIII), selon la revendication 25, dans lequel R est l'hydrogène ; et les sels pharmaceutiquement acceptables dudit composé.

27. Composé O-thiocarbamoyl-aminoalcanol représenté par la formule développée (XIII), selon la revendication 25, dans lequel R est l'hydrogène, R₁ et R₂ peuvent être identiques ou différents l'un de l'autre et sont sélectionnés indépendamment parmi le groupe constitué d'un atome d'hydrogène, d'un groupe alkyle inférieur de 1 à 8 atomes de carbone, de composés cycliques aliphatiques de 3 à 7 atomes et de R₁ et R₂, ainsi que l'atome N voisin, formant conjointement un composé cyclique de 5 à 7 atomes qui comprend facultativement 0 à 1 atome d'azote supplémentaire substitué par un membre sélectionné dans le groupe constitué d'un atome d'hydrogène, de groupes alkyles et aryles, ou 0 à 1 atome d'oxygène non directement lié ; R₃ et R₄ peuvent être identiques ou différents l'un de l'autre et sont sélectionnés indépendamment parmi le groupe constitué d'un atome d'hydrogène, d'un groupe alkyle inférieur de 1 à 8 atomes de carbone, de composés cycliques aliphatiques de 3 à 7 atomes et de R₃ et R₄, ainsi que l'atome N voisin, formant conjointement un composé cyclique de 5 à 7 atomes qui comprend facultativement 0 à 1 atome d'azote supplémentaire substitué par un membre sélectionné dans le groupe constitué d'un atome d'hydrogène, de groupes alkyles et aryles, ou 0 à 1 atome d'oxygène non directement lié ; et les sels pharmaceutiquement acceptables dudit composé.

28. Composé O-thiocarbamoyl-aminoalcanol représenté par la formule développée (XIII), selon la revendication 25, dans lequel x vaut 1 ; et les sels pharmaceutiquement acceptables dudit composé.

29. Composé O-thiocarbamoyl-(D)-aminoalcanol enrichi en un énantiomère selon la revendication 1, représenté par la formule développée suivante (XIV):

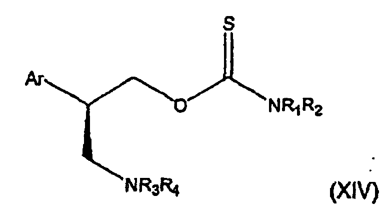

30. Composé O-thiocarbamoyl-aminoalcanol représenté par la formule développée (XIV), selon la revendication 29, dans lequel R est l'hydrogène ; et les sels pharmaceutiquement acceptables dudit composé.

31. Composé O-thiocarbamoyl-aminoalcanol représenté par la formule développée (XIV), selon la revendication 29, dans lequel R est l'hydrogène, R₁ et R₂ peuvent être identiques ou différents l'un de l'autre et sont sélectionnés indépendamment parmi le groupe constitué d'un atome d'hydrogène, d'un groupe alkyle inférieur de 1 à 8 atomes de carbone, de composés cycliques aliphatiques de 3 à 7 atomes et de R₁ et R₂, ainsi que l'atome N voisin, formant conjointement un composé cyclique de 5 à 7 atomes qui comprend facultativement 0 à 1 atome d'azote supplémentaire substitué par un membre sélectionné dans le groupe constitué d'un atome d'hydrogène, de groupes alkyles et aryles, ou 0 à 1 atome d'oxygène non directement lié ; R₃ et R₄ peuvent être identiques ou différents l'un de l'autre et sont sélectionnés indépendamment parmi le groupe constitué d'un atome d'hydrogène, d'un groupe alkyle inférieur de 1 à 8 atomes de carbone, de composés cycliques aliphatiques de 3 à 7 atomes et de R₃ et R₄, ainsi que l'atome N voisin, formant conjointement un composé cyclique de 5 à 7 atomes qui comprend facultativement 0 à 1 atome d'azote supplémentaire substitué par un membre sélectionné dans le groupe constitué d'un atome d'hydrogène, de groupes alkyles et aryles, ou 0 à 1 atome d'oxygène non directement lié ; et les sels pharmaceutiquement acceptables dudit composé.

32. Composé O-thiocarbamoyl-aminoalcanol représenté par la formule développée (XIV), selon la revendication 29, dans lequel x vaut 1 ; et les sels pharmaceutiquement acceptables dudit composé.

33. Composé O-thiocarbamoyl-(L)-aminoalcanol enrichi en un énantiomère selon la revendication 1, représenté par la formule développée suivante (XV):

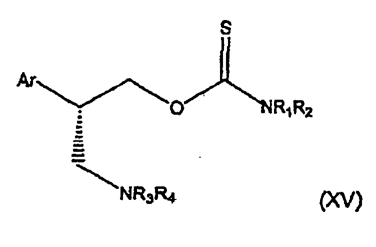

34. Composé O-thiocarbamoyl-aminoalcanol représenté par la formule développée (XV), selon la revendication 33, dans lequel R est l'hydrogène ; et les sels pharmaceutiquement acceptables dudit composé.

35. Composé O-thiocarbamoyl-aminoalcanol représenté par la formule développée (XV), selon la revendication 33, dans lequel R est l'hydrogène, R₁ et R₂ peuvent être identiques ou différents l'un de l'autre et sont sélectionnés indépendamment parmi le groupe constitué d'un atome d'hydrogène, d'un groupe alkyle inférieur de 1 à 8 atomes de carbone, de composés cycliques aliphatiques de 3 à 7 atomes et de R₁ et R₂, ainsi que l'atome N voisin, formant conjointement un composé cyclique de 5 à 7 atomes qui comprend facultativement 0 à 1 atome d'azote supplémentaire substitué par un membre sélectionné dans le groupe constitué d'un atome d'hydrogène, de groupes alkyles et aryles, ou 0 à 1 atome d'oxygène non directement lié ; R₃ et R₄ peuvent être identiques ou différents l'un de l'autre et sont sélectionnés indépendamment parmi le groupe constitué d'un atome d'hydrogène, d'un groupe alkyle inférieur de 1 à 8 atomes de carbone, de composés cycliques aliphatiques de 3 à 7 atomes et de R₃ et R₄, ainsi que l'atome N voisin, formant conjointement un composé cyclique de 5 à 7 atomes qui comprend facultativement 0 à 1 atome d'azote supplémentaire substitué par un membre sélectionné dans le groupe constitué d'un atome d'hydrogène, de groupes alkyles et aryles, ou 0 à 1 atome d'oxygène non directement lié ; et les sels pharmaceutiquement acceptables dudit composé.

36. Composé O-thiocarbamoyl-aminoalcanol représenté par la formule développée (XV), selon la revendication 33, dans lequel x vaut 1 ; et les sels pharmaceutiquement acceptables dudit composé.

37. Composé O-thiocarbamol-aminoalcanol racémique ou enrichi en un énantiomère selon la revendication 1, représenté par la formule développée suivante (XVI):

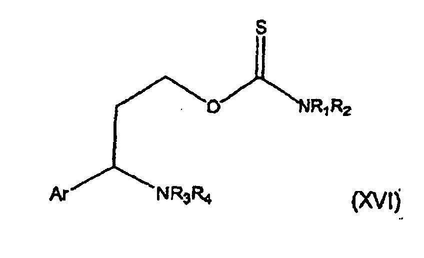

38. Composé O-thiocarbamoyl-aminoalcanol représenté par la formule développée (XVI), selon la revendication 37, dans lequel R est l'hydrogène ; et les sels pharmaceutiquement acceptables dudit composé.

39. Composé O-thiocarbamoyl-aminoalcanol représenté par la formule développée (XVI), selon la revendication 37, dans lequel R est l'hydrogène, R₁ et R₂ peuvent être identiques ou différents l'un de l'autre et sont sélectionnés indépendamment parmi le groupe constitué d'un atome d'hydrogène, d'un groupe alkyle inférieur de 1 à 8 atomes de carbone, de composés cycliques aliphatiques de 3 à 7 atomes et de R₁ et R₂, ainsi que l'atome N voisin, formant conjointement un composé cyclique de 5 à 7 atomes qui comprend facultativement 0 à 1 atome d'azote supplémentaire substitué par un membre sélectionné dans le groupe constitué d'un atome d'hydrogène, de groupes alkyles et aryles, ou 0 à 1 atome d'oxygène non directement lié ; R₃ et R₄ peuvent être identiques ou différents l'un de l'autre et sont sélectionnés indépendamment parmi le groupe constitué d'un atome d'hydrogène, d'un groupe alkyle inférieur de 1 à 8 atomes de carbone, de composés cycliques aliphatiques de 3 à 7 atomes et de R₃ et R₄, ainsi que l'atome N voisin, formant conjointement un composé cyclique de 5 à 7 atomes qui comprend facultativement 0 à 1 atome d'azote supplémentaire substitué par un membre sélectionné dans le groupe constitué d'un atome d'hydrogène, de groupes alkyles et aryles, ou 0 à 1 atome d'oxygène non directement lié ; et les sels pharmaceutiquement acceptables dudit composé.

40. Composé O-thiocarbamoyl-aminoalcanol représenté par la formule développée (XVI), selon la revendication 37, dans lequel x vaut 1; et les sels pharmaceutiquement acceptables dudit composé.

41. Composé O-thiocarbamoyl-(D)-aminoalcanol enrichi en un énantiomère selon la revendication 1, représenté par la formule développée suivante (XVII):

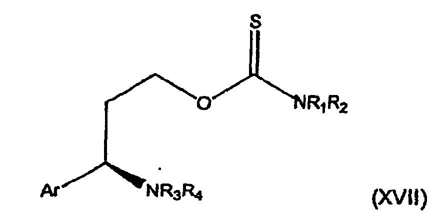

42. Composé O-thiocarbamoyl-aminoalcanol représenté par la formule développée (XVII), selon la revendication 41, dans lequel R est l'hydrogène ; et les sels pharmaceutiquement acceptables dudit composé.

43. Composé O-thiocarbamoyl-aminoalcanol représenté par la formule développée (XVII), selon la revendication 41, dans lequel R est l'hydrogène, R₁ et R₂ peuvent être identiques ou différents l'un de l'autre et sont sélectionnés indépendamment parmi le groupe constitué d'un atome d'hydrogène, d'un groupe alkyle inférieur de 1 à 8 atomes de carbone, de composés cycliques aliphatiques de 3 à 7 atomes et de R₁ et R₂, ainsi que l'atome N voisin, formant conjointement un composé cyclique de 5 à 7 atomes qui comprend facultativement 0 à 1 atome d'azote supplémentaire substitué par un membre sélectionné dans le groupe constitué d'un atome d'hydrogène, de groupes alkyles et aryles, ou 0 à 1 atome d'oxygène non directement lié ; R₃ et R₄ peuvent être identiques ou différents l'un de l'autre et sont sélectionnés indépendamment parmi le groupe constitué d'un atome d'hydrogène, d'un groupe alkyle inférieur de 1 à 8 atomes de carbone, de composés cycliques aliphatiques de 3 à 7 atomes et de R₃ et R₄, ainsi que l'atome N voisin, formant conjointement un composé cyclique de 5 à 7 atomes qui comprend facultativement 0 à 1 atome d'azote supplémentaire substitué par un membre sélectionné dans le groupe constitué d'un atome d'hydrogène, de groupes alkyles et aryles, ou 0 à 1 atome d'oxygène non directement lié ; et les sels pharmaceutiquement acceptables dudit composé.

44. Composé O-thiocarbamoyl-aminoalcanol représenté par la formule développée (XVII), selon la revendication 41, dans lequel x vaut 1 ; et les sels pharmaceutiquement acceptables dudit composé.

45. Composé O-thiocarbamoyl-(L)-aminoalcanol enrichi en un énantiomère selon la revendication 1, représenté par la formule développée suivante (XVIII):

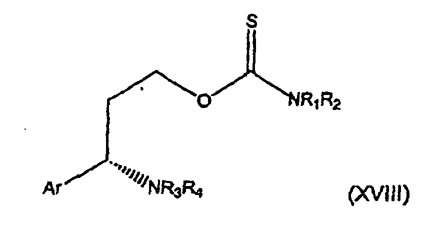

46. Composé O-thiocarbamoyl-aminoalcanol représenté par la formule développée (XVIII), selon la revendication 45, dans lequel R est l'hydrogène ; et les sels pharmaceutiquement acceptables dudit composé.

47. Composé O-thiocarbamoyl-aminoalcanol représenté par la formule développée (XVIII), selon la revendication 45, dans lequel R est l'hydrogène, R₁ et R₂ peuvent être identiques ou différents l'un de l'autre et sont sélectionnés indépendamment parmi le groupe constitué d'un atome d'hydrogène, d'un groupe alkyle inférieur de 1 à 8 atomes de carbone, de composés cycliques aliphatiques de 3 à 7 atomes et de R₁ et R₂, ainsi que l'atome N voisin, formant conjointement un composé cyclique de 5 à 7 atomes qui comprend facultativement 0 à 1 atome d'azote supplémentaire substitué par un membre sélectionné dans le groupe constitué d'un atome d'hydrogène, de groupes alkyles et aryles, ou 0 à 1 atome d'oxygène non directement lié ; R₃ et R₄ peuvent être identiques ou différents l'un de l'autre et sont sélectionnés indépendamment parmi le groupe constitué d'un atome d'hydrogène, d'un groupe alkyle inférieur de 1 à 8 atomes de carbone, de composés cycliques aliphatiques de 3 à 7 atomes et de R₃ et R₄, ainsi que l'atome N voisin, formant conjointement un composé cyclique de 5 à 7 atomes qui comprend facultativement 0 à 1 atome d'azote supplémentaire substitué par un membre sélectionné dans le groupe constitué d'un atome d'hydrogène, de groupes alkyles et aryles, ou 0 à I atome d'oxygène non directement lié ; et les sels pharmaceutiquement acceptables dudit composé.

48. Composé O-thiocarbamoyl-aminoalcanol représenté par la formule développée (XVIII), selon la revendication 45, dans lequel x vaut 1 ; et les sels pharmaceutiquement acceptables dudit composé.

49. Composé O-thiocarbamoyl-aminoalcanol racémique ou enrichi en un énantiomère selon la revendication 1, représenté par la formule développée suivante (XIX):

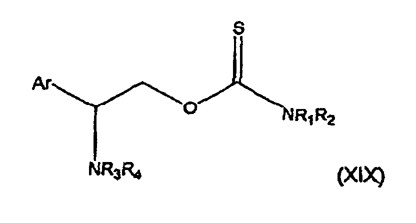

50. Composé O-thiocarbamoyl-aminoalcanol représenté par la formule développée (XIX), selon la revendication 49, dans lequel R est l'hydrogène ; et les sels pharmaceutiquement acceptables dudit composé.

51. Composé O-thiocarbamoyl-aminoalcanol représenté par la formule développée (XIX), selon la revendication 49, dans lequel R est l'hydrogène, R₁ et R₂ peuvent être identiques ou différents l'un de l'autre et sont sélectionnés indépendamment parmi le groupe constitué d'un atome d'hydrogène, d'un groupe alkyle inférieur de 1 à 8 atomes de carbone, de composés cycliques aliphatiques de 3 à 7 atomes et de R₁ et R₂, ainsi que l'atome N voisin, formant conjointement un composé cyclique de 5 à 7 atomes qui comprend facultativement 0 à 1 atome d'azote supplémentaire substitué par un membre sélectionné dans le groupe constitué d'un atome d'hydrogène, de groupes alkyles et aryles, ou 0 à 1 atome d'oxygène non directement lié ; R₃ et R₄ peuvent être identiques ou différents l'un de l'autre et sont sélectionnés indépendamment parmi le groupe constitué d'un atome d'hydrogène, d'un groupe alkyle inférieur de 1 à 8 atomes de carbone, de composés cycliques aliphatiques de 3 à 7 atomes et de R₃ et R₄, ainsi que l'atome N voisin, formant conjointement un composé cyclique de 5 à 7 atomes qui comprend facultativement 0 à 1 atome d'azote supplémentaire substitué par un membre sélectionné dans le groupe constitué d'un atome d'hydrogène, de groupes alkyles et aryles, ou 0 à 1 atome d'oxygène non directement lié ; et les sels pharmaceutiquement acceptables dudit composé.

52. Composé O-thiocarbamoyl-aminoalcanol représenté par la formule développée (XIX), selon la revendication 49, dans lequel x vaut 1; et les sels pharmaceutiquement acceptables dudit composé.

53. Composé O-thiocarbamoyl-(D)-aminoalcanol enrichi en un énantiomère selon la revendication 1, représenté par la formule développée suivante (XX) :

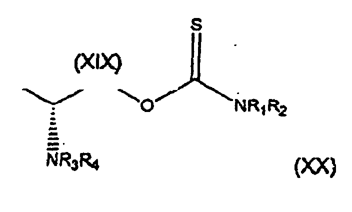

54. Composé O-thiocarbamoyl-aminoalcanol représenté par la formule développée (XX), selon la revendication 53, dans lequel R est l'hydrogène ; et les sels pharmaceutiquement acceptables dudit composé.

55. Composé O-thiocarbamoyl-aminoalcanol représenté par la formule développée (XX), selon la revendication 53, dans lequel R est l'hydrogène, R₁ et R₂ peuvent être identiques ou différents l'un de l'autre et sont sélectionnés indépendamment parmi le groupe constitué d'un atome d'hydrogène, d'un groupe alkyle inférieur de 1 à 8 atomes de carbone, de composés cycliques aliphatiques de 3 à 7 atomes et de R₁ et R₂, ainsi que l'atome N voisin, formant conjointement un composé cyclique de 5 à 7 atomes qui comprend facultativement 0 à 1 atome d'azote supplémentaire substitué par un membre sélectionné dans le groupe constitué d'un atome d'hydrogène, de groupes alkyles et aryles, ou 0 à 1 atome d'oxygène non directement lié ; R₃ et R₄ peuvent être identiques ou différents l'un de l'autre et sont sélectionnés indépendamment parmi le groupe constitué d'un atome d'hydrogène, d'un groupe alkyle inférieur de 1 à 8 atomes de carbone, de composés cycliques aliphatiques de 3 à 7 atomes et de R₃ et R₄, ainsi que l'atome N voisin, formant conjointement un composé cyclique de 5 à 7 atomes qui comprend facultativement 0 à 1 atome d'azote supplémentaire substitué par un membre sélectionné dans le groupe constitué d'un atome d'hydrogène, de groupes alkyles et aryles, ou 0 à 1 atome d'oxygène non directement lié ; et les sels pharmaceutiquement acceptables dudit composé.

56. Composé O-thiocarbamoyl-aminoalcanol représenté par la formule développée (XX), selon la revendication 53, dans lequel x vaut 1; et les sels pharmaceutiquement acceptables dudit composé.

57. Composé O-thiocarbamoyl-(L)-aminoalcanol enrichi en un énantiomère selon la revendication 1, représenté par la formule développée suivante (XXI):

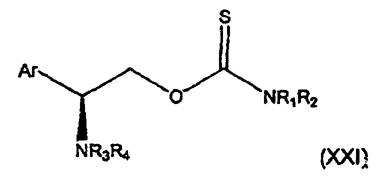

58. Composé O-thiocarbamoyl-aminoalcanol représenté par la formule développée (XXI), selon la revendication 57, dans lequel R est l'hydrogène ; et les sels pharmaceutiquement acceptables dudit composé.

59. Composé O-thiocarbamoyl-aminoalcanol représenté par la formule développée (XXI), selon la revendication 57, dans lequel R est l'hydrogène, R₁ et R₂ peuvent être identiques ou différents l'un de l'autre et sont sélectionnés indépendamment parmi le groupe constitué d'un atome d'hydrogène, d'un groupe alkyle inférieur de 1 à 8 atomes de carbone, de composés cycliques aliphatiques de 3 à 7 atomes et de R₁ et R₂, ainsi que l'atome N voisin, formant conjointement un composé cyclique de 5 à 7 atomes qui comprend facultativement 0 à 1 atome d'azote supplémentaire substitué par un membre sélectionné dans le groupe constitué d'un atome d'hydrogène, de groupes alkyles et aryles, ou 0 à 1 atome d'oxygène non directement lié ; R₃ et R₄ peuvent être identiques ou différents l'un de l'autre et sont sélectionnés indépendamment parmi le groupe constitué d'un atome d'hydrogène, d'un groupe alkyle inférieur de 1 à 8 atomes de carbone, de composés cycliques aliphatiques de 3 à 7 atomes et de R₃ et R₄, ainsi que l'atome N voisin, formant conjointement un composé cyclique de 5 à 7 atomes qui comprend facultativement 0 à 1 atome d'azote supplémentaire substitué par un membre sélectionné dans le groupe constitué d'un atome d'hydrogène, de groupes alkyles et aryles, ou 0 à 1 atome d'oxygène non directement lié ; et les sels pharmaceutiquement acceptables dudit composé.

60. Composé O-thiocarbamoyl-aminoalcanol représenté par la formule développée (XXI), selon la revendication 57, dans lequel x vaut 1 ; et les sels pharmaceutiquement acceptables dudit composé.

61. Procédé pour la préparation d'un composé de formule (VI) telle que définie dans la revendication 1, dans lequel on fait réagir un aminoalcanol de formule générale (II) 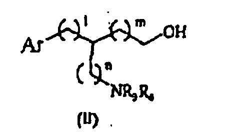 avec du dicarbonate de di-*t*-butyl pour synthétiser le composé N-t-butyloxycarbonyl-aminoalcanol représenté par la formule (III), 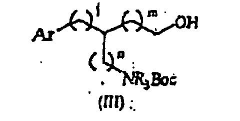 puis on fait subir un traitement en présence de soude, de sulfure de carbone et d'iodure de méthyle en solution d'éther, suivi d'un traitement avec un composé aminé de formule (IV)
HNR₁R₂ (IV)
pour produire le composé O-thiocarbamoyl-N-t-butyloxycarbonyl-aminoalcanol représenté par la formule générale (V), 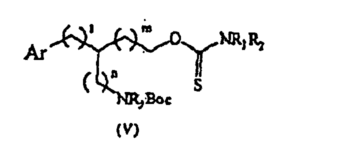 dans lequel Boc représente un radical *t*-butyloxy-carbonyle, dont la protection est retirée à l'aide d'une solution aqueuse d'acide chlorhydrique, pour donner un composé O-thiocarbamoyl-aminoalcanol représenté par la formule générale (VI), 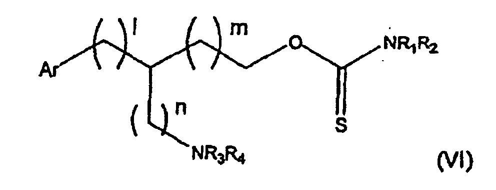 dans lequel Ar, R₁, R₂ , R₃, R₄, l, m et n sont conformes à la définition fournie ci-dessus dans la revendication 1, à condition que R₃ = R₄ = H ou R₄ = H.

62. Procédé de la revendication 61 dans lequel le composé de formule (VI) peut être converti, sans purification supplémentaire, en ses sels pharmaceutiquement acceptables de formule (I).

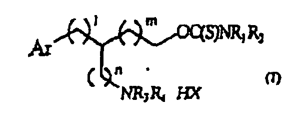

63. Procédé pour la préparation d'un composé de formule (VI) telle que définie dans la revendication 1, dans lequel on fait réagir un aminoalcanol de formule générale (II) 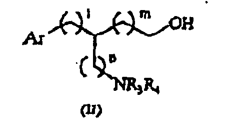 avec de la soude, du sulfure de carbone et de l'iodure de méthyle en solution d'éther, puis on fait subir un traitement en présence d'un composé aminé de formule (IV)
HNR₁R₂ (IV)
pour produire un composé O-thiocarbamoyl-aminoalcanol représenté par la formule générale (VI), 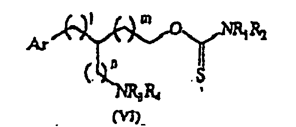 dans lequel Ar, R₁, R₂ , R₃, R₄, l, m et n sont conformes à la définition fournie ci-dessus dans la revendication 1, à condition que R₃ et R₄ ne soient pas de l'hydrogène.

64. Procédé de la revendication 63 dans lequel le composé de formule (VI) peut être converti, sans purification supplémentaire, en ses sels pharmaceutiquement acceptables de formule (I).

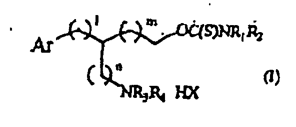

65. Procédé pour la préparation d'un composé de formule (VI) telle que définie dans la revendication 1, dans lequel on fait réagir un aminoalcanol de formule générale (II) 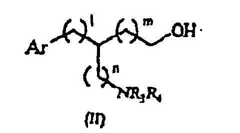 avec du dicarbonate de di-t-butyl pour synthétiser le composé N-*t*-butyloxycarbonyl-aminoalcanol représenté par la formule (III), 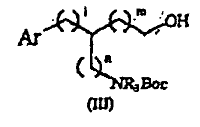 puis on fait subir un traitement en présence d'un composé isothiocyanate de formule (VII)
R₂NCS (VII)
en solution d'hydrocarbure halogéné, pour produire le composé O-thiocarbamoyl-N-*t*-butyloxycarbonyl-aminoalcanol représenté par la formule générale (V), 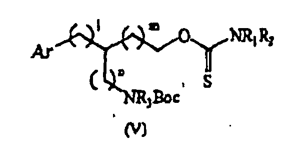 dans lequel Boc représente un radical *t*-butyloxy-carbonyle, dont la protection est retirée à l'aide d'une solution aqueuse d'acide chlorhydrique, pour donner un composé O-thiocarbamoyl-aminoalcanol représenté par la formule générale (VI), 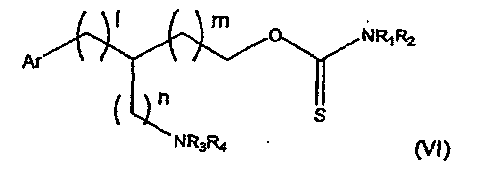 dans lequel Ar, R₁, R₂ , R₃, R₄, l, m et n sont conformes à la définition fournie ci-dessus dans la revendication 1, à condition que R₁ = R₃ = R₄ = H ou R₁ = R₄ = H.

66. Procédé de la revendication 65 dans lequel le composé de formule (VI) peut être converti, sans purification supplémentaire, en ses sels pharmaceutiquement acceptables de formule (I).

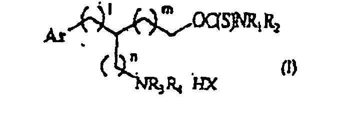

67. Procédé pour la préparation d'un composé de formule (VI) telle que définie dans la revendication 1, dans lequel on fait réagir un aminoalcanol de formule générale (II) 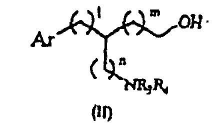 avec un composé isothiocyanate de formule (VII)
R₂NCS (VII)
en solution d'hydrocarbure halogéné, pour produire une composé O-thiocarbamoyl-aminoalcanol représenté par la formule générale (VI), 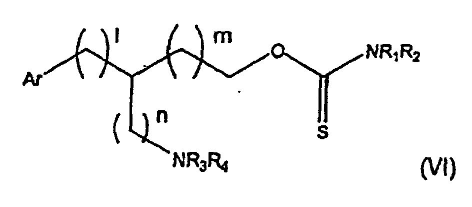 dans lequel Ar, R₁, R₂ , R₃, R₄, l, m et n sont conformes à la définition fournie ci-dessus dans la revendication 1, à condition que R₁ soit de l'hydrogène et que R₃ et R₄ ne soient pas de l'hydrogène.

68. Procédé de la revendication 67 dans lequel le composé de formule (VI) peut être converti, sans purification supplémentaire, en ses sels pharmaceutiquement acceptables de formule (I).

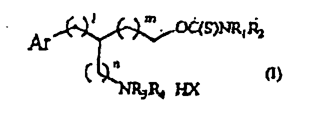

69. Procédé pour la préparation d'un composé de formule (VIII) selon la revendication 61, dans lequel un composé (D)-aminoalcanol est employé.

70. Procédé pour la préparation d'un composé de formule (VIII) selon la revendication 63, dans lequel un composé (D)-aminoalcanol est employé.

71. Procédé pour la préparation d'un composé de formule (VIII) selon la revendication 65, dans lequel un composé (D)-aminoalcanol est employé.

72. Procédé pour la préparation d'un composé de formule (VIII) selon la revendication 67, dans lequel un composé (D)-aminoalcanol est employé.

73. Procédé pour la préparation d'un composé de formule (IX) selon la revendication 61, dans lequel un composé (L)-aminoalcanol est employé.

74. Procédé pour la préparation d'un composé de formule (VIII) selon la revendication 63, dans lequel un composé (L)-aminoalcanol est employé.

75. Procédé pour la préparation d'un composé de formule (VIII) selon la revendication 65, dans lequel un composé (L)-aminoalcanol est employé.

76. Procédé pour la préparation d'un composé de formule (VIII) selon la revendication 67, dans lequel un composé (L)-aminoalcanol est employé.

77. Composition pharmaceutique, comprenant un composé de formule (VI) ou un sel pharmaceutiquement acceptable dudit composé selon la revendication 1, ainsi qu'un véhicule ou diluant pharmaceutiquement acceptable non toxique.

78. Composition pharmaceutique, comprenant un composé de formule (VIII) ou un sel pharmaceutiquement acceptable selon la revendication 5, ainsi qu'un véhicule ou diluant pharmaceutiquement acceptable non toxique.

79. Composition pharmaceutique, comprenant un composé de formule (IX) ou un sel pharmaceutiquement acceptable selon la revendication 9, ainsi qu'un véhicule ou diluant pharmaceutiquement acceptable non toxique.

80. Composé selon la revendication 1, dans lequel le composé est sélectionné parmi O-thiocarbomoyl-(DL)-phénylalaninol, O-thiocarbomoyl-(D)-phénylalaninol et O-thiocarbomoyl-(L)-phénylalaninol, et leurs sels pharmacologiquement acceptables non toxiques.

81. Composition pharmaceutique comprenant un composé selon la revendication 1, dans lequel le composé est O-thiocarbomoyl-(D)-phénylalaninol et ses sels pharmacologiquement acceptables non toxiques.

82. Composition pharmaceutique comprenant un composé selon la revendication 1, dans lequel le composé est O-thiocarbomoyl-(L)-phénylalaninol et ses sels pharmacologiquement acceptables non toxiques.
